# EUROPEAN PATENT SPECIFICATION

(11) **EP 4 225 186 B1**
(45) Date of publication and mention of the grant of the patent: **12.08.2026**
(21) Application number: 21810085.7
(22) Date of filing: 05.10.2021
(51) Int. Cl.: A61B 18/14, A61B 5/00

(54) **SYSTEMS FOR THERAPEUTIC NASAL TREATMENT**
SYSTEME ZUR THERAPEUTISCHEN NASALEN BEHANDLUNG
SYSTÈMES DE TRAITEMENT NASAL THÉRAPEUTIQUE

(30) Priority: 06.10.2020 US 202063087978 P
(43) Date of publication of application: 16.08.2023
(73) Proprietor: Neurent Medical Limited, Oranmore, Galway (IE)
(72) Inventor: TOWNLEY, David, Newmarket-on-Fergus, County Clare (IE)
(74) Representative: HGF
(86) International application number: PCT/IB2021/000699
(87) International publication number: WO 2022/074460

(56) References cited:
- WO-A1-2016/132339
- WO-A1-2021/205231
- WO-A1-2021/260435
- WO-A1-2022/058797
- US-A1- 2004 186 469
- US-A1- 2015 112 321
- US-A1- 2016 331 459
- US-A1- 2017 215 952
- US-A1- 2018 133 460
- US-A1- 2019 223 944
- US-A1- 2020 289 185

## Description

### Cross-Reference to Related Applications

This application claims priority to, and the benefit of, U.S. Provisional Application No. 63/087,978, filed October 6, 2020.

### Field of the Invention

The invention generally relates to treating nasal conditions, and, more particularly, to systems for providing precision targeting and comprehensive treatment of specific tissue(s) of interest within a nasal region of a patient to undergo therapeutic treatment while minimizing or avoiding unintended collateral damage to surrounding or adjacent non-targeted tissue.

### Background

A common goal of any medical procedure is to address a particular condition without causing any further complications, or to at least minimize negative side effects.

For example, surgical procedures, such as ablation therapy, require a surgeon to apply precise energy treatment to the intended target site (i.e., tissue intended to receive treatment) at appropriate levels so as to treat the condition while avoiding collateral damage to surrounding tissue, which can lead to further complications and even death. Certain procedures require increased precision due to the nature of the tissue to be treated and the location of such tissue in relation to any nearby or underlying tissue that may be highly sensitive and/or is critical to keep intact and free of unintended damage (i.e., blood vessels, muscle or nerve fibers, bone, surface tissue, etc.).

This is particularly true for surgical procedures treating rhinosinusitis, an inflammatory disease of the nose. One surgical option includes the delivery of thermal energy to inflamed soft tissue within the nasal cavity, resulting in scarring and temporary volumetric reduction of the tissue to improve nasal airflow. Another surgical option includes microdebrider resection of the inflamed soft tissue, resulting in the removal of tissue to improve nasal airflow. While both options may address one of the main symptoms (e.g., nasal congestion), they fail to address the other main symptom, rhinorrhea, and further have risks ranging from bleeding and scarring, to the use of general anesthetic. Additionally, these surgical procedures cannot precisely target neural tissue, thereby causing significant collateral damage to surrounding non-neural tissue in an attempt to treat rhinosinusitis.

Furthermore, some current procedures employing electrotherapeutic treatment (i.e., neuromodulation, ablation, etc.) lack the ability to deliver such treatment in a controlled manner. For example, in some instances, an insufficient amount of energy may be delivered to the intended target site. This may include delivering energy at a level that is too low and/or for an inadequate period of time such that the targeted tissue remains untreated. Additionally, energy may be delivered inconsistently across portions of the targeted tissue, further resulting in incomplete treatment and failure to fully address the rhinosinusitis condition. Alternatively, an excess amount of energy may be delivered to the intended target site (i.e., energy level is too high and/or energy is delivered to an overextended period of time). While this may achieve the intended result to the targeted tissue, it further results in unintended collateral damage, thereby leading to further complications. US2018/133460A1 relates to a devices, systems, and methods for specializing, monitoring, and/or evaluating therapeutic nasal neuromodulation. WO2021/260435A1 and WO2021/205231A1 relate to systems and methods for providing detection, identification, and precision targeting of specific tissue of interest to undergo a therapeutic treatment while minimizing or avoiding collateral damage to surrounding or adjacent non- targeted tissue. US2017/215952A1 relates to a system for ablating and characterizing tissue. US2016/331459A1 relates to devices for therapeutic nasal neuromodulation and associated systems and methods. US2019/223944A1 relates to neuromodulation catheter systems with controlled irrigation capabilities and methods for using such systems. US2020/289185A1 relates to an ablation delivery system is configured to allow separate voltage levels of a capacitor stack to be accessed for use in therapy delivery. US2004/186469, WO2016/132339 and WO2022/058797 refer to further prior art documents relevant for the present invention.

### Summary

The invention is defined in the appended claims. Methods are per se not part of the claimed invention. The invention recognizes that knowing certain properties of tissue, both active and passive, at a given target site prior to and during electrotherapeutic treatment (i.e., neuromodulation, ablation, etc.) provides an ability to more precisely target a specific tissue of interest (i.e., targeted tissue) to treat a condition. More specifically, the invention provides systems and methods with an ability to tune and provide precision targeting and comprehensive treatment of a tissue of interest in a nasal region of a patient for the treatment of rhinosinusitis, while minimizing or avoiding collateral damage to surrounding, non-targeted tissue.

For example, in one scenario, a surgeon may wish to provide electrotherapeutic stimulation to a nerve tissue, while avoiding the delivery of any such stimulation to surrounding, adjacent tissue at the target site, as unintended collateral damage may result in damage to non-targeted tissue and cause further complications. At the same time, it is critical that the appropriate amount of energy is delivered to the targeted tissue to ensure that the entirety of the targeted tissue receives such stimulation to elicit the desired effect. **In** such a scenario, the specific type of targeted neural tissue may generally dictate the level of electrical stimulation required to elicit a desired effect. Furthermore, physical properties of the targeted tissue, including the specific location and depth of the targeted neural tissue, in relation to the non-targeted tissue, further impacts the level of electrical stimulation necessary to result in effective therapeutic treatment.

The invention provides systems and methods with an ability to characterize, prior to an electrotherapeutic treatment, the type of tissue at a target site by sensing at least one of physiological properties, bioelectric properties, and thermal properties of tissue, wherein such characterization includes identifying specific types of tissue present at the target site. For example, different tissue types include different physiological and histological characteristics. As a result of the different characteristics, different tissue types have different associated bioelectrical properties and thus exhibit different behavior in response to application of energy applied thereto.

By knowing such properties of a given tissue, the systems and methods are configured to determine a specific pulsed energy treatment pattern for controlling delivery of pulsed energy to the targeted tissue which is sufficient to maintain a consistent temperature during treatment to ensure successful and complete ablation and/or modulation of the targeted tissue, while minimizing or entirely preventing unintended collateral damage to non-targeted tissue. In particular, a given pulsed energy treatment pattern may include, for example, a specific profile, including a predetermined treatment time, a precise level of energy to be delivered, and a predetermined temperature range for that particular tissue. As a result, the profile (i.e., specific level of energy, treatment time, and temperature range) is precisely tuned to the targeted tissue (based on the known and characterized properties of the targeted tissue), such that the appropriate amount of energy is delivered and the entirety of the targeted tissue receives adequate treatment while unintended collateral damage to surrounding or adjacent non-targeted tissue is minimized or prevented.

The systems and methods are further configured to receive and process real-time feedback data associated with the targeted tissue undergoing treatment to further ensure that energy delivered is maintained within the scope of the pulsed energy treatment pattern. More specifically, the systems and methods are configured to automatically control delivery of energy to the targeted tissue based on the processing of the real-time feedback data, wherein such data includes at least temperature measurement data associated with the targeted tissue collected during delivery of energy to the targeted tissue. The controller is configured to process temperature measurement data to detect whether the temperature at the target site, notably a temperature reading of the targeted tissue undergoing treatment, falls within a predetermined temperature range (i.e., above a minimum set point and below a maximum set point) to thereby maintain a consistent temperature ensuring the targeted tissue in its entirety received adequate treatment.

In turn, the controller is configured to automatically control the delivery of energy to the targeted tissue based on real-time monitoring of feedback data, most notably temperature measurement data, to ensure the desired ablation/modulation is achieved. The controller is configured to cause delivery of pulsed energy to the targeted tissue, wherein the pulsed energy is at a specific level and an associated duty cycle for a specific period of time based on feedback data. The pulsed energy is sufficient to maintain the temperature of the targeted tissue within the appropriate range so as to cause ablation and/or modulation thereof, while further preventing, in some instances, irreversible tissue damage.

As a result, the systems and methods are able to ensure that optimal energy is delivered until the target ablation/modulation depth is achieved, while maintaining clinically relevant treatment time. This is particularly important with respect to electrotherapeutic treatment in the nasal cavity, as intermittent electrode-tissue contact may result in coagulum forming due to fluctuating waveforms, thereby causing a treatment process to be less effective as certain portions of a treatment device (i.e., electrodes) may have inconsistent delivery of energy. The systems and methods ensure repeatable and controllable delivery of treatment energy, specifically pulsed energy, from each of a plurality of electrodes in order to achieve the desired surgical outcome.

One aspect of the present disclosure provides a method for treating a condition within a sino-nasal cavity of a patient. The method includes delivering treatment energy to tissue at a target site within a sino-nasal cavity of the patient based, at least in part, on a pulsed energy treatment pattern sufficient to maintain a consistent temperature of targeted tissue receving the treatment energy to thereby cause ablation and/or modulation of targeted tissue for the treatment of a nasal condition.

In some embodiments, the treatment energy is delivered via one or more electrodes of an end effector and supplied thereto from a controller operably associated with the end effector and based, at least in part, on the pulsed energy treatment pattern. The pulsed energy treatment pattern is determined based, at least in part, on processing of, via the controller, identifying data received from the end effector associated with tissue at the target site. The identifying data may be associated with one or more properties of the one or more tissues, the one or more properties comprising at least one of a type, a depth, and a location of each of the one or more tissues.

In some embodiments, a subset of the one or more electrodes is configured to deliver non-therapeutic stimulating energy at a frequency/waveform to respective positions at the one or more target sites to thereby sense at least one of physiological properties, bioelectric properties, and thermal properties of the one or more tissues at the target site. The processing of the identifying data, via the controller, includes comparing the identifying data received from the device with electric signature data associated with a plurality of known tissue types. The comparison includes correlating the identifying data received from the end effector with electric signature data from a supervised and/or an unsupervised trained neural network.

The pulsed energy treatment pattern may include data associated with at least one of a predetermined treatment time, a level of energy to be delivered from the electrodes, and a predetermined temperature range. The treatment energy is delivered based, at least in part, on processing, via the controller, of real-time feedback data associated with the one or more tissues upon supplying treatment energy thereto. The feedback data includes, for example, at least one of temperature measurement data associated with the targeted tissue, a level of energy delivered, and an elapsed delivery time. In some embodiments, the temperature measurement data is collected via one or more temperature sensors operably associated with the end effector. For example, each of the one or more electrodes of the end effector may be operably associated with the one or more temperature sensors.

The controller is configured to process the feedback data using an algorithm to determine efficacy of ablation/modulation of the targeted tissue based, at least in part, on a comparison of the feedback data with pulsed energy treatment pattern data. The predetermined temperature range may be between 50 °C and 60 °C. Accordingly, delivery of treatment energy to the targeted tissue may include delivering pulsed energy from one or more electrodes at a level and an associated duty cycle sufficient to regulate and maintain temperature of the targeted tissue receiving the treatment energy at approximately 60 °C with a tolerance of approximately ±0.2 °C. The delivery of energy based on the pulsed energy treatment pattern may result in ablation and/or modulation of targeted tissue sufficient to treat the nasal condition while minimizing or preventing collateral damage to non-targeted tissue at the target site.

For example, in some embodiments, the treatment energy delivered disrupts multiple neural signals to, and/or results in local hypoxia of, mucus producing and/or mucosal engorgement elements, thereby reducing production of mucus and/or mucosal engorgement within a nose of the patient and reducing or eliminating one or more symptoms associated with the rhinosinusitis. In some embodiments, the targeted tissue may be associated with one or more target sites proximate or inferior to a sphenopalatine foramen, wherein energy is delivered at a level sufficient to therapeutically modulate postganglionic parasympathetic nerves innervating nasal mucosa at foramina and/or microforamina of a palatine bone of the patient and causes multiple points of interruption of neural branches extending through foramina and/or microforamina of palatine bone. In some embodiments, the targeted tissue comprises neural tissue. In other embodiments, energy is delivered at a level sufficient to ablate targeted tissue to thereby cause thrombus formation within one or more blood vessels associated with mucus producing and/or mucosal engorgement elements within the nose, wherein the resulting local hypoxia of the mucus producing and/or mucosal engorgement elements results in decreased mucosal engorgement to thereby increase volumetric flow through a nasal passage of the patient.

According to the invention a system for treating a condition within a sino-nasal cavity of a patient is provided.

The system includes a treatment device including an end effector comprising one or more electrodes and a controller operably associated with the treatment device. The controller is configured to control delivery of treatment energy from the one or more electrodes to tissue at a target site within a sino-nasal cavity of the patient based, at least in part, on a pulsed energy treatment pattern sufficient to maintain a consistent temperature of targeted tissue receving the treatment energy to thereby cause ablation and/or modulation of targeted tissue for the treatment of a nasal condition.

The controller is configured to determine the pulsed energy treatment pattern based, at least in part, on processing identifying data received from the end effector associated with tissue at the target site. The identifying data is associated with a type of each of the one or more tissues.

A subset of the one or more electrodes is configured to deliver non-therapeutic stimulating energy at a frequency/waveform to respective positions at the one or more target sites to thereby sense at least one of physiological properties, bioelectric properties, and thermal properties of the one or more tissues at the target site. The processing of the identifying data, via the controller, includes comparing the identifying data received from the device with electric signature data associated with a plurality of known tissue types. The comparison includes correlating the identifying data received from the end effector with electric signature data from a supervised and/or an unsupervised trained neural network.

The pulsed energy treatment pattern includes data associated with a predetermined treatment time, a level of energy to be delivered from the electrodes, and a predetermined temperature range. The treatment energy is delivered based, at least in part, on processing, via the controller, of real-time feedback data associated with the one or more tissues upon supplying treatment energy thereto. The feedback data includes temperature measurement data associated with the targeted tissue, a level of energy delivered, and an elapsed delivery time. In some embodiments, the temperature measurement data is collected via one or more temperature sensors operably associated with the end effector. For example, each of the one or more electrodes of the end effector may be operably associated with the one or more temperature sensors. Yet still, in some embodiments, each of the electrodes is operably associated with a separate respective one of the temperature sensors.

The controller is configured to process the feedback data using an algorithm to determine efficacy of ablation/modulation of the targeted tissue based, at least in part, on a comparison of the feedback data with pulsed energy treatment pattern data. The predetermined temperature range may be between 50 °C and 60 °C. Accordingly, delivery of treatment energy to the targeted tissue may include delivering pulsed energy from one or more electrodes at a level and an associated duty cycle sufficient to regulate and maintain temperature of the targeted tissue receiving the treatment energy at approximately 60 °C with a tolerance of approximately ±0.2 °C. The delivery of energy based on the pulsed energy treatment pattern results in ablation modulation of targeted tissue sufficient to treat the nasal condition while minimizing or preventing collateral damage to non-targeted tissue at the target site.

For example, in some embodiments, the treatment energy delivered disrupts multiple neural signals to, and/or results in local hypoxia of, mucus producing and/or mucosal engorgement elements, thereby reducing production of mucus and/or mucosal engorgement within a nose of the patient and reducing or eliminating one or more symptoms associated with the rhinosinusitis. In some embodiments, the targeted tissue may be associated with one or more target sites proximate or inferior to a sphenopalatine foramen, wherein energy is delivered at a level sufficient to therapeutically modulate postganglionic parasympathetic nerves innervating nasal mucosa at foramina and/or microforamina of a palatine bone of the patient and causes multiple points of interruption of neural branches extending through foramina and/or microforamina of palatine bone. In some embodiments, the targeted tissue comprises neural tissue. In other embodiments, energy is delivered at a level sufficient to ablate targeted tissue to thereby cause thrombus formation within one or more blood vessels associated with mucus producing and/or mucosal engorgement elements within the nose, wherein the resulting local hypoxia of the mucus producing and/or mucosal engorgement elements results in decreased mucosal engorgement to thereby increase volumetric flow through a nasal passage of the patient.

### Brief Description of the Drawings

FIGS. 1A and 1B are diagrammatic illustrations of a therapeutic neuromodulation system for treating a condition within a nasal cavity using a handheld device according to some embodiments of the present disclosure.
FIG. 2 is a diagrammatic illustration of the console coupled to the handheld neuromodulation device consistent with the present disclosure, further illustrating one embodiment of an end effector of the handheld device for delivering energy to tissue at the one or more target sites within the nasal cavity.
FIG. 3 is a diagrammatic illustration of the console coupled to the handheld neuromodulation device consistent with the present disclosure, further illustrating another embodiment of an end effector of the handheld device for delivering energy, via proximal and distal segments, to tissue at the one or more target sites within the nasal cavity.
FIG. 4A is a cut-away side view illustrating the anatomy of a lateral nasal wall.
FIG. 4B is an enlarged side view of the nerves of the lateral nasal wall of FIG. 4A.
FIG. 4C is a front view of a left palatine bone illustrating geometry of microforamina in the left palatine bone.
FIG. 5 is a side view of one embodiment of a handheld device for providing therapeutic nasal neuromodulation consistent with the present disclosure.
FIG. 6 is an enlarged, perspective view of the end effector of FIG. 2 in an expanded state.

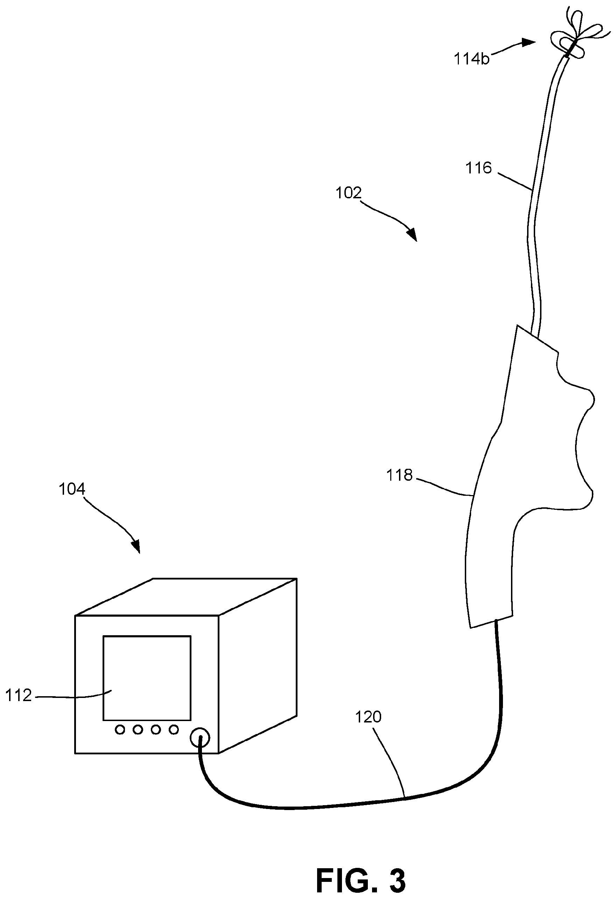

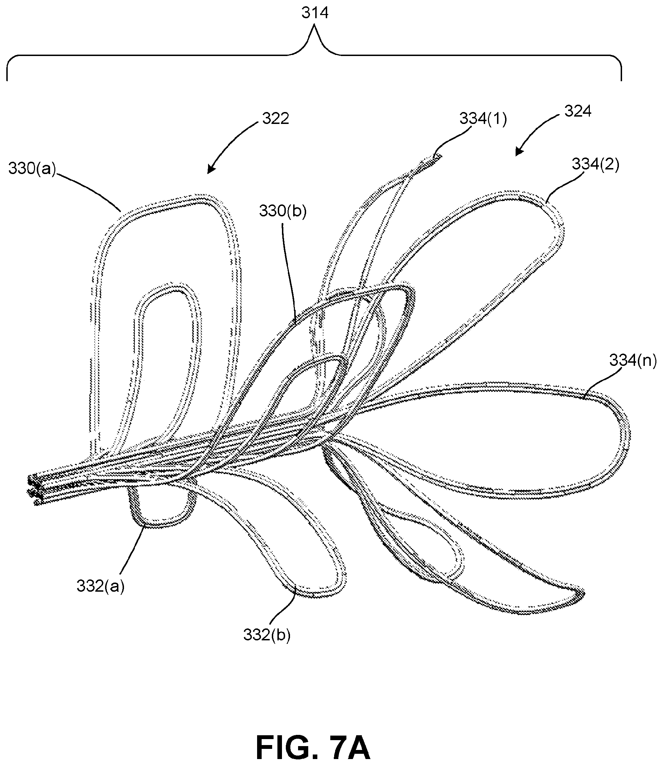

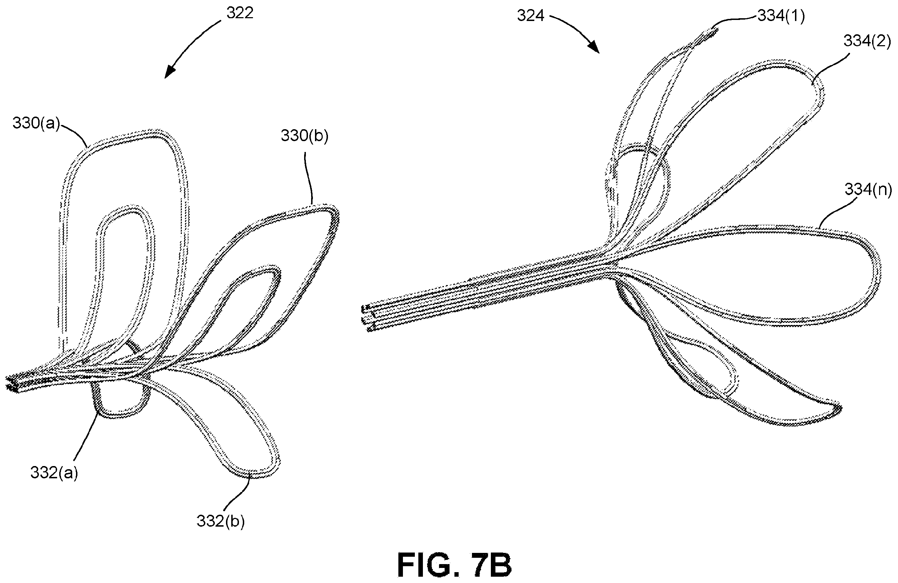

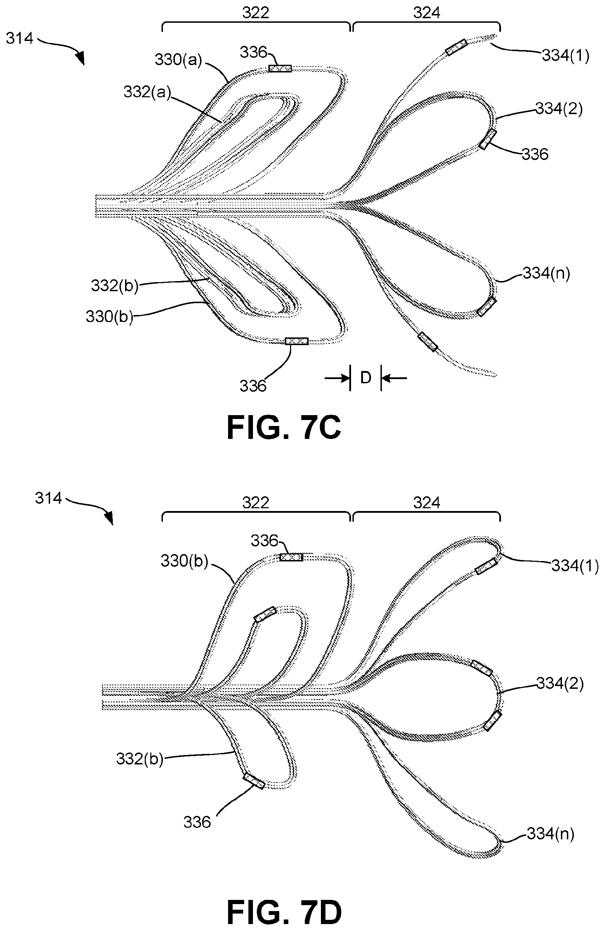

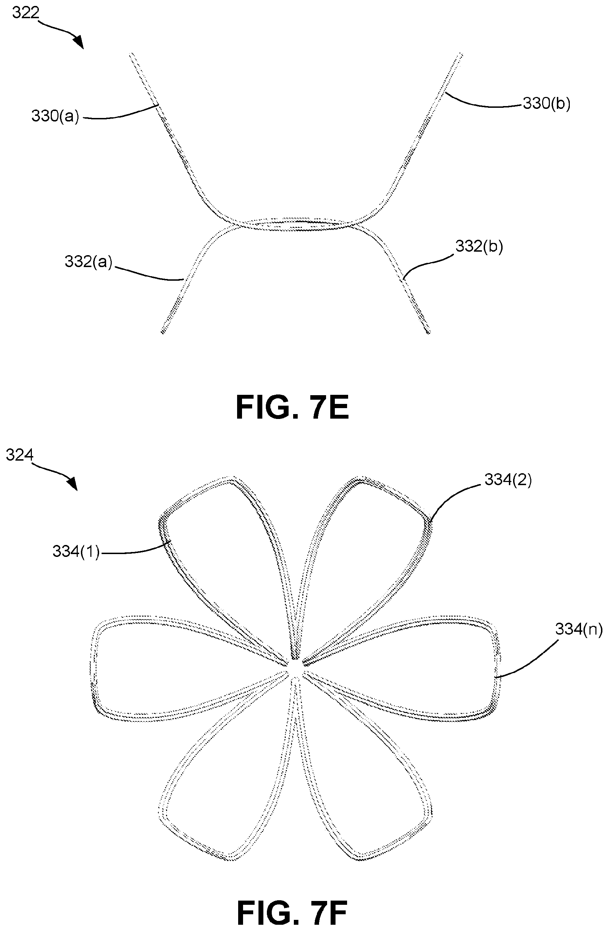

FIGS. 7A-7F are various views of the multi-segment end effector of FIG. 3. FIG. 7A is an enlarged, perspective view of the multi-segment end effector illustrating the first (proximal) segment and second (distal) segment. FIG. 7B is an exploded, perspective view of the multi-segment end effector. FIG. 7C is an enlarged, top view of the multi-segment end effector. FIG. 7D is an enlarged, side view of the multi-segment end effector. FIG. 7E is an enlarged, front (proximal facing) view of the first (proximal) segment of the multi-segment end effector. FIG. 7F is an enlarged, front (proximal facing) view of the second (distal) segment of the multi-segment end effector.
FIG. 8 is a perspective view, partly in section, of a portion of a support element illustrating an exposed conductive wire serving as an energy delivery element or electrode element.
FIG. 9 is a cross-sectional view of a portion of the shaft of the handheld device taken along lines 9-9 of FIG. 5.
FIG. 10A is a side view of the handle of the handheld device.
FIG. 10B is a side view of the handle illustrating internal components enclosed within.
FIG. 11 is a partial cut-away side view illustrating one approach for delivering an end effector a target site within a nasal region in accordance with embodiments of the present disclosure.
FIG. 12A is a block diagram illustrating delivery of non-therapeutic energy from electrodes of the end effector at a frequency/waveform for sensing one or more properties associated with one or more tissues at a target site in response to the non-therapeutic energy.
FIG. 12B is a block diagram illustrating communication of sensor data from the handheld device to the controller and subsequent determination, via the controller, of a pulsed energy treatment pattern for controlling energy delivery based on the sensor data for precision targeting of tissue of interest and to be treated (i.e., neural tissue).
FIG. 12C is a block diagram illustrating delivery of energy to the target site based on the treatment pattern output from the controller, monitoring of real-time feedback data associated with the targeted neural tissue undergoing treatment, and subsequent control over the delivery of energy based on the processing of the feedback data.
FIG. 13 is a flow diagram illustrating one embodiment of a method for treating a condition.
FIGS. 14A, 14B, and 14B are graphs illustrating power profiles associated with pulsed energy treatment patterns delivered at 3 watts, 4 watts, and 5 watts, respectively.
FIGS. 15A, 15B, and 15C are graphs illustrating voltage profiles associated with pulsed energy treatment patterns at 3 watts, 4 watts, and 5 watts, respectively.
FIGS. 16A, 16B, and 16C are graphs illustrating impedance profiles associated with pulsed energy treatment patterns at 3 watts, 4 watts, and 5 watts, respectively.
FIGS. 17A, 17B, and 17C are graphs illustrating temperature profiles associated with pulsed energy treatment patterns at 3 watts, 4 watts, and 5 watts, respectively.
FIGS. 18A, 18B, and 18C illustrate resulting ablation zones of tissue depth following delivery of pulsed energy based on a pulsed energy treatment pattern at 3 watts, 4 watts, and 5 watts, respectively.

### Detailed Description

There are various conditions related to the nasal cavity which may impact breathing and other functions of the nose. One of the more common conditions is rhinitis, which is defined as inflammation of the membranes lining the nose. The symptoms of rhinitis include nasal blockage, obstruction, congestion, nasal discharge (e.g., rhinorrhea and/or posterior nasal drip), facial pain, facial pressure, and/or reduction or complete loss of smell and/or taste. Sinusitis is another common condition, which involves an inflammation or swelling of the tissue lining the sinuses, which can lead to subsequent. Rhinitis and sinusitis are frequently associated with one another, as sinusitis is often preceded by rhinitis. Accordingly, the term rhinosinusitis is often used to describe both conditions.

Depending on the duration and type of systems, rhinosinusitis can fall within different subtypes, including allergic rhinitis, non-allergic rhinitis, chronic rhinitis, acute rhinitis, recurrent rhinitis, chronic sinusitis, acute sinusitis, recurrent sinusitis, and medical resistant rhinitis and/or sinusitis, in addition to combinations of one or more of the preceding conditions. It should be noted that an acute rhinosinusitis condition is one in which symptoms last for less than twelve weeks, whereas a chronic rhinosinusitis condition refers to symptoms lasting longer than twelve weeks.

A recurrent rhinosinusitis condition refers to four or more episodes of an acute rhinosinusitis condition within a twelve-month period, with resolution of symptoms between each episode. There are numerous environmental and biological causes of rhinosinusitis. Non-allergic rhinosinusitis, for example, can be caused by environmental irritants, medications, foods, hormonal changes, and/or nasal septum deviation. Triggers of allergic rhinitis can include exposure to seasonal allergens, perennial allergens that occur any time of year, and/or occupational allergens. Accordingly, rhinosinusitis affects millions of people and is a leading cause for patients to seek medical care.

The invention recognizes that knowing certain properties of tissue, both active and passive, at a given target site within the nasal anatomy prior to and during electrotherapeutic treatment (i.e., neuromodulation, ablation, etc.) provides an ability to more precisely target a tissue of interest (i.e., targeted tissue) and minimize or prevent collateral damage to surrounding, non-targeted tissue. The invention provides systems and methods with an ability to tune and provide precision targeting and comprehensive treatment of a tissue of interest in a nasal region of a patient for the treatment of rhinosinusitis, while minimizing or avoiding collateral damage to surrounding, non-targeted tissue.

For example, certain target sites within the nasal cavity intended to undergo treatment may consist of more than one type of tissue (i.e., nerves, muscles, bone, blood vessels, etc.). In particular, a tissue of interest (i.e., the specific tissue to undergo treatment), such as neural tissue, may be adjacent to one or more tissues that are not of interest (i.e., tissue that is not intended to undergo treatment). In one scenario, a surgeon may wish to provide electrotherapeutic stimulation to a nerve tissue, while avoiding the delivery of any such stimulation to surrounding, adjacent tissue at the target site, as unintended collateral damage may result in damage to non-targeted tissue and cause further complications. At the same time, it is critical that the appropriate amount of energy is delivered to the targeted tissue to ensure that the entirety of the targeted tissue receives such stimulation to elicit the desired effect. In such a scenario, the specific type of targeted neural tissue may generally dictate the level of electrical stimulation required to elicit a desired effect. Furthermore, physical properties of the targeted tissue, including the specific location and depth of the targeted neural tissue, in relation to the non-targeted tissue, further impacts the level of electrical stimulation necessary to result in effective therapeutic treatment.

The invention provides systems and methods with an ability to characterize, prior to an electrotherapeutic treatment, the type of tissue at a target site by sensing at least one of physiological properties, bioelectric properties, and thermal properties of tissue, wherein such characterization includes identifying specific types of tissue present at the target site. For example, different tissue types include different physiological and histological characteristics. As a result of the different characteristics, different tissue types have different associated bioelectrical properties and thus exhibit different behavior in response to application of energy applied thereto.

By knowing such properties of a given tissue, the systems and methods are configured to determine a specific pulsed energy treatment pattern for controlling delivery of pulsed energy to the targeted tissue which is sufficient to maintain a consistent temperature during treatment to ensure successful and complete ablation and/or modulation of the targeted tissue, while minimizing or entirely preventing unintended collateral damage to non-targeted tissue. In particular, a given pulsed energy treatment pattern may include, for example, a specific ablation profile, including a predetermined treatment time, a precise level of energy to be delivered, and a predetermined temperature range for that particular tissue. As a result, the ablation profile (i.e., specific level of energy, treatment time, and temperature range) is precisely tuned to the targeted tissue (based on the known and characterized properties of the targeted tissue), such that the appropriate amount of energy is delivery and the entirety of the targeted tissue receives adequate treatment while unintended collateral damage to surrounding or adjacent non-targeted tissue is minimized or prevented.

The systems and methods are further configured to receive and process real-time feedback data associated with the targeted tissue undergoing treatment to further ensure that energy delivered is maintained within the scope of the pulsed energy treatment pattern. More specifically, the systems and methods are configured to automatically control delivery of energy to the targeted tissue based on the processing of the real-time feedback data, wherein such data includes at least temperature measurement data associated with the targeted tissue collected during delivery of energy to the targeted tissue. The controller is configured to process temperature measurement data to detect whether the temperature at the target site, notably a temperature reading of the targeted tissue undergoing treatment, falls within a predetermined temperature range (wherein such temperature range is associated with a successful treatment of the given targeted tissue to elicit the desired effect).

In turn, the controller is configured to automatically control the delivery of energy to the targeted tissue based on real-time monitoring of feedback data, most notably temperature measurement data, to ensure the desired ablation/modulation is achieved. The controller is configured to cause delivery of pulsed energy to the targeted tissue, wherein the pulsed energy is at a specific level and an associated duty cycle for a specific period of time based on feedback data. The pulsed energy is sufficient to maintain the temperature of the targeted tissue within the appropriate range so as to cause ablation and/or modulation thereof, while further preventing, in some instances, irreversible tissue damage.

As a result, the systems and methods are able to ensure that optimal energy is delivered until the target ablation/modulation depth is achieved, while maintaining clinically relevant treatment time. This is particularly important with respect to electrotherapeutic treatment in the nasal cavity, as intermittent electrode-tissue contact may result in coagulum forming due to fluctuating waveforms, thereby causing a treatment process to be less effective as certain portions of a treatment device (i.e., electrodes) may have inconsistent delivery of energy. The systems and methods ensure repeatable and controllable delivery of treatment energy, specifically pulsed energy, from each of a plurality of electrodes in order to achieve the desired surgical outcome.

It should be noted that, although many of the embodiments are described with respect to devices, systems, and methods for therapeutically modulating nerves in the nasal region for the treatment of rhinitis, other applications and other embodiments in addition to those described herein are within the scope of the present disclosure. For example, at least some embodiments of the present disclosure may be useful for the treatment of other indications, such as the treatment of chronic sinusitis and epistaxis. In particular, the embodiments described herein may be configured to treat allergic rhinitis, non-allergic rhinitis, chronic rhinitis, acute rhinitis, chronic sinusitis, acute sinusitis, chronic rhinosinusitis, acute rhinosinusitis, and/or medical resistant rhinitis.

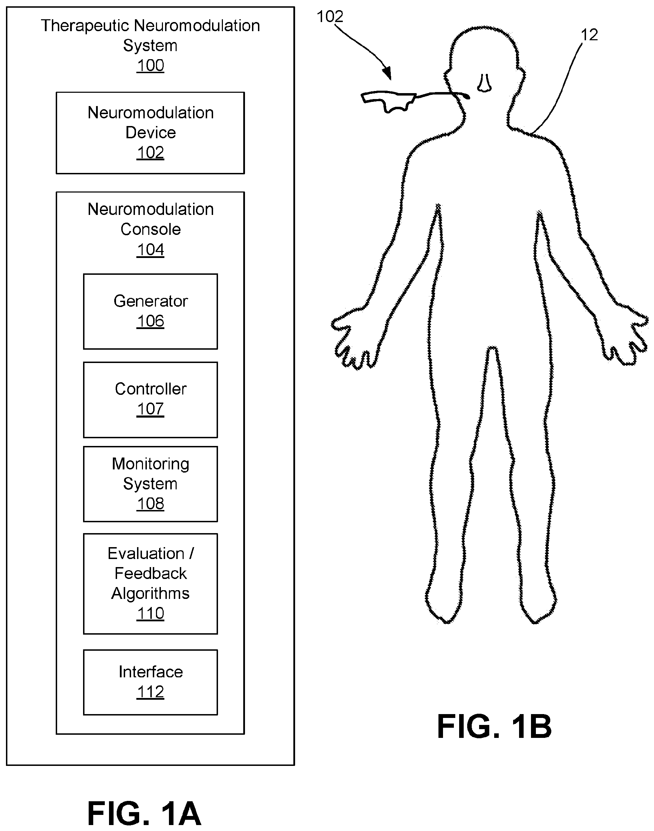

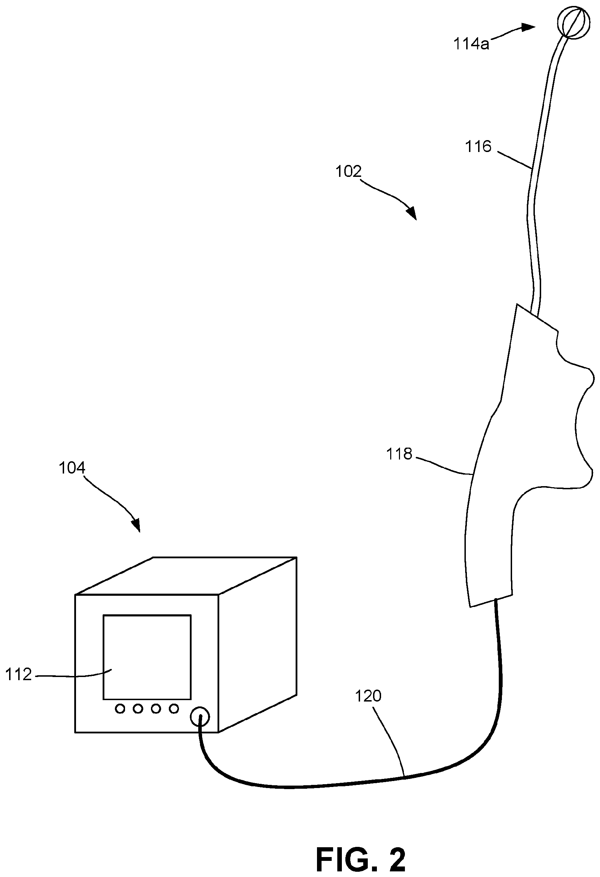

FIGS. 1A and 1B are diagrammatic illustrations of a therapeutic system 100 for treating a condition of a patient using a handheld device 102 according to some embodiments of the present disclosure. The system 100 generally includes a device 102 and a console 104 to which the device 102 is to be connected. FIGS. 2 and 3 are diagrammatic illustrations of the console 104 coupled to the handheld neuromodulation device 102 illustrating two different embodiments of an end effector (end effector 114a and end effector 114b) for delivering energy to tissue at the one or more target sites within the nasal cavity. For ease of description, the end effector embodiments 114a and 114b may be collectively referred to as "end effector 114" in the following description. As illustrated, the device 102 is a handheld device, which includes end effector 114, a shaft 116 operably associated with the end effector 114, and a handle 118 operably associated with the shaft 116. The end effector 114 may be collapsible/retractable and expandable, thereby allowing for the end effector 114 to be minimally invasive (i.e., in a collapsed or retracted state) upon delivery to one or more target sites within a patient and then expanded once positioned at the target site. It should be noted that the terms "end effector" and "therapeutic assembly" may be used interchangeably throughout this disclosure.

For example, a surgeon or other medical professional performing a procedure can utilize the handle 118 to manipulate and advance the shaft 116 to a desired target site, wherein the shaft 116 is configured to locate at least a distal portion thereof intraluminally at a treatment or target site within a portion of the patient associated with tissue to undergo electrotherapeutic stimulation for subsequent treatment of an associated condition or disorder. In the event that the tissue to be treated is a nerve, such that electrotherapeutic stimulation thereof results in treatment of an associated neurological condition, the target site may generally be associated with peripheral nerve fibers. The target site may be a region, volume, or area in which the target nerves are located and may differ in size and shape depending upon the anatomy of the patient. Once positioned, the end effector 114 may be deployed and subsequently deliver energy to the one or more target sites. The energy delivered may be non-therapeutic stimulating energy at a frequency for locating neural tissue and further sensing one or more properties of the neural tissue. For example, the end effector 114 may include an electrode array, which includes at least a subset of electrodes configured to sense the presence of neural tissue at a respective position of each of the electrodes, as well as morphology of the neural tissue, wherein such data may be used for determining, via the console 104, the type of neural tissue, depth of neural tissue, and location of neural tissue.

Based on the identification of the neural tissue type, the console 104 is configured to determine a specific treatment pattern for controlling delivery of energy from the end effector 114 upon the target site at a specific level for a specific period of time to the tissue of interest (i.e., the targeted tissue) sufficient to ensure successful ablation/modulation of the targeted tissue while minimizing and/or preventing collateral damage to surrounding or adjacent non-targeted tissue at the target site (such as surface tissue adjacent to underlying targeted neural tissue). Accordingly, the end effector 114 is able to therapeutically modulating nerves of interest, particularly nerves associated with a peripheral neurological conditional or disorder so as to treat such condition or disorder, while minimizing and/or preventing collateral damage to adjacent surface tissue at the one or more target sites.

For example, the end effector 114 may include at least one energy delivery element, such as an electrode, configured to delivery energy to the target tissue which may be used for sensing presence and/or specific properties of tissue (such tissue including, but not limited to, muscle, nerves, blood vessels, bones, etc.) for therapeutically modulating tissues of interest, such as neural tissue. For example, one or more electrodes may be provided by one or more portions of the end effector 114, wherein the electrodes may be configured to apply electromagnetic neuromodulation energy (e.g., radiofrequency (RF) energy) to target sites. In other embodiments, the end effector 114 may include other energy delivery elements configured to provide therapeutic neuromodulation using various other modalities, such as cryotherapeutic cooling, ultrasound energy (e.g., high intensity focused ultrasound ("HIFU") energy), microwave energy (e.g., via a microwave antenna), direct heating, high and/or low power laser energy, mechanical vibration, and/or optical power.

In some embodiments, the end effector 114 may include one or more sensors (not shown), such as one or more temperature sensors (e.g., thermocouples, thermistors, etc.), impedance sensors, and/or other sensors. The sensors and/or the electrodes may be connected to one or more wires extending through the shaft 116 and configured to transmit signals to and from the sensors and/or convey energy to the electrodes.

As shown, the device 102 is operatively coupled to the console 104 via a wired connection, such as cable 120. It should be noted, however, that the device 102 and console 104 may be operatively coupled to one another via a wireless connection. The console 104 is configured to provide various functions for the device 102, which may include, but is not limited to, controlling, monitoring, supplying, and/or otherwise supporting operation of the device 102. For example, when the device 102 is configured for electrode-based, heat-element-based, and/or transducer-based treatment, the console 104 may include an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy.

In some embodiments, the console 104 may include a controller 107 communicatively coupled to the device 102. However, in the embodiments described herein, the controller 107 may generally be carried by and provided within the handle 118 of the device 102. The controller 107 is configured to initiate, terminate, and/or adjust operation of one or more electrodes provided by the end effector 114 directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via evaluation/feedback algorithms 110. For example, the evaluation/feedback algorithms 110 can be configured to provide information associated with the location of nerves at the treatment site, the temperature of the tissue at the treatment site, and/or the effect of the therapeutic neuromodulation on the nerves at the treatment site. In certain embodiments, the evaluation/feedback algorithm 110 can include features to confirm efficacy of the treatment and/or enhance the desired performance of the system 100. For example, the evaluation/feedback algorithm 110, in conjunction with the controller 107, can be configured to monitor temperature at the treatment site during therapy and automatically shut off the energy delivery when the temperature reaches a predetermined maximum (e.g., when applying RF energy) or predetermined minimum (e.g., when applying cryotherapy). In other embodiments, the evaluation/feedback algorithm 110, in conjunction with the controller 107, can be configured to automatically terminate treatment after a predetermined maximum time, a predetermined maximum impedance rise of the targeted tissue (i.e., in comparison to a baseline impedance measurement), a predetermined maximum impedance of the targeted tissue, and/or other threshold values for biomarkers associated with autonomic function. This and other information associated with the operation of the system 100 can be communicated to the operator via a graphical user interface (GUI) 112 provided via a display on the console 104 and/or a separate display (not shown) communicatively coupled to the console 104, such as a tablet or monitor. The GUI 112 may generally provide operational instructions for the procedure, such as indicating when the device 102 is primed and ready to perform treatment and further providing status of therapy during the procedure, including indicating when the treatment is complete.

For example, as previously described, the end effector 114 and/or other portions of the system 100 can be configured to detect various parameters of a tissue of interest at the target site to determine the anatomy at the target site (e.g., tissue types, tissue locations, vasculature, bone structures, foramen, sinuses, etc.), locate nerves and/or other structures, and allow for neural mapping. For example, the end effector 114 may be configured to detect impedance, dielectric properties, temperature, and/or other properties that indicate the presence of neural tissue or fibers in the target region, as described in greater detail herein.

As shown in FIG. 1A, the console 104 further includes a monitoring system 108 configured to receive data from the end effector 114 (i.e., detected electrical and/or thermal measurements of tissue at the target site), specifically sensed by appropriate sensors (e.g., temperature sensors and/or impedance sensors, or the like), and process this information to identify the presence of nerves, the location of nerves, neural activity at the target site, and/or other properties of the neural tissue, such a physiological properties (e.g., depth), bioelectric properties, and thermal properties. The nerve monitoring system 108 can be operably coupled to the electrodes and/or other features of the end effector 114 via signal wires (e.g., copper wires) that extend through the cable 120 and through the length of the shaft 116. In other embodiments, the end effector 114 can be communicatively coupled to the nerve monitoring system 108 using other suitable communication means.

The nerve monitoring system 108 can determine neural locations and activity before therapeutic neuromodulation to determine precise treatment regions corresponding to the positions of the desired nerves. The nerve monitoring system 108 can further be used during treatment to determine the effect of the therapeutic neuromodulation, and/or after treatment to evaluate whether the therapeutic neuromodulation treated the target nerves to a desired degree. This information can be used to make various determinations related to the nerves proximate to the target site, such as whether the target site is suitable for neuromodulation. In addition, the nerve monitoring system 108 can also compare the detected neural locations and/or activity before and after therapeutic neuromodulation, and compare the change in neural activity to a predetermined threshold to assess whether the application of therapeutic neuromodulation was effective across the treatment site. For example, the nerve monitoring system 108 can further determine electroneurogram (ENG) signals based on recordings of electrical activity of neurons taken by the end effector 114 before and after therapeutic neuromodulation. Statistically meaningful (e.g., measurable or noticeable) decreases in the ENG signal(s) taken after neuromodulation can serve as an indicator that the nerves were sufficiently ablated. Additional features and functions of the nerve monitoring system 108, as well as other functions of the various components of the console 104, including the evaluation/feedback algorithms 110 for providing real-time feedback capabilities for ensuring optimal therapy for a given treatment is administered, are described in at least U.S. Publication No. 2016/0331459 and U.S. Publication No. 2018/0133460.

As will be described in greater detail herein, the neuromodulation device 102 provides access to target sites deep within the nasal region, such as at the immediate entrance of parasympathetic fibers into the nasal cavity to therapeutically modulate autonomic activity within the nasal cavity. In certain embodiments, for example, the neuromodulation device 102 can position the end effector 114 into contact with target sites within nasal cavity associated with postganglionic parasympathetic fibers that innervate the nasal mucosa.

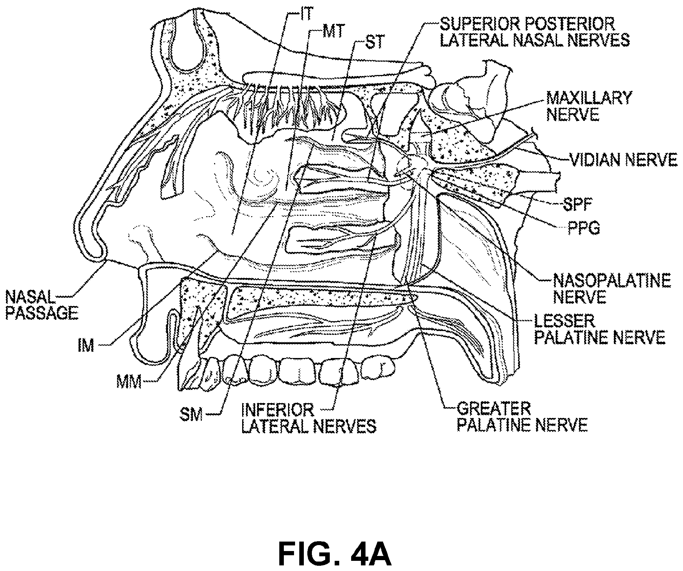

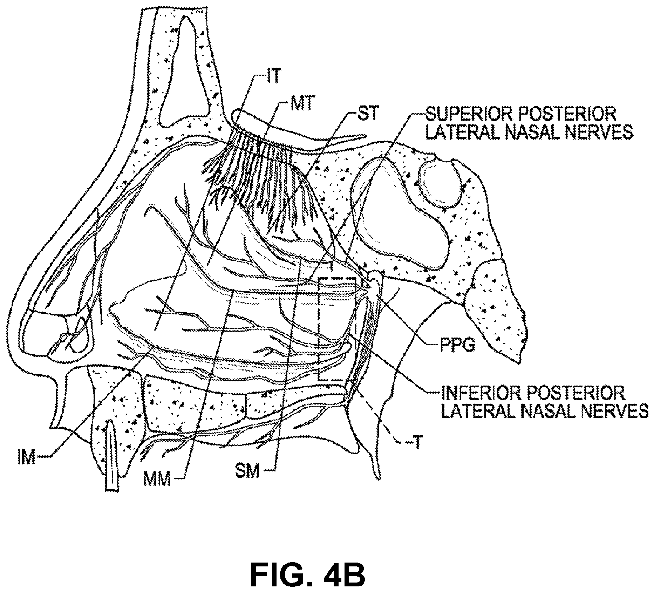

FIG. 4A is a cut-away side view illustrating the anatomy of a lateral nasal wall and FIG. 4B is an enlarged side view of the nerves of the lateral nasal wall of FIG. 3A. The sphenopalatine foramen (SPF) is an opening or conduit defined by the palatine bone and the sphenoid bone through which the sphenopalatine vessels and the posterior superior nasal nerves travel into the nasal cavity. More specifically, the orbital and sphenoidal processes of the perpendicular plate of the palatine bone define the sphenopalatine notch, which is converted into the SPF by the articulation with the surface of the body of the sphenoid bone.

The location of the SPF is highly variable within the posterior region of the lateral nasal cavity, which makes it difficult to visually locate the SPF. Typically, the SPF is located in the middle meatus (MM). However, anatomical variations also result in the SPF being located in the superior meatus (SM) or at the transition of the superior and middle meatuses. In certain individuals, for example, the inferior border of the SPF has been measured at about 19 mm above the horizontal plate of the palatine bone (i.e., the nasal sill), which is about 13 mm above the horizontal lamina of the inferior turbinate (IT) and the average distance from the nasal sill to the SPF is about 64.4 mm, resulting in an angle of approach from the nasal sill to the SPA of about 11.4°. However, studies to measure the precise location of the SPF are of limited practical application due to the wide variation of its location.

The anatomical variations of the SPF are expected to correspond to alterations of the autonomic and vascular pathways traversing into the nasal cavity. In general, it is thought that the posterior nasal nerves (also referred to as lateral posterior superior nasal nerves) branch from the pterygopalatine ganglion (PPG), which is also referred to as the sphenopalatine ganglion, through the SPF to enter the lateral nasal wall of the nasal cavity, and the sphenopalatine artery passes from the pterygopalatine fossa through the SPF on the lateral nasal wall. The sphenopalatine artery branches into two main portions: the posterior lateral nasal branch and the posterior septal branch. The main branch of the posterior lateral nasal artery travels inferiorly into the inferior turbinate IT (e.g., between about 1.0 mm and 1.5 mm from the posterior tip of the inferior turbinate IT), while another branch enters the middle turbinate MT and branches anteriorly and posteriorly.

Beyond the SPF, studies have shown that over 30% of human patients have one or more accessory foramen that also carries arteries and nerves into the nasal cavity. The accessory foramen are typically smaller than the SPF and positioned inferior to the SPF. For example, there can be one, two, three or more branches of the posterior nasal artery and posterior nasal nerves that extend through corresponding accessory foramen. The variability in location, size, and quantity associated with the accessory foramen and the associated branching arteries and nerves that travel through the accessory foramen gives rise to a great deal of uncertainty regarding the positions of the vasculature and nerves of the sphenopalatine region. Furthermore, the natural anatomy extending from the SPF often includes deep inferior and/or superior grooves that carry neural and arterial pathways, which make it difficult to locate arterial and neural branches. For example the grooves can extend more than 5 mm long, more than 2 mm wide, and more than 1 mm deep, thereby creating a path significant enough to carry both arteries and nerves. The variations caused by the grooves and the accessory foramen in the sphenopalatine region make locating and accessing the arteries and nerves (positioned posterior to the arteries) extremely difficult for surgeons.

Recent microanatomic dissection of the pterygopalatine fossa (PPF) have further evidenced the highly variable anatomy of the region surrounding the SPF, showing that a multiplicity of efferent rami that project from the pterygopalatine ganglion (PPG) to innervate the orbit and nasal mucosa via numerous groups of small nerve fascicles, rather than an individual postganglionic autonomic nerves (e.g., the posterior nasal nerve). Studies have shown that at least 87% of humans have microforamina and micro rami in the palatine bone.

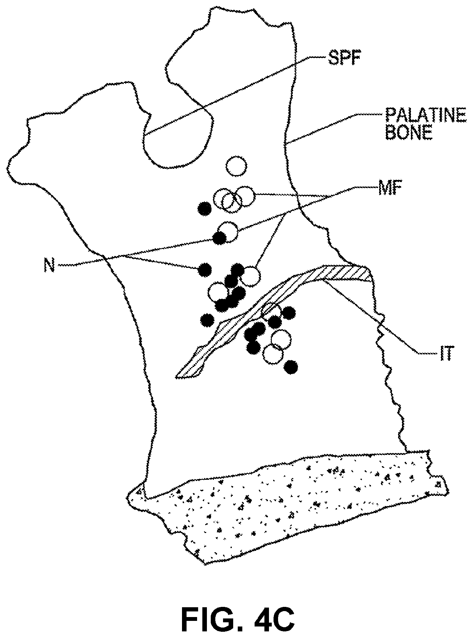

FIG. 4C, for example, is a front view of a left palatine bone illustrating geometry of microforamina and micro rami in a left palatine bone. In FIG. 34, the solid regions represent nerves traversing directly through the palatine bone, and the open circles represent nerves that were associated with distinct microforamina. As such, FIG. 4C illustrates that a medial portion of the palatine bone can include at least 25 accessory posterolateral nerves.

The respiratory portion of the nasal cavity mucosa is composed of a type of ciliated pseudostratified columnar epithelium with a basement membrane. Nasal secretions (e.g., mucus) are secreted by goblet cells, submucosal glands, and transudate from plasma. Nasal seromucous glands and blood vessels are highly regulated by parasympathetic innervation deriving from the vidian and other nerves. Parasympathetic (cholinergic) stimulation through acetylcholine and vasoactive intestinal peptide generally results in mucus production. Accordingly, the parasympathetic innervation of the mucosa is primarily responsible submucosal gland activation/hyper activation, venous engorgement (e.g., congestion), and increased blood flow to the blood vessels lining the nose. Accordingly, severing or modulating the parasympathetic pathways that innervate the mucosa are expected to reduce or eliminate the hyper activation of the submucosal glands and engorgement of vessels that cause symptoms associated with rhinosinusitis and other indications.

As previously described herein, postganglionic parasympathetic fibers that innervate the nasal mucosa (i.e., posterior superior nasal nerves) were thought to travel exclusively through the SPF as a sphenopalatine neurovascular bundle. The posterior nasal nerves are branches of the maxillary nerve that innervate the nasal cavity via a number of smaller medial and lateral branches extending through the mucosa of the superior and middle turbinates ST, MT (i.e., nasal conchae) and to the nasal septum. The nasopalatine nerve is generally the largest of the medial posterior superior nasal nerves, and it passes anteroinferiorly in a groove on the vomer to the floor of the nasal cavity. From here, the nasopalatine nerve passes through the incisive fossa of the hard palate and communicates with the greater palatine nerve to supply the mucosa of the hard palate. The posterior superior nasal nerves pass through the pterygopalatine ganglion PPG without synapsing and onto the maxillary nerve via its ganglionic branches.

Based on the understanding that the posterior nasal nerves exclusively traverse the SPF to innervate the nasal mucosa, surgeries have been performed to selectively sever the posterior nasal nerve as it exits the SPF. However, as discussed above, the sinonasal parasympathetic pathway actually comprises individual rami project from the pterygopalatine ganglion (PPG) to innervate the nasal mucosa via multiple small nerve fascicles (i.e., accessory posterolateral nerves), not a single branch extending through the SPF. These rami are transmitted through multiple fissures, accessory foramina, and microforamina throughout the palatine bone and may demonstrate anastomotic loops with both the SPF and other accessory nerves. Thus, if only the parasympathetic nerves traversing the SPF were severed, almost all patients (e.g., 90% of patients or more) would retain intact accessory secretomotor fibers to the posterolateral mucosa, which would result in the persistence of symptoms the neurectomy was meant to alieve.

Accordingly, embodiments of the present disclosure are configured to therapeutically modulate nerves at precise and focused treatment sites corresponding to the sites of rami extending through fissures, accessory foramina, and microforamina throughout the palatine bone (e.g., target region T shown in FIG. 4B). In certain embodiments, the targeted nerves are postganglionic parasympathetic nerves that go on to innervate the nasal mucosa. This selective neural treatment is also expected to decrease the rate of postoperative nasal crusting and dryness because it allows a clinician to titrate the degree of anterior denervation through judicious sparing of the rami orbitonasal. Furthermore, embodiments of the present disclosure are also expected to maintain at least some sympathetic tone by preserving a portion of the sympathetic contributions from the deep petrosal nerve and internal maxillary periarterial plexus, leading to improved outcomes with respect to nasal obstruction. In addition, embodiments of the present disclosure are configured to target a multitude of parasympathetic neural entry locations (e.g., accessory foramen, fissures, and microforamina) to the nasal region to provide for a complete resection of all anastomotic loops, thereby reducing the rate of long-term re-innervation.

Furthermore, embodiments of the present disclosure are configured to therapeutically modulate non-neural tissue at precise and focused treatment sites corresponding to targeted structures associated with mucus producing and/or mucosal engorgement elements, including, but not limited to, nasal mucosa and related mucus glands. For example, nasal congestion, or "stuffiness", occurs when nasal tissues and blood vessels that line the passages inside the nasal cavity become swollen with excess fluid, thereby causing a "stuffy" feeling. More specifically, certain mucus producing and/or mucosal engorgement elements within the nasal cavity are responsible for causing nasal congestion. As previously described, such elements include nasal mucosa and related mucus glands, which are responsible for producing mucus in response to neural signals, as well as associated blood vessels which may become engorged and swollen as a result of increased blood flow as a result of irritation or inflammation of nasal tissues. In certain embodiments, that targeted structures to receive therapeutic modulation (i.e., receive delivery of energy) include, for example, blood vessels associated with mucus glands, as will be described in greater detail herein.

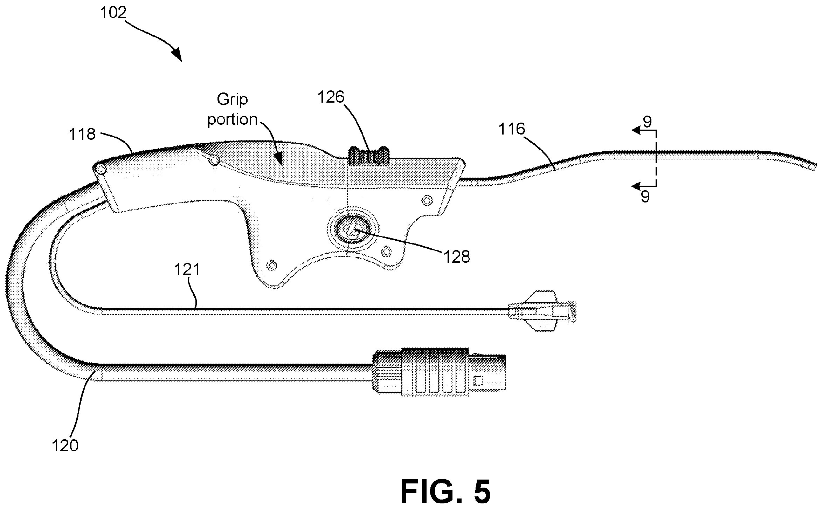

FIG. 5 is a side view of one embodiment of a handheld device 102 for providing therapeutic nasal neuromodulation consistent with the present disclosure. As previously described, the device 102 includes an end effector (not shown) transformable between a collapsed/retracted configuration and an expanded deployed configuration, a shaft 116 operably associated with the end effector, and a handle 118 operably associated with the shaft 116. The handle 118 includes at least a first mechanism 126 for deployment of the end effector from collapsed/retracted configuration to the expanded, deployed configuration, and a second mechanism 128, separate from the first mechanism 124, for control of energy output by the end effector, specifically electrodes or other energy elements provided by the end effector. The handheld device 102 may further include an auxiliary line 121, which may provide a fluid connection between a fluid source, for example, and the shaft 116 such that fluid may be provided to a target site via the distal end of the shaft 116. In some embodiments, the auxiliary line 121 may provide a connection between a vacuum source and the shaft 116, such that the device 102 may include suction capabilities (via the distal end of the shaft 116).

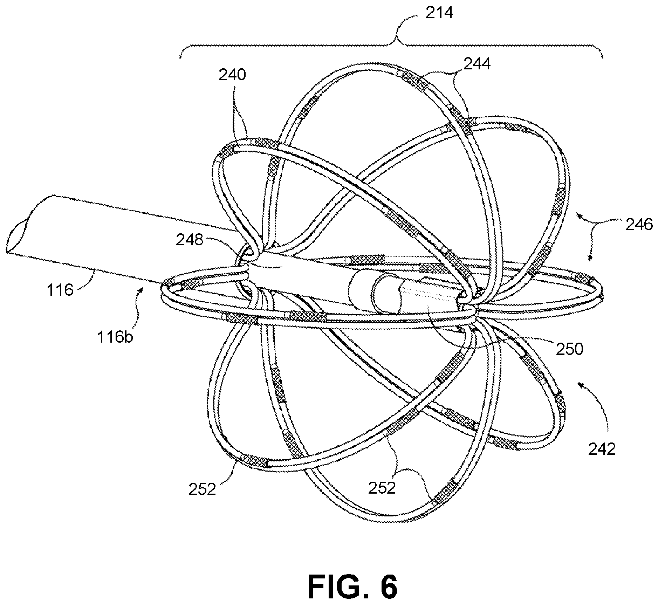

FIG. 6 is an enlarged, perspective view of one embodiment of an end effector 214 consistent with the present disclosure. As shown, the end effector 214 is generally positioned at a distal portion 116b of the shaft 116. The end effector 214 is transformable between a low-profile delivery state to facilitate intraluminal delivery of the end effector 214 to a treatment site within the nasal region and an expanded state, as shown. The end effector 214 includes a plurality of struts 240 that are spaced apart from each other to form a frame or basket 242 when the end effector 214 is in the expanded state. The struts 240 can carry one or more energy delivery elements, such as a plurality of electrodes 244. In the expanded state, the struts 240 can position at least two of the electrodes 244 against tissue at a target site within the nasal region (e.g., proximate to the palatine bone inferior to the SPF). The electrodes 244 can apply bipolar or multi-polar RF energy to the target site to therapeutically modulate postganglionic parasympathetic nerves that innervate the nasal mucosa proximate to the target site. In various embodiments, the electrodes 244 can be configured to apply pulsed RF energy with a desired duty cycle (e.g., 1 second on/0.5 seconds off) to regulate the temperature increase in the target tissue.

In the embodiment illustrated in FIG. 6, the basket 242 includes eight branches 246 spaced radially apart from each other to form at least a generally spherical structure, and each of the branches 246 includes two struts 240 positioned adjacent to each other. In other embodiments, however, the basket 242 can include fewer than eight branches 246 (e.g., two, three, four, five, six, or seven branches) or more than eight branches 246. In further embodiments, each branch 246 of the basket 242 can include a single strut 240, more than two struts 240, and/or the number of struts 240 per branch can vary. In still further embodiments, the branches 246 and struts 240 can form baskets or frames having other suitable shapes for placing the electrodes 244 in contact with tissue at the target site. For example, when in the expanded state, the struts 240 can form an ovoid shape, a hemispherical shape, a cylindrical structure, a pyramid structure, and/or other suitable shapes.

The end effector 214 can further include an internal or interior support member 248 that extends distally from the distal portion 116b of the shaft 116. A distal end portion 250 of the support member 248 can support the distal end portions of the struts 240 to form the desired basket shape. For example, the struts 240 can extend distally from the distal potion 116b of the shaft 116 and the distal end portions of the struts 240 can attach to the distal end portion 250 of the support member 248. In certain embodiments, the support member 248 can include an internal channel (not shown) through which electrical connectors (e.g., wires) coupled to the electrodes 244 and/or other electrical features of the end effector 214 can run. In various embodiments, the internal support member 248 can also carry an electrode (not shown) at the distal end portion 250 and/or along the length of the support member 248.

The basket 242 can transform from the low-profile delivery state to the expanded state (shown in FIG. 6) by either manually manipulating a handle of the device 102, interacting with the first mechanism 126 for deployment of the end effector 214 from collapsed/retracted configuration to the expanded, deployed configuration, and/or other feature at the proximal portion of the shaft 116 and operably coupled to the basket 242. For example, to move the basket 242 from the expanded state to the delivery state, an operator can push the support member 248 distally to bring the struts 240 inward toward the support member 248. An introducer or guide sheath (not shown) can be positioned over the low-profile end effector 214 to facilitate intraluminal delivery or removal of the end effector 214 from or to the target site. In other embodiments, the end effector 214 is transformed between the delivery state and the expanded state using other suitable means, such as the first mechanism 126, as will be described in greater detail herein.

The individual struts 240 can be made from a resilient material, such as a shape-memory material (e.g., Nitinol) that allows the struts 240 to self-expand into the desired shape of the basket 242 when in the expanded state. In other embodiments, the struts 240 can be made from other suitable materials and/or the end effector 214 can be mechanically expanded via a balloon or by proximal movement of the support member 248. The basket 242 and the associated struts 240 can have sufficient rigidity to support the electrodes 244 and position or press the electrodes 244 against tissue at the target site. In addition, the expanded basket 242 can press against surrounding anatomical structures proximate to the target site (e.g., the turbinates, the palatine bone, etc.) and the individual struts 240 can at least partially conform to the shape of the adjacent anatomical structures to anchor the end effector 214 at the treatment site during energy delivery. In addition, the expansion and conformability of the struts 240 can facilitate placing the electrodes 244 in contact with the surrounding tissue at the target site.

At least one electrode 244 is disposed on individual struts 240. In the illustrated embodiment, two electrodes 244 are positioned along the length of each strut 240. In other embodiments, the number of electrodes 244 on individual struts 240 be only one, more than two, zero, and/or the number of electrodes 244 on the different struts 240 can vary. The electrodes 244 can be made from platinum, iridium, gold, silver, stainless steel, platinum-iridium, cobalt chromium, iridium oxide, polyethylenedioxythiophene ("PEDOT"), titanium, titanium nitride, carbon, carbon nanotubes, platinum grey, Drawn Filled Tubing ("DFT") with a silver core made by Fort Wayne Metals of Fort Wayne, Ind., and/or other suitable materials for delivery RF energy to target tissue.

In certain embodiments, each electrode 244 can be operated independently of the other electrodes 244. For example, each electrode can be individually activated and the polarity and amplitude of each electrode can be selected by an operator or a control algorithm (e.g., executed by the controller 107 of FIG. 1A). Various embodiments of such independently controlled electrodes 244 are described in greater detail herein. The selective independent control of the electrodes 244 allows the end effector 214 to deliver RF energy to highly customized regions and to further create multiple micro-lesions to selectively modulate a target neural structure by effectively causing multi-point interruption of a neural signal due to the multiple micro-lesions. For example, a select portion of the electrodes 244 can be activated to target neural fibers in a specific region while the other electrodes 244 remain inactive. In certain embodiments, for example, electrodes 244 may be activated across the portion of the basket 242 that is adjacent to tissue at the target site, and the electrodes 244 that are not proximate to the target tissue can remain inactive to avoid applying energy to non-target tissue. Such configurations facilitate selective therapeutic modulation of nerves on the lateral nasal wall within one nostril without applying energy to structures in other portions of the nasal cavity.

The electrodes 244 can be electrically coupled to an RF generator (e.g., the generator 106 of FIG. 1A) via wires (not shown) that extend from the electrodes 244, through the shaft 116, and to the RF generator. When each of the electrodes 244 is independently controlled, each electrode 244 couples to a corresponding wire that extends through the shaft 116. In other embodiments, multiple electrodes 244 can be controlled together and, therefore, multiple electrodes 244 can be electrically coupled to the same wire extending through the shaft 116. The RF generator and/or components operably coupled (e.g., a control module) thereto can include custom algorithms to control the activation of the electrodes 244. For example, the RF generator can deliver RF power at about 200-300 W to the electrodes 244, and do so while activating the electrodes 244 in a predetermined pattern selected based on the position of the end effector 214 relative to the treatment site and/or the identified locations of the target nerves. In other embodiments, the RF generator delivers power at lower levels (e.g., less than 15 W, 15-50 W, 50-150 W, etc.) and/or higher power levels.

The end effector 214 can further include one or more sensors 252 (e.g., temperature sensors, impedance sensors, etc.) disposed on the struts 240 and/or other portions of the end effector 214 and configured to sense/detect one or more properties associated with neural tissue. For example, temperature sensors are configured to detect the temperature adjacent thereto. The sensors 252 can be electrically coupled to a console (e.g., the console 104 of FIG. 1A) via wires (not shown) that extend through the shaft 116. In various embodiments, the sensors 252 can be positioned proximate to the electrodes 244 to detect various properties of the neural tissue and/or the treatment associated therewith. As will be described in greater detail herein, the sensed data can be provided to the console 104, wherein such data is generally related to at least the presence of neural tissue at a given location (in which electrodes 244 and/or sensors 252 are present at the target site(s)) and depth of identified neural tissue, as well as other physiological, bioelectric, and/or thermal properties. In turn, the console 104 (via the controller 107, monitoring system 108, and evaluation/feedback algorithms 110) is configured to process such data and determine a level of therapeutic energy to be delivered by one or more the plurality of electrodes such that the energy delivered at each position by the one or more electrodes is sufficient to ablate neural tissue at each position and minimize and/or prevent damage to a surrounding or adjacent structure or tissue, such as surface tissue adjacent to underlying targeted neural tissue, or an artery or arterial wall adjacent to the neural tissue at each position.

Such sensed data can further include feedback data associated with the effect of the therapeutic neuromodulation on neural tissue at any given location. For example, feedback data (sensed during therapeutic neuromodulation of neural tissue) may be associated with efficacy of ablation of the neural tissue at each position during and/or after delivery of initial energy from one or more of the plurality of electrodes. Accordingly, in certain embodiments, the console 104 (via the controller 107, monitoring system 108, and evaluation/feedback algorithms 110) is configured to process such feedback data to determine if certain properties of the neural tissue undergoing treatment (i.e., tissue temperature, tissue impedance, current density, etc.) reach predetermined thresholds for irreversible tissue damage. For example, in some embodiments, as described in greater detail herein, the console 104 (via the controller 107, monitoring system 108, and evaluation/feedback algorithms 110) is configured to determine if tissue temperature (of the neural tissue undergoing treatment) falls outside of a predetermined temperature range (i.e., falling either below a low set point or rising above a high set point). The controller 107 can tune energy output individually for the one or more electrodes after an initial level of energy has been delivered based, at least in part, on feedback data. For example, once the threshold is reached, the application of therapeutic neuromodulation energy can be terminated to allow the tissue to remain intact. In certain embodiments, the energy delivery can automatically be tuned based on an evaluation/feedback algorithm (e.g., the evaluation/feedback algorithm 110 of FIG. 1A) stored on a console (e.g., the console 104 of FIG. 1A) operably coupled to the end effector 214.

FIGS. 7A-7F are various views of another embodiment of an end effector 314 consistent with the present disclosure. As generally illustrated, the end effector 314 is a multi-segmented end effector, which includes at least a first segment 322 and a second segment 324 spaced apart from one another. The first segment 322 is generally positioned closer to a distal portion of the shaft 116, and is thus sometimes referred to herein as the proximal segment 322, while the second segment 324 is generally positioned further from the distal portion of the shaft 116 and is thus sometimes referred to herein as the distal segment 324. Each of the first and second segments 322 and 324 is transformable between a retracted configuration, which includes a low-profile delivery state to facilitate intraluminal delivery of the end effector 314 to a treatment site within the nasal region, and a deployed configuration, which includes an expanded state, as shown in the figures.

FIG. 7A is an enlarged, perspective view of the multi-segment end effector illustrating the first (proximal) segment 322 and second (distal) segment 324. FIG. 7B is an exploded, perspective view of the multi-segment end effector 314. FIG. 7C is an enlarged, top view of the multi-segment end effector 314. FIG. 7D is an enlarged, side view of the multi-segment end effector 314. FIG. 7E is an enlarged, front (proximal facing) view of the first (proximal) segment 322 of the multi-segment end effector 314 and FIG. 7F is an enlarged, front (proximal facing) view of the second (distal) segment 324 of the multi-segment end effector 314.

As illustrated, the first segment 322 includes at least a first set of flexible support elements, generally in the form of wires, arranged in a first configuration, and the second segment 324 includes a second set of flexible support elements, also in the form of wires, arranged in a second configuration. The first and second sets of flexible support elements include composite wires having conductive and elastic properties. For example, in some embodiments, the composite wires include a shape memory material, such as nitinol. The flexible support elements may further include a highly lubricious coating, which may allow for desirable electrical insulation properties as well as desirable low friction surface finish. Each of the first and second segments 322, 324 is transformable between a retracted configuration and an expanded deployed configuration such that the first and second sets of flexible support elements are configured to position one or more electrodes provided on the respective segments (see electrodes 336 in FIGS. 7E and 7F) into contact with one or more target sites when in the deployed configuration.

As shown, when in the expanded deployed configuration, the first set of support elements of the first segment 322 includes at least a first pair of struts 330a, 330b, each comprising a loop (or leaflet) shape and extending in an upward direction and a second pair of struts 332a, 332b, each comprising a loop (or leaflet) shape and extending in a downward direction, generally in an opposite direction relative to at least the first pair of struts 330a, 330b. It should be noted that the terms upward and downward are used to describe the orientation of the first and second segments 322, 324 relative to one another. More specifically, the first pair of struts 330a, 330b generally extend in an outward inclination in a first direction relative to a longitudinal axis of the multi-segment end effector 314 and are spaced apart from one another. Similarly, the second pair of struts 332a, 332b extend in an outward inclination in a second direction substantially opposite the first direction relative to the longitudinal axis of the multi-segment end effector and spaced apart from one another.

The second set of support elements of the second segment 324, when in the expanded deployed configuration, includes a second set of struts 334(1), 334(2), 334(n) (approximately six struts), each comprising a loop shape extending outward to form an open-ended circumferential shape. As shown, the open-ended circumferential shape generally resembles a blooming flower, wherein each looped strut 334 may generally resemble a flower petal. It should be noted that the second set of struts 334 may include any number of individual struts and is not limited to six, as illustrated. For example, in some embodiments, the second segment 124 may include two, three, four, five, six, seven, eight, nine, ten, or more struts 334.

The first and second segments 322, 324, specifically struts 330, 332, and 334 include one or more energy delivery elements, such as a plurality of electrodes 336. It should be noted that any individual strut may include any number of electrodes 336 and is not limited to one electrode, as shown. In the expanded state, the struts 330, 332, and 334 can position any number of electrodes 336 against tissue at a target site within the nasal region (e.g., proximate to the palatine bone inferior to the SPF). The electrodes 336 can apply bipolar or multi-polar radiofrequency (RF) energy to the target site to therapeutically modulate postganglionic parasympathetic nerves that innervate the nasal mucosa proximate to the target site. In various embodiments, the electrodes 336 can be configured to apply pulsed RF energy with a desired duty cycle (e.g., 1 second on/0.5 seconds off) to regulate the temperature increase in the target tissue.

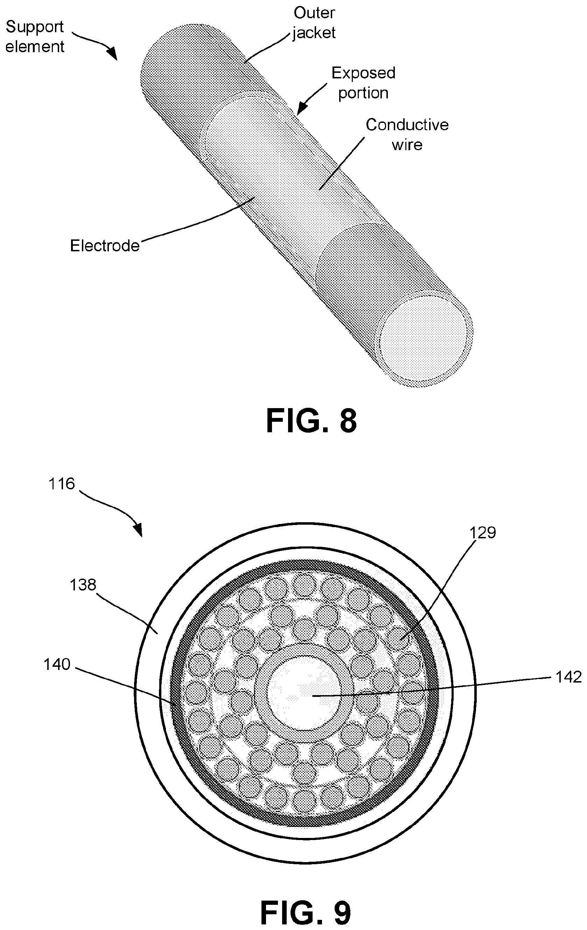

The first and second segments 322, 324 and the associated struts 330, 332, and 334 can have sufficient rigidity to support the electrodes 336 and position or press the electrodes 336 against tissue at the target site. In addition, each of the expanded first and second segments 322, 324 can press against surrounding anatomical structures proximate to the target site (e.g., the turbinates, the palatine bone, etc.) and the individual struts 330, 332, 334 can at least partially conform to the shape of the adjacent anatomical structures to anchor the end effector 314. In addition, the expansion and conformability of the struts 330, 332, 334 can facilitate placing the electrodes 336 in contact with the surrounding tissue at the target site. The electrodes 336 can be made from platinum, iridium, gold, silver, stainless steel, platinum-iridium, cobalt chromium, iridium oxide, polyethylenedioxythiophene (PEDOT), titanium, titanium nitride, carbon, carbon nanotubes, platinum grey, Drawn Filled Tubing (DFT) with a silver core, and/or other suitable materials for delivery RF energy to target tissue. In some embodiments, such as illustrated in FIG. 8, a strut may include an outer jacket surrounding a conductive wire, wherein portions of the outer jacket are selectively absent along a length of the strut, thereby exposing the underlying conductive wire so as to act as an energy delivering element (i.e., an electrode) and/or sensing element, as described in greater detail herein.

In certain embodiments, each electrode 336 can be operated independently of the other electrodes 336. For example, each electrode can be individually activated and the polarity and amplitude of each electrode can be selected by an operator or a control algorithm (e.g., executed by the controller 107 previously described herein). The selective independent control of the electrodes 336 allows the end effector 314 to deliver RF energy to highly customized regions. For example, a select portion of the electrodes 336 can be activated to target neural fibers in a specific region while the other electrodes 336 remain inactive. In certain embodiments, for example, electrodes 336 may be activated across the portion of the second segment 324 that is adjacent to tissue at the target site, and the electrodes 336 that are not proximate to the target tissue can remain inactive to avoid applying energy to non-target tissue. Such configurations facilitate selective therapeutic modulation of nerves on the lateral nasal wall within one nostril without applying energy to structures in other portions of the nasal cavity.

The electrodes 336 are electrically coupled to an RF generator (e.g., the generator 106 of FIG. 1A) via wires (not shown) that extend from the electrodes 336, through the shaft 116, and to the RF generator. When each of the electrodes 336 is independently controlled, each electrode 336 couples to a corresponding wire that extends through the shaft 116. In other embodiments, multiple electrodes 336 can be controlled together and, therefore, multiple electrodes 336 can be electrically coupled to the same wire extending through the shaft 116. As previously described, the RF generator and/or components operably coupled (e.g., a control module) thereto can include custom algorithms to control the activation of the electrodes 336. For example, the RF generator can deliver RF power at about 460-480 kHz (+ or - 5 kHz) to the electrodes 336, and do so while activating the electrodes 336 in a predetermined pattern selected based on the position of the end effector 314 relative to the treatment site and/or the identified locations of the target nerves. It should further be noted that the electrodes 336 may be individually activated and controlled (i.e., controlled level of energy output and delivery) based, at least in part, on feedback data. The RF generator is able to provide bipolar low power (10 watts with maximum setting of 50 watts) RF energy delivery, and further provide multiplexing capabilities (across a maximum of 30 channels).

Once deployed, the first and second segments 322, 324 contact and conform to a shape of the respective locations, including conforming to and complementing shapes of one or more anatomical structures at the respective locations. In turn, the first and second segments 322, 324 become accurately positioned within the nasal cavity to subsequently deliver, via one or more electrodes 336, precise and focused application of RF thermal energy to the one or more target sites to thereby therapeutically modulate associated neural tissue. More specifically, the first and second segments 322, 324 have shapes and sizes when in the expanded configuration that are specifically designed to place portions of the first and second segments 322, 324, and thus one or more electrodes associated therewith 336, into contact with target sites within nasal cavity associated with postganglionic parasympathetic fibers that innervate the nasal mucosa.

For example, the first set of flexible support elements of the first segment 322 conforms to and complements a shape of a first anatomical structure at the first location when the first segment 322 is in the deployed configuration and the second set of flexible support elements of the second segment 124 conforms to and complements a shape of a second anatomical structure at the second location when the second segment is in the deployed configuration. The first and second anatomical structures may include, but are not limited to, inferior turbinate, middle turbinate, superior turbinate, inferior meatus, middle meatus, superior meatus, pterygopalatine region, pterygopalatine fossa, sphenopalatine foramen, accessory sphenopalatine foramen(ae), and sphenopalatine micro-foramen(ae).

In some embodiments, the first segment 322 of the multi-segment end effector 314 is configured in a deployed configuration to fit around at least a portion of a middle turbinate at an anterior position relative to the middle turbinate and the second segment 324 of the multi-segment end effector is configured in a deployed configuration to contact a plurality of tissue locations in a cavity at a posterior position relative to the middle turbinate.

For example, the first set of flexible support elements of the first segment (i.e., struts 330 and 332) conforms to and complements a shape of a lateral attachment and posterior-inferior edge of the middle turbinate when the first segment 322 is in the deployed configuration and the second set of flexible support elements (i.e., struts 334) of the second segment 324 contact a plurality of tissue locations in a cavity at a posterior position relative to the lateral attachment and posterior-inferior edge of middle turbinate when the second segment 324 is in the deployed configuration. Accordingly, when in the deployed configuration, the first and second segments 322, 324 are configured to position one or more associated electrodes 336 at one or more target sites relative to either of the middle turbinate and the plurality of tissue locations in the cavity behind the middle turbinate. In turn, electrodes 336 are configured to deliver RF energy at a level sufficient to therapeutically modulate postganglionic parasympathetic nerves innervating nasal mucosa at an innervation pathway within the nasal cavity of the patient.

As illustrated in FIG. 7E, the first segment 322 comprises a bilateral geometry. In particular, the first segment 322 includes two identical sides, including a first side formed of struts 330a, 332a and a second side formed of struts 330b, 332b. This bilateral geometry allows at least one of the two sides to conform to and accommodate an anatomical structure within the nasal cavity when the first segment 322 is in an expanded state. For example, when in the expanded state, the plurality of struts 330a, 332a contact multiple locations along multiple portions of the anatomical structure and electrodes provided by the struts are configured to emit energy at a level sufficient to create multiple micro-lesions in tissue of the anatomical structure that interrupt neural signals to mucus producing and/or mucosal engorgement elements. In particular, struts 330a, 332a conform to and complement a shape of a lateral attachment and posterior-inferior edge of the middle turbinate when the first segment 322 is in the deployed configuration, thereby allowing for both sides of the anatomical structure to receive energy from the electrodes. By having this independence between first and second side (i.e., right and left side) configurations, the first segment 322 is a true bilateral device. By providing a bilateral geometry, the multi-segment end effector 314 does not require a repeat use configuration to treat the other side of the anatomical structure, as both sides of the structure are accounted at the same time due to the bilateral geometry. The resultant micro-lesion pattern can be repeatable and is predictable in both macro element (depth, volume, shape parameter, surface area) and can be controlled to establish low to high effects of each, as well as micro elements (the thresholding of effects within the range of the macro envelope can be controlled), as well be described in greater detail herein. The systems of the present invention are further able to establish gradients within allowing for control over neural effects without having widespread effect to other cellular bodies, as will be described in greater detail herein.

FIG. 8 is a cross-sectional view of a portion of the shaft 116 of the handheld device taken along lines 9-9 of FIG. 5. As illustrated, the shaft 116 may be constructed from multiple components so as to have the ability to constrain the end effector 314 in the retracted configuration (i.e., the low-profile delivery state) when the end effector 314 is retracted within the shaft 116, and to further provide an atraumatic, low profile and durable means to deliver the end effector 314 to the target site. The shaft 116 includes coaxial tubes which travel from the handle 118 to a distal end of the shaft 116. The shaft 116 assembly is low profile to ensure transnasal delivery of therapy. The shaft 116 includes an outer sheath 138, surrounding a hypotube 140, which is further assembled over electrode wires 129 which surround an inner lumen 142. The outer sheath 138 serves as the interface between the anatomy and the device 102. The outer sheath 138 may generally include a low friction PTFE liner to minimize friction between the outer sheath 138 and the hypotube 140 during deployment and retraction. In particular the outer sheath 138 may generally include an encapsulated braid along a length of the shaft 116 to provide flexibility while retaining kink resistance and further retaining column and/or tensile strength. For example, the outer sheath 138 may include a soft Pebax material, which is atraumatic and enables smooth delivery through the nasal passage.

The hypotube 140 is assembled over the electrode wires starting within the handle 118 and travelling to the proximal end of the end effector 314. The hypotube 140 generally acts to protect the wires during delivery and is malleable to enable flexibility without kinking to thereby improve trackability. The hypotube 140 provides stiffness and enables torqueability of the device 102 to ensure accurate placement of the end effector 314. The hypotube 140 also provides a low friction exterior surface which enables low forces when the outer sheath 138 moves relative to the hypotube 140 during deployment and retraction or constraint. The shaft 116 may be pre-shaped in such a manner so as to complement the nasal cavity. For example, the hypotube 140 may be annealed to create a bent shaft 116 with a pre-set curve. The hypotube 140 may include a stainless-steel tubing, for example, which interfaces with a liner in the outer sheath 138 for low friction movement.

The inner lumen 142 may generally provide a channel for fluid extraction during a treatment procedure. For example, the inner lumen 142 extends from the distal end of the shaft 116 through the hypotube 140 and to atmosphere via a fluid line (line 121 of FIG. 5). The inner lumen 142 materials are chosen to resist forces of external components acting thereon during a procedure.

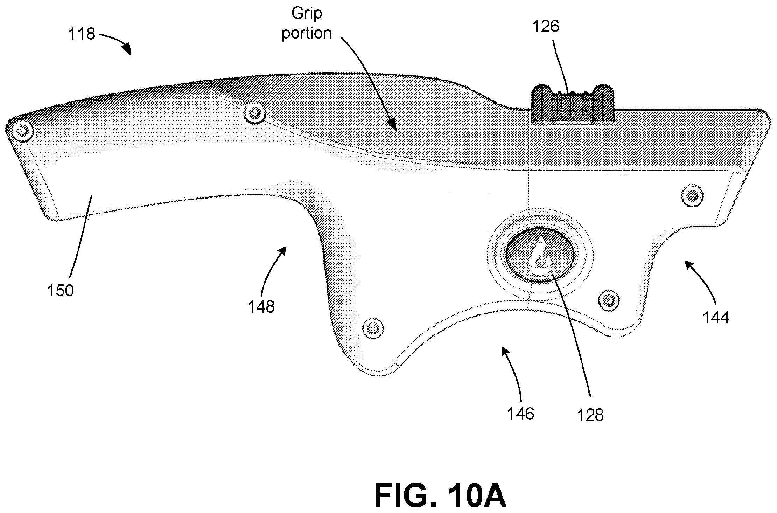

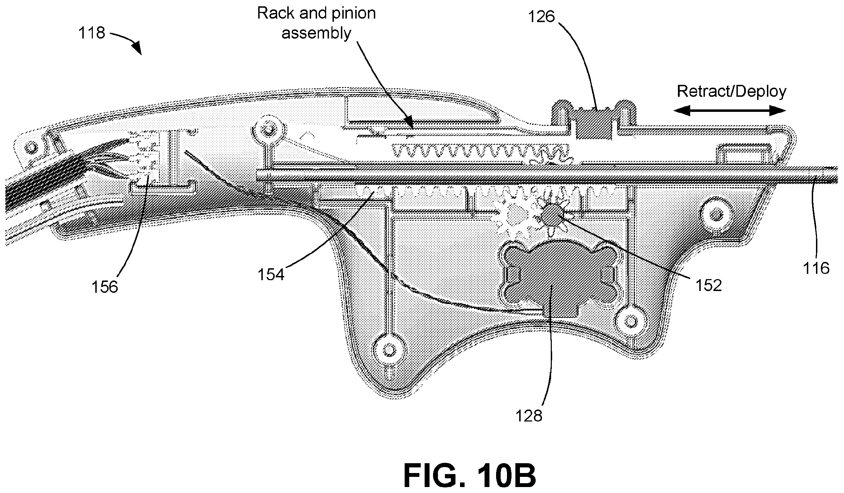

FIG. 10A is a side view of the handle of the handheld 118 and FIG. 10B is a side view of the handle 118 illustrating internal components enclosed within. The handle 118 generally includes an ergonomically-designed grip portion which provides ambidextrous use for both left and right handed use and conforms to hand anthropometrics to allow for at least one of an overhand grip style and an underhand grip style during use in a procedure. For example, the handle 118 may include specific contours, including recesses 144, 146, and 148 which are designed to naturally receive one or more of an operator's fingers in either of an overhand grip or underhand grip style and provide a comfortable feel for the operator. For example, in an underhand grip, recess 144 may naturally receive an operator's index finger, recess 146 may naturally receive an operator's middle finger, and recess 148 may naturally receive an operator's ring and little (pinkie or pinky) fingers which wrap around the proximal protrusion 150 and the operator's thumb naturally rests on a top portion of the handle 118 in a location adjacent to the first mechanism 126. In an overhand grip, the operator's index finger may naturally rest on the top portion of the handle 118, adjacent to the first mechanism 126, while recess 144 may naturally receive the operator's middle finger, recess 146 may naturally receive a portion of the operator's middle and/or ring fingers, and recess 148 may naturally receive and rest within the space (sometimes referred to as the purlicue) between the operator's thumb and index finger.

As previously described, the handle includes multiple user-operated mechanisms, including at least a first mechanism 126 for deployment of the end effector from the collapsed/retracted configuration to the expanded deployed configuration and a second mechanism 128 for controlling of energy output by the end effector, notably energy delivery from one or more electrodes. As shown, the user inputs for the first and second mechanisms 126, 128 are positioned a sufficient distance to one another to allow for simultaneous one-handed operation of both user inputs during a procedure. For example, user input for the first mechanism 126 is positioned on a top portion of the handle 118 adjacent the grip portion and user input for the second mechanism 128 is positioned on side portions of the handle 118 adjacent the grip portion. As such, in an underhand grip style, the operator's thumb rests on the top portion of the handle adjacent to the first mechanism 126 and at least their middle finger is positioned adjacent to the second mechanism 128, each of the first and second mechanisms 126, 128 accessible and able to be actuated. In an overhand grip system, the operator's index finger rests on the top portion of the handle adjacent to the first mechanism 126 and at least their thumb is positioned adjacent to the second mechanism 128, each of the first and second mechanisms 126, 128 accessible and able to be actuated. Accordingly, the handle accommodates various styles of grip and provides a degree of comfort for the surgeon, thereby further improving execution of the procedure and overall outcome.

Referring to FIG. 10B, the various components provided within the handle 118 are illustrated. As shown, the first mechanism 126 may generally include a rack and pinion assembly providing movement of end effector 314 between the retracted and deployed configurations in response to input from a user-operated controller. The rack and pinion assembly generally includes a set of gears 152 for receiving input from the user-operated controller and converting the input to linear motion of a rack member 154 operably associated with at least one of the shaft 116 and the end effector 314. The rack and pinion assembly comprises a gearing ratio sufficient to balance a stroke length and retraction and deployment forces, thereby improving control over the deployment of the end effector. As shown, the rack member 154 may be coupled to a portion of the shaft 116, for example, such that movement of the rack member 154 in a direction towards a proximal end of the handle 118 results in corresponding movement of the shaft 116 while the end effector 314 remains stationary, thereby exposing the end effector 314 and allowing the end effector 314 to transition from the constrained, retracted configuration to the expanded, deployed configuration. Similarly, upon movement of the rack member 154 in a direction towards a distal end of the handle 118 results in corresponding movement of the shaft 116 while the end effector 314 remains stationary, thereby enclosing the end effector 314 within the shaft 116. It should be noted that, in other embodiments, the rack member 154 may be directly coupled to a portion of the end effector 314 such that movement of the rack member 154 results in corresponding movement of the end effector 314 while the shaft 116 remains stationary, thereby transitioning the end effector 314 between the retracted and deployed configurations.

The user-operated controller associated with the first mechanism 126 may include a slider mechanism operably associated with the rack and pinion rail assembly. Movement of the slider mechanism in a rearward direction towards a proximal end of the handle results in transitioning of the end effector 314 to the deployed configuration and movement of the slider mechanism in a forward direction towards a distal end of the handle results in transitioning of the end effector to the retracted configuration. In other embodiment, the user-operated controller associated with the first mechanism 126 may include a scroll wheel mechanism operably associated with the rack and pinion rail assembly. Rotation of the wheel in a rearward direction towards a proximal end of the handle results in transitioning of the end effector to the deployed configuration and rotation of the wheel in a forward direction towards a distal end of the handle results in transitioning of the end effector to the retracted configuration.

The user-operated controller associated with the first mechanism 126 may generally provide a high degree of precision and control over the deployment (and retraction) of the first and second segments 322, 324. For example, in some instances, the operator may wish to only deploy the second segment 324 during the procedure, while the first segment 322 remains in the retracted configuration. The user-operated controller allows for an operator to provide a sufficient degree of input (i.e., slide the slider mechanism or scroll the scroll wheel to a specific position) which results in only the second segment 324 transitioning from the retracted configuration to the deployed configuration (while the first segment 322 remains enclosed within the shaft 116 and in the retracted configuration). For example, in some embodiments, the end effector 314 may further include a detent feature, such as a catch or similar element, positioned between the first and second segments 322, 324 and configured to provide a surgeon with feedback, such as tactile feedback, during deployment of the end effector segments, alerting the surgeon when at least the second segment 324 is fully deployed. In particular, as the surgeon slides the slider mechanism or scrolls the scroll wheel during deployment of the second segment 324, the detect feature (provided between the first and second segments 322, 324) may then reach a portion of the shaft 116 and cause an increase in resistance on the slider mechanism or scroll wheel, thereby indicating to the surgeon that the second segment 324 has been deployed and the first segment 322 remains in the retracted configuration. Accordingly, the surgeon can position and orient the second segment 324 as they desire without concern over the first segment 322 as it remains in the retracted configuration. In turn, one the second segment 324 is positioned at the desired target site, the surgeon may then deploy the first segment 322 to perform the procedure. Yet still, in some instances, only the second segment 324 may be used to perform a procedure (i.e., deliver energy to one or more target sites in contact with the second segment 324) and, as such, the first segment 322 may never be deployed.

The second mechanism 128 may generally include a user-operated controller configured to be actuated between at least an active position and an inactive position to thereby control delivery of energy from the end effector, notable delivery of energy from the electrodes. The user-operated controller may be multi-modal in that the user-operated controller may be actuated between multiple positions providing different functions/modes. For example, upon a single user input (i.e., single press of button associated within controller), the second mechanism may provide a baseline apposition / sensing check function prior to modulation. Upon pressing and holding the controller button for a pre-defined period of time, the energy output from the end effector may be activated. Further, upon double-tapping the controller button, energy output is deactivated.

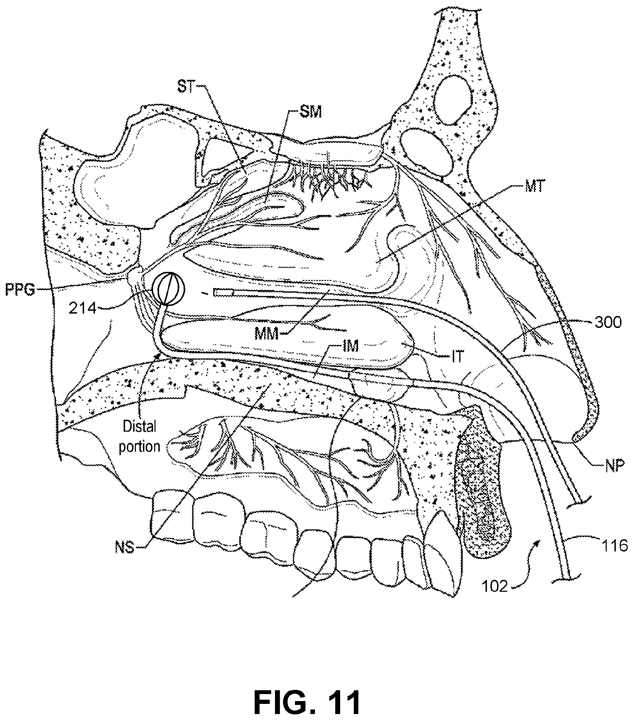

FIG. 11 is a partial cut-away side view illustrating one approach for delivering an end effector a target site within a nasal region in accordance with embodiments of the present disclosure. As shown, the end effector 214 is positioned within a nasal region. However, it should be noted that the following description related to the delivery and deployment of the end effector 214 also applies to end effector 314. The distal portion of the shaft 116 extends into the nasal passage NP, through the inferior meatus IM between the inferior turbinate IT and the nasal sill NS, and around the posterior portion of the inferior turbinate IT where the end effector 214 is deployed at a treatment site. The treatment site can be located proximate to the access point or points of postganglionic parasympathetic nerves (e.g., branches of the posterior nasal nerve and/or other parasympathetic neural fibers that innervate the nasal mucosa) into the nasal cavity. In other embodiments, the target site can be elsewhere within the nasal cavity depending on the location of the target nerves.

In various embodiments, the distal portion of the shaft 116 may be guided into position at the target site via a guidewire (not shown) using an over-the-wire (OTW) or a rapid exchange (RX) technique. For example, the end effector 214 can include a channel for engaging the guidewire. Intraluminal delivery of the end effector 214 can include inserting the guide wire into an orifice in communication with the nasal cavity (e.g., the nasal passage or mouth), and moving the shaft 116 and/or the end effector 214 along the guide wire until the end effector 214 reaches a target site (e.g., inferior to the SPF).

Yet still, in further embodiments, the neuromodulation device 102 can be configured for delivery via a guide catheter or introducer sheath (not shown) with or without using a guide wire. The introducer sheath can first be inserted intraluminally to the target site in the nasal region, and the distal portion of the shaft 116 can then be inserted through the introducer sheath. At the target site, the end effector 214 can be deployed through a distal end opening of the introducer sheath or a side port of the introducer sheath. In certain embodiments, the introducer sheath can include a straight portion and a pre-shaped portion with a fixed curve (e.g., a 5 mm curve, a 4 mm curve, a 3 mm curve, etc.) that can be deployed intraluminally to access the target site. In this embodiment, the introducer sheath may have a side port proximal to or along the pre-shaped curved portion through which the end effector 214 can be deployed. In other embodiments, the introducer sheath may be made from a rigid material, such as a metal material coated with an insulative or dielectric material. In this embodiment, the introducer sheath may be substantially straight and used to deliver the end effector 214 to the target site via a substantially straight pathway, such as through the middle meatus MM (FIG. 4A).

Image guidance may be used to aid the surgeon's positioning and manipulation of the distal portion of the shaft 116, as well as the deployment and manipulation of the end effector 214. For example, an endoscope 100 and/or other visualization device can be positioned to visualize the target site, the positioning of the end effector 214 at the target site, and/or the end effector 214 during therapeutic neuromodulation. The endoscope 100 may be delivered proximate to the target site by extending through the nasal passage NP and through the middle meatus MM between the inferior and middle turbinates IT and MT. From the visualization location within the middle meatus MM, the endoscope 100 can be used to visualize the treatment site, surrounding regions of the nasal anatomy, and the end effector 214.

In some embodiments, the distal portion of the shaft 116 may be delivered via a working channel extending through an endoscope, and therefore the endoscope can provide direct in-line visualization of the target site and the end effector 214. In other embodiments, an endoscope is incorporated with the end effector 114 and/or the distal portion of the shaft 116 to provide in-line visualization of the end effector 114 and/or the surrounding nasal anatomy. In other embodiments, image guidance can be provided with various other guidance modalities, such as image filtering in the infrared (IR) spectrum to visualize the vasculature and/or other anatomical structures, computed tomography (CT), fluoroscopy, ultrasound, optical coherence tomography (OCT), and/or combinations thereof. Yet still, in some embodiments, image guidance components may be integrated with the neuromodulation device 102 to provide image guidance during positioning of the end effector 214.

Once positioned at the target site, the therapeutic modulation may be applied via the one or more electrodes 244 and/or other features of the end effector 214 to precise, localized regions of tissue to induce one or more desired therapeutic neuromodulating effects to disrupt parasympathetic motor sensory function. The end effector 214 can selectively target postganglionic parasympathetic fibers that innervate the nasal mucosa at a target or treatment site proximate to or at their entrance into the nasal region. For example, the end effector 214 can be positioned to apply therapeutic neuromodulation at least proximate to the SPF (FIG. 4A) to therapeutically modulate nerves entering the nasal region via the SPF. The end effector 214 can also be positioned to inferior to the SPF to apply therapeutic neuromodulation energy across accessory foramen and microforamina (e.g., in the palatine bone) through which smaller medial and lateral branches of the posterior superior lateral nasal nerve enter the nasal region. The purposeful application of the energy at the target site may achieve therapeutic neuromodulation along all or at least a portion of posterior nasal neural fibers entering the nasal region. The therapeutic neuromodulating effects are generally a function of, at least in part, power, time, and contact between the energy delivery elements and the adjacent tissue. For example, in certain embodiments therapeutic neuromodulation of autonomic neural fibers are produced by applying RF energy at a power of about 2-20 W (e.g., 5 W, 7 W, 10 W, etc.) for a time period of about 1-20 sections (e.g., 5-10 seconds, 8-10 seconds, 10-12 seconds, etc.).

The therapeutic neuromodulating effects may include partial or complete denervation via thermal ablation and/or non-ablative thermal alteration or damage (e.g., via sustained heating and/or resistive heating). Desired thermal heating effects may include raising the temperature of target neural fibers above a desired threshold to achieve non-ablative thermal alteration, or above a higher temperature to achieve ablative thermal alteration. For example, the target temperature may be above body temperature (e.g., approximately 37° C.) but less than about 90° C. (e.g., 70-75° C.) for non-ablative thermal alteration, or the target temperature may be about 100° C. or higher (e.g., 110° C., 120° C., etc.) for the ablative thermal alteration. Desired non-thermal neuromodulation effects may include altering the electrical signals transmitted in a nerve.

Sufficiently modulating at least a portion of the parasympathetic nerves is expected to slow or potentially block conduction of autonomic neural signals to the nasal mucosa to produce a prolonged or permanent reduction in nasal parasympathetic activity. This is expected to reduce or eliminate activation or hyperactivation of the submucosal glands and venous engorgement and, thereby, reduce or eliminate the symptoms of rhinosinusitis. Further, because the device 102 applies therapeutic neuromodulation to the multitude of branches of the posterior nasal nerves rather than a single large branch of the posterior nasal nerve branch entering the nasal cavity at the SPF, the device 102 provides a more complete disruption of the parasympathetic neural pathway that affects the nasal mucosa and results in rhinosinusitis. Accordingly, the device 102 is expected to have enhanced therapeutic effects for the treatment of rhinosinusitis and reduced re-innervation of the treated mucosa.

**In** other embodiments, the device 102 can be configured to therapeutically modulate nerves and/or other structures to treat different indications. For example, the device 102 can be used to therapeutically modulate nerves that innervate the para-nasal sinuses to treat chronic sinusitis. **In** further embodiments, the system 100 and the device 102 disclosed herein can be configured therapeutically modulate the vasculature within the nasal anatomy to treat other indications, such as epistaxis (i.e., excessive bleeding from the nose). For example, the system 100 and the device 102 devices described herein can be used to apply therapeutically effective energy to arteries (e.g., the sphenopalatine artery and its branches) as they enter the nasal cavity (e.g., via the SPF, accessory foramen, etc.) to partially or completely coagulate or ligate the arteries. In other embodiments, the system 100 and the device 102 can be configured to partially or completely coagulate or ligate veins and/or other vessels. For such embodiments in which the end effector ligates or coagulates the vasculature, the system 100 and device 102 would be modified to deliver energy at significantly higher power (e.g., about 100 W) and/or longer times (e.g., 1 minute or longer) than would be required for therapeutic neuromodulation.

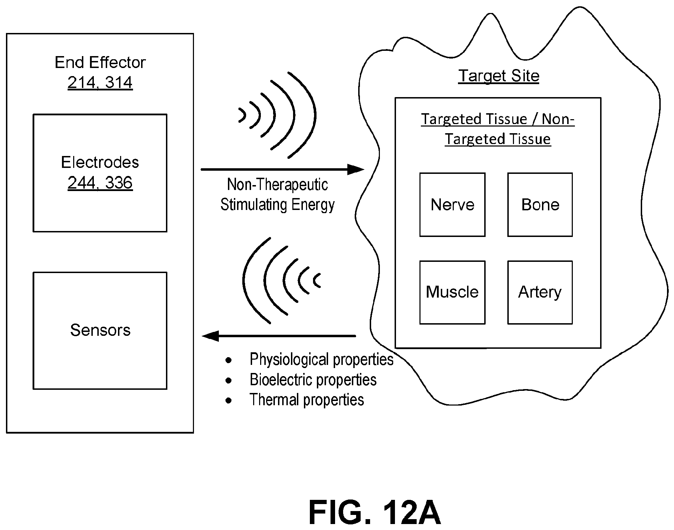

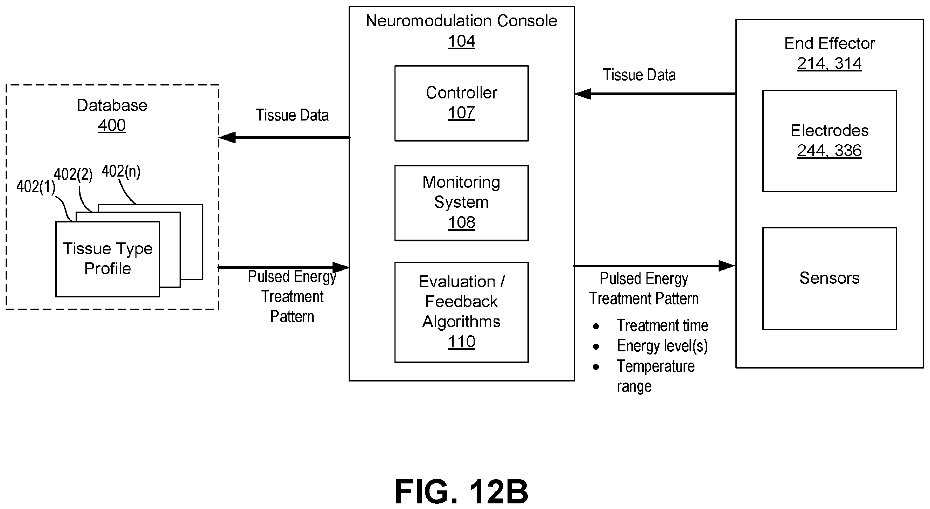

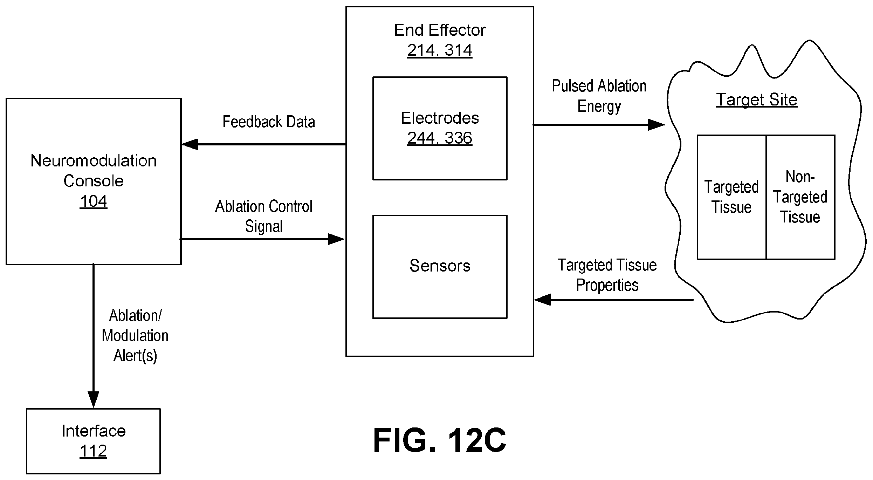

FIGS. 12A, 12B, and 12C are block diagrams illustrating the process of sensing, via an end effector, data associated with one or more tissues at a target site, notably bioelectric properties of one more tissues at the target site, and the subsequent processing of such data (via the controller 107, monitoring system 108, and evaluation/feedback algorithms 110) to determine the type of tissue(s) at the target site, determining a pulsed energy treatment pattern to be delivered by one or more of the plurality of electrodes of the end effector based on identified tissue types (as well as tissue location and/or depth), and subsequent receipt and processing of real-time feedback data associated with the targeted tissue undergoing treatment. The ablation energy associated with the pulsed energy treatment pattern is at a level sufficient to maintain a consistent temperature at the target site, most notably to maintain a stable temperature of the tissue undergoing treatment (i.e., the targeted neural tissue) to ensure that ablation and/or modulation of the targeted neural tissue occurs in entirety, while minimizing and/or preventing collateral damage to adjacent non-targeted tissue at the target site.

It should be noted that, while the block diagrams of FIGS. 12A, 12B, and 12C include reference to both end effector 214 and end effector 314, which are similarly configured with respect to sensing data associated with at least the presence of neural tissue and other properties of the neural tissue, including neural tissue depth. Accordingly, the following processes can be carried out by each end effector 214, 314, and other end effector embodiments described herein.

FIG. 12A is a block diagram illustrating delivery of non-therapeutic energy from electrodes 244, 336 of the end effector 214, 314, respectively, at a frequency/waveform for sensing one or more properties associated with one or more tissues at a target site in response to the non-therapeutic energy.

As previously described, the handheld treatment device includes an end effector comprising a micro-electrode array arranged about a plurality of struts. For example, end effector 214 includes a plurality of struts 240 that are spaced apart from each other to form a frame or basket 242 when the end effector 214 is in the expanded state. The struts 240 include a plurality of energy delivery elements, such as a plurality of electrodes 244. In the expanded state, each of the plurality of struts is able to conform to and accommodate an anatomical structure at a target site. When positioned, the struts may contact multiple locations along multiple portions of a target site and thereby position one or more electrodes 244 against tissue at a target site. At least a subset of electrodes is configured to deliver non-therapeutic stimulating energy at a frequency/waveform to respective positions at the target site to thereby sense the bioelectric properties of the one or more tissues at the target site, and further convey such data to the console 104. In addition to bioelectric properties, the data may also include at least one of physiological properties and thermal properties of tissue at the target site.

For example, upon delivering non-therapeutic stimulating energy (via one or more electrodes 244) to respective positions, various properties of the tissue at the one or more target sites can be detected. This information can then be transmitted to the console 104, particularly the controller 107, monitoring system 108, and evaluation/feedback algorithms 110 to determine the anatomy at the target site (e.g., tissue types, tissue locations, vasculature, bone structures, foramen, sinuses, etc.), locate a tissue of interest (targeted tissue to receive electric therapeutic stimulation), such as neural tissue, differentiate between different types of neural tissue, and map the anatomical and/or neural structure at the target site. For example, the end effector 214 can be used to detect resistance, complex electrical impedance, dielectric properties, temperature, and/or other properties that indicate the presence of neural fibers and/or other anatomical structures in the target region. In certain embodiments, the end effector 214, together with the console 104 components, can be used to determine resistance (rather than impedance) of the tissue (i.e., the load) to more accurately identify the characteristics of the tissue. For example, the evaluation/feedback algorithms 110 can determine resistance of the tissue by detecting the actual power and current of the load (e.g., via the electrodes 244).

In some embodiments, the system 100 provides resistance measurements with a high degree of accuracy and a very high degree of precision, such as precision measurements to the hundredths of an Ohm (e.g., 0.01Ω) for the range of 1-50Ω. The high degree of resistance detection accuracy provided by the system 100 allows for the detection sub-microscale structures, including the firing of neural tissue, differences between neural tissue and other anatomical structures (e.g., blood vessels), and even different types of neural tissue. This information can be analyzed by the evaluation/feedback algorithms 110 and/or the controller 107 and communicated to the operator via a high resolution spatial grid (e.g., on the display 112) and/or other type of display to identify neural tissue and other anatomy at the treatment site and/or indicate predicted neuromodulation regions based on the ablation pattern with respect to the mapped anatomy.

As previously described, in certain embodiments, each electrode 244 can be operated independently of the other electrodes 244. For example, each electrode can be individually activated and the polarity and amplitude of each electrode can be selected by an operator or a control algorithm executed by the controller 107. The selective independent control of the electrodes 244 allows the end effector 214 to detect information (i.e., the presence of neural tissue, depth of neural tissue, and other physiological and bioelectrical properties) and subsequently deliver RF energy to highly customized regions. For example, a select portion of the electrodes 244 can be activated to target specific neural fibers in a specific region while the other electrodes 244 remain inactive. In addition, the electrodes 244 can be individually activated to stimulate or therapeutically modulate certain regions in a specific pattern at different times (e.g., via multiplexing), which facilitates detection of anatomical parameters across a zone of interest and/or regulated therapeutic neuromodulation.

As previously described, the system 100 can identify tissue type of one or more tissues at a target site prior to therapy such that the therapeutic stimulation can be applied to precise regions including targeted tissue, while avoiding negative effects on non-targeted tissue and structures (e.g., surface tissue). For example, the system 100 can detect various bioelectrical parameters in an interest zone to determine the location and morphology of various tissue types (e.g., different types of neural tissue, neuronal directionality, etc.) and/or other tissue (e.g., glandular structures, vessels, bony regions, etc.). The system 100 is further configured to measure bioelectric potential.

To do so, one or more of the electrodes 244, 336 is placed in contact with an epithelial surface at a region of interest (e.g., a treatment site). Electrical stimuli (e.g., constant or pulsed currents at one or more frequencies, and/or alternating (sine, square, triangle, sawtooth, etc.) wave or direct constant current/power/voltage source at one or more frequencies) are applied to the tissue by one or more electrodes 244, 336 at or near the treatment site, and the voltage and/or current differences based on the wave applied at various different frequencies between various pairs of electrodes 244, 336 of the end effector 214, 314 may be measured to produce a spectral profile or map of the detected bioelectric potential, which can be used to identify different types of tissues (e.g., vessels, neural tissue, and/or other types of tissue) in the region of interest. For example, a fixed current (i.e., direct or alternating current) can be applied to a pair of electrodes 244, 336 adjacent to each other and the resultant voltages and/or currents between other pairs of adjacent electrodes 244, 336 are measured. Conversely, a fixed voltage (i.e. mono or bi-phasic) can be applied to a pair of electrodes 244, 336 adjacent to each other and the resultant current between other pairs of adjacent electrodes 244, 336 are measured. It will be appreciated that the current injection electrodes 244, 336 and measurement electrodes 244, 336 need not be adjacent, and that modifying the spacing between the two current injection electrodes 244, 336 can affect the depth of the recorded signals. For example, closely-spaced current injection electrodes 244, 336 provided recorded signals associated with tissue deeper from the surface of the tissue than further spaced apart current injection electrodes 244, 336 that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector), and this information can be used to map the neural and anatomical structures by the use of frequency differentiation reconstruction. In particular, current-voltage data may be observed with the difference in dielectric and conductive properties of tissue type when different levels of current frequencies are applied. Applying the stimuli at different frequencies will target different stratified layers or cellular bodies or clusters. At high signal frequencies (e.g., electrical injection or stimulation), for example, cell membranes of the neural tissue do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures within a given region or zone of a patient's body react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural tissue, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy.

In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes 244 at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., muscle, bone, blood vessels, surrounding or adjacent tissues, such as surface tissue adjacent to underlying targeted neural tissue at the target sites) because the targeted signal (having a frequency tuned to a target neural structure) will not have the same modulating effects on the non-target structures.

Accordingly, passive bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes 244, 336 and the adjacent tissue. The impedance or resistance measurements can also be used to detect whether the electrodes 244, 336 are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural tissue that are to be disrupted), anatomical landmarks, anatomical structures to avoid (e.g., vascular structures or neural tissue that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate neural fibers associated with certain neurological conditions or disorders to be treated. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

According, the system 100 is able to characterize, prior to an electrotherapeutic treatment, the type of tissue at a target site by sensing at least one of physiological properties, bioelectric properties, and thermal properties of tissue, wherein such characterization includes identifying specific types of tissue present at the target site. For example, different tissue types include different physiological and histological characteristics. As a result of the different characteristics, different tissue types have different associated bioelectrical properties and thus exhibit different behavior in response to application of energy applied thereto.

FIG. 12B is a block diagram illustrating communication of sensor data from the handheld device 102 to the controller and subsequent determination, via the controller, of a pulsed energy treatment pattern for controlling energy delivery based on the sensor data for precision targeting of tissue of interest and to be treated (i.e., neural tissue).

As previously described, by knowing such properties of a given tissue, the system 100 is configured to determine a specific pulsed energy treatment pattern for controlling delivery of pulsed energy to the targeted tissue which is sufficient to maintain a consistent temperature during treatment (consistent temperature of the targeted tissue during the treatment) to ensure successful and complete ablation and/or modulation of the targeted tissue.

As shown, the end effector 214, 314 communicates the tissue data (i.e., bioelectric properties of tissue at the target site) to the console 104. The tissue data may include physiological properties, bioelectric properties, and thermal properties. The bioelectric properties may include, but are not limited to, complex impedance, resistance, reactance, current density, capacitance, inductance, permittivity, conductivity, dielectric properties, muscle or nerve firing voltage, muscle or nerve firing current, depolarization, hyperpolarization, magnetic field, induced electromotive force, and combinations thereof. The dielectric properties may include, for example, at least a complex relative dielectric permittivity.

In turn, console 104 (via the controller 107, monitoring system 108, and evaluation/feedback algorithms 110) is configured to process such data and determine a type of tissue at the target site. The console 104 (via the controller 107, monitoring system 108, and evaluation/feedback algorithms 110) is further configured to determine a treatment pattern to be delivered by one or more of the plurality of electrodes of the end effector based, at least in part, on identified tissues. The treatment pattern (also referred to herein as "ablation pattern"), includes various parameters associated with the delivery of energy, including a predetermined treatment time, a precise level of energy to be delivered, and a predetermined temperature range for that particular tissue. The console 104 (via the controller 107, monitoring system 108, and evaluation/feedback algorithms 110) is configured to tune energy output (i.e., delivery of electrical therapeutic stimulation) based on the treatment pattern of a tissue of interest such that the energy delivered is at a specific frequency for a predetermined period of time and maintain tissue temperate within a predetermined temperature range, including a low set point (i.e., minimum temperature value) and a high set point (i.e., maximum temperature value), such that energy delivery is tuned and targeted to the tissue of interest to ensure complete treatment thereof.

The system 100 includes a database 400 containing a plurality of profiles 402(1)-402(n) of identified and known tissue types, wherein each profile includes electric signature data for the associated tissue type. The electric signature data generally includes previously identified bioelectric properties of the tissue type, including ablation profiles with known temperature range values associated with the modulation treatment of that particular tissue. Accordingly, the console 104 (via the controller 107, monitoring system 108, and evaluation/feedback algorithms 110) is configured to process data received from the end effector (i.e., physiological, bioelectric, and thermal properties of one or more tissues at the target site) and determine a type of tissue at the target site, and a treatment pattern for each of the one or more identified tissue types based on a comparison of the data with the electric signature data stored in each of the profiles 402. Upon a positive correlation between data sets, the console 104 is configured to identify a matching profile and thus determine the one or more tissue types at the target site and the respective treatment patterns of each.

A treatment pattern includes a predetermined treatment time, a precise level of energy to be delivered, and a predetermined temperature range for that particular tissue. As a result, the ablation profile (i.e., specific level of energy and treatment time) is precisely tuned to the targeted tissue (based on the known and characterized properties of the targeted tissue), such that the targeted tissue undergoes a complete and comprehensive treatment while unintended collateral damage to surrounding or adjacent non-targeted tissue is minimized or prevented.

FIG. 12C is a block diagram illustrating delivery of energy to the target site based on the treatment pattern output from the controller, monitoring of real-time feedback data associated with the targeted tissue undergoing treatment, and subsequent control over the delivery of energy based on the processing of the feedback data. Upon delivery energy from the electrodes to the targeted tissue (based on the treatment pattern), the device 102, via the electrodes/sensors is further configured to provide the console 104 with sensed data in the form of feedback data, in real-, or near-real, time. The real-time feedback data is associated with the effect of the therapeutic stimulation on the targeted tissue. For example, feedback data may be associated with efficacy of ablation upon targeted tissue (e.g., neural tissue) during and/or after delivery of initial energy from one or more of the plurality of electrodes. The console 104 (via the controller 107, monitoring system 108, and evaluation/feedback algorithms 110) is configured to process such real-time feedback data to determine if certain properties of the targeted tissue undergoing treatment (e.g., current density, tissue temperature, tissue impedance, etc.) reach predetermined thresholds for irreversible tissue damage.

More specifically, the console 104 (via the controller 107, monitoring system 108, and evaluation/feedback algorithms 110) is configured to automatically control delivery of energy to the targeted tissue based on the processing of the real-time feedback data, wherein such data includes temperature measurement data associated with the targeted tissue (and/or non-targeted tissue) collected during delivery of energy to the targeted tissue. The console 104 (via the controller 107, monitoring system 108, and evaluation/feedback algorithms 110) is configured to process temperature measurement data to determine whether the temperature falls outside of the predetermined temperature ranges (i.e., falls below a minimum set point or above a maximum set point). In turn, the controller 107 is configured to automatically control the delivery of energy to the targeted tissue based on real-time monitoring of feedback data, most notably temperature measurement data, to ensure the desired ablation/modulation is achieved. For example, temperature measurement readings a taken multiple times over a given period during the delivery of energy to the targeted tissue. Upon detecting an event in which the temperature falls outside of the predetermined temperature range (i.e., the temperature is lower than a minimum set point of the temperature range or the temperature is higher than a maximum set point of the temperature range), the application of therapeutic neuromodulation energy can be adjusted to compensate and cause the temperature of the targeted tissue to fall within the predetermined temperature range. For example, in certain embodiments, the energy delivery can automatically be tuned based on an evaluation/feedback algorithm (e.g., the evaluation/feedback algorithm 110 of FIG. 1A) stored on a console (e.g., the console 104 of FIG. 1A) operably coupled to the end effector 214 via specific controller hardware for providing pulsed energy delivery.

The controller 107 includes bang-bang control capabilities. For example, bang-bang controller (also known as a 2-step or on-off controller), also known as a hysteresis controller, is a feedback controller that switches abruptly between two states. The controller 107 may generally be configured to process temperature measurement data to detect whether the temperature at the target site, notably a temperature reading of the targeted tissue undergoing treatment, falls within a predetermined temperature range (i.e., above a minimum set point and below a maximum set point). By processing the real-time feedback data, specifically the continuously received temperature measurement data, the controller 107 is configured to automatically adjust energy output, by way of pulsed energy delivery, to either increase or decrease the targeted tissue temperature so as to cause the tissue temperature to fall within the predetermined temperature range, thereby maintaining a consistent temperature and ensuring the targeted tissue in its entirety receives adequate treatment.

The pulsed energy is at a specific level and an associated duty cycle for a specific period of time based on feedback data. The pulsed energy is sufficient to maintain the temperature of the targeted tissue within the appropriate range so as to cause ablation and/or modulation thereof, while further preventing, in some instances, irreversible tissue damage. For example, the predetermined temperature range may be between 50 °C and 60 °C. More specifically, the predetermined temperature range may be approximately 60 °C. Accordingly, the pulsed energy may be delivered from one or more electrodes at a level and an associated duty cycle sufficient to regulate and maintain temperature of the targeted tissue receiving the treatment energy at approximately 60 °C with a tolerance of approximately ±0.2 °C.

The electrodes 244, 336 are configured to be independently controlled and activated by the controller 107 (in conjunction with the evaluation/feedback algorithms 110) to thereby deliver energy independent of one another. Accordingly, the controller 107 can tune energy output individually for the one or more electrodes 244, 336 after an initial level of energy has been delivered based, at least in part, on feedback data. For example, based on the feedback data (i.e., temperature measurement data) the controller 107 can maintain, reduce, or increase energy output to a given electrode 244, 336 to ensure that the tissue temperature is within the predetermined temperature range, and for the predetermined time sufficient to cause adequate ablation and/or modulation of the targeted tissue. Accordingly, the energy delivery of any given electrode 244, 336 can automatically be tuned based on an evaluation/feedback algorithm (e.g., the evaluation/feedback algorithm 110 of FIG. 1A) stored on a console (e.g., the console 104 of FIG. 1A) operably coupled to the end effector 214. For example, at least some of the electrodes 244, 336 may have different levels of energy to be delivered at respective positions sufficient to ablate neural tissue at the respective positions based on the feedback data received for the respective locations.

The console 104 (via the controller 107, monitoring system 108, and evaluation/feedback algorithms 110) is further configured to transmit a signal resulting in an output, via interactive interface 112, of an alert to a user indicating a status of the efficacy of ablation/modulation of the targeted tissue. The alert may include, for example, a visual alert including at least one of a color and text displayed on a graphical user interface (GUI) and indicating whether the ablation/modulation is successful or unsuccessful, particularly with respect to respective sets of electrodes.

As a result, the systems and methods are able to ensure that optimal energy is delivered until the target ablation/modulation depth is achieved, while maintaining clinically relevant treatment time. This is particularly important with respect to electrotherapeutic treatment in the nasal cavity, as intermittent electrode-tissue contact may result in coagulum forming due to fluctuating waveforms, thereby causing a treatment process to be less effective as certain portions of a treatment device (i.e., electrodes) may have inconsistent delivery of energy. The systems and methods ensure repeatable and controllable delivery of treatment energy, specifically pulsed energy, from each of a plurality of electrodes in order to achieve the desired surgical outcome.

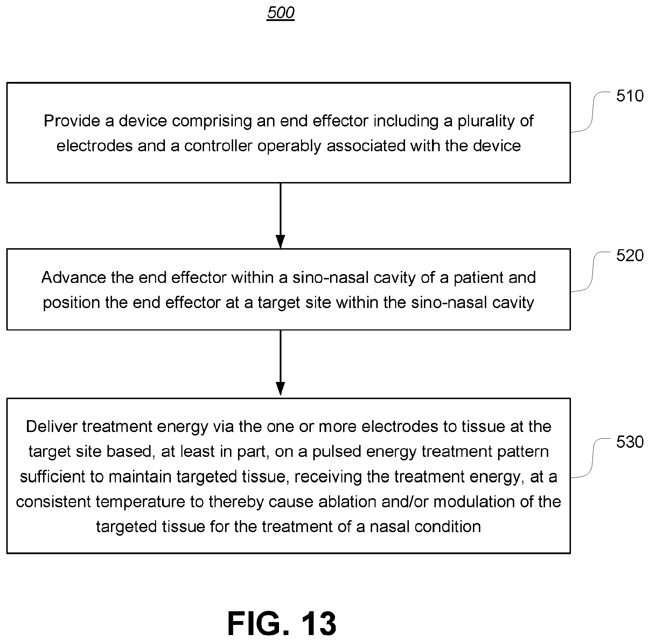

FIG. 13 is a flow diagram illustrating one embodiment of a method 500 for treating a condition within a sino-nasal cavity of a patient. The method 500 includes providing a device comprising an end effector including a plurality of electrodes and a controller operably associated with the device (operation 510). The method 500 further includes advancing the end effector within a sino-nasal cavity of a patient and positioning the end effector at a target site within the sino-nasal cavity (operation 520). The end effector generally includes a micro-electrode array arranged about a plurality of struts, wherein each of the plurality of struts is able to conform to and accommodate an anatomical structure within the nasal cavity. When positioned within the nasal cavity, the struts contact multiple locations along multiple portions of a target site.

The method 500 further includes delivering treatment energy to tissue at the target site based, at least in part, on a pulsed energy treatment pattern sufficient to maintain targeted tissue, receiving the treatment energy, at a consistent temperature to thereby cause modulation of the targeted tissue for the treatment of a nasal condition (operation 530).

The treatment energy is delivered via one or more electrodes of the micro-electrode array and supplied thereto from the controller. The pulsed energy treatment pattern is determined based on processing, via the controller, identifying data received from the end effector associated with tissue at the one or more target sites. The identifying data comprising a type of each of the one or more tissues. The micro-electrode array includes at least a subset of electrodes configured to deliver non-therapeutic stimulating energy at a frequency/waveform to respective positions at the one or more target sites to thereby sense at least one of physiological properties, bioelectric properties, and thermal properties of the one or more tissues at the target site. The processing of the identifying data, via the controller, includes comparing the identifying data received from the device with electric signature data associated with a plurality of known tissue types. The comparison includes correlating the identifying data received from the end effector with electric signature data from a supervised and/or an unsupervised trained neural network.

The pulsed energy treatment pattern includes a predetermined treatment time, a level of energy to be delivered from the electrodes, and a predetermined temperature range. The treatment energy is delivered based, at least in part, on processing, via the controller, of real-time feedback data associated with the one or more tissues upon supplying treatment energy thereto. The feedback data includes temperature measurement data associated with the targeted tissue, a level of energy delivered, and an elapsed delivery time. The controller is configured to process the feedback data using an algorithm to determine efficacy of ablation/modulation of the targeted tissue based, at least in part, on a comparison of the feedback data with pulsed energy treatment pattern data.

The controller is configured to process temperature measurement data to detect whether the temperature at the target site, notably a temperature reading of the targeted tissue undergoing treatment, falls within a predetermined temperature range (i.e., above a minimum set point and below a maximum set point) to thereby maintain a consistent temperature ensuring the targeted tissue in its entirety received adequate treatment. In turn, the controller is configured to automatically control the delivery of energy to the targeted tissue based on real-time monitoring of feedback data, most notably temperature measurement data, to ensure the desired ablation/modulation is achieved. The controller is configured to cause delivery of pulsed energy to the targeted tissue, wherein the pulsed energy is at a specific level and an associated duty cycle for a specific period of time based on feedback data. The pulsed energy is sufficient to maintain the temperature of the targeted tissue within the appropriate range so as to cause ablation and/or modulation thereof, while further preventing, irreversible tissue damage.

As a result, the systems and methods are able to ensure that optimal energy is delivered until the target ablation/modulation depth is achieved, while maintaining clinically relevant treatment time. This is particularly important with respect to electrotherapeutic treatment in the nasal cavity, as intermittent electrode-tissue contact may result in coagulum forming due to fluctuating waveforms, thereby causing a treatment process to be less effective as certain portions of a treatment device (i.e., electrodes) may have inconsistent delivery of energy. The systems and methods ensure repeatable and controllable delivery of treatment energy, specifically pulsed energy, from each of a plurality of electrodes in order to achieve the desired surgical outcome.

For example, in some embodiments, the energy delivered disrupts multiple neural signals to mucus producing and/or mucosal engorgement elements, thereby reducing production of mucus and/or mucosal engorgement within a nose of the patient and reducing or eliminating one or more symptoms associated with rhinosinusitis. In some embodiments, the targeted neural tissue is associated with one or more target sites proximate or inferior to a sphenopalatine foramen, wherein energy is delivered at a level sufficient to therapeutically modulate postganglionic parasympathetic nerves innervating nasal mucosa at foramina and/or microforamina of a palatine bone of the patient and causes multiple points of interruption of neural branches extending through foramina and/or microforamina of palatine bone.

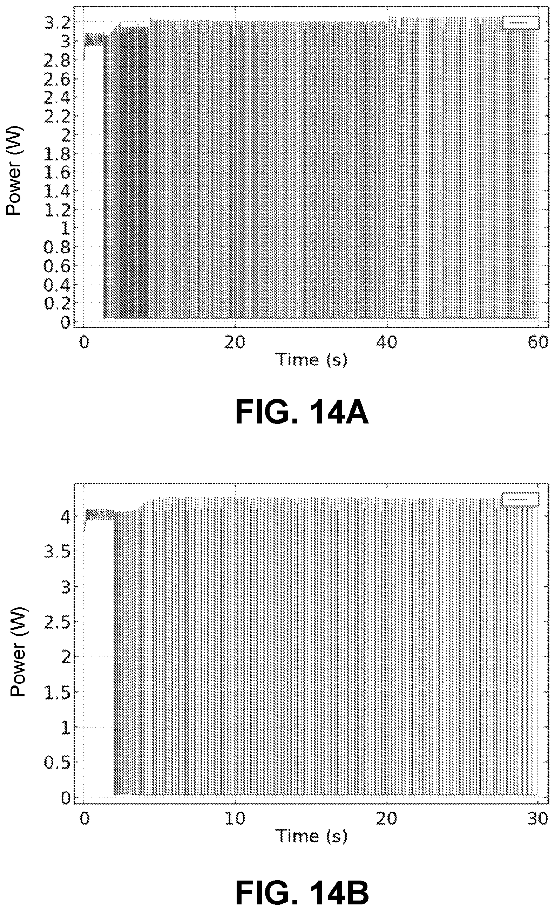

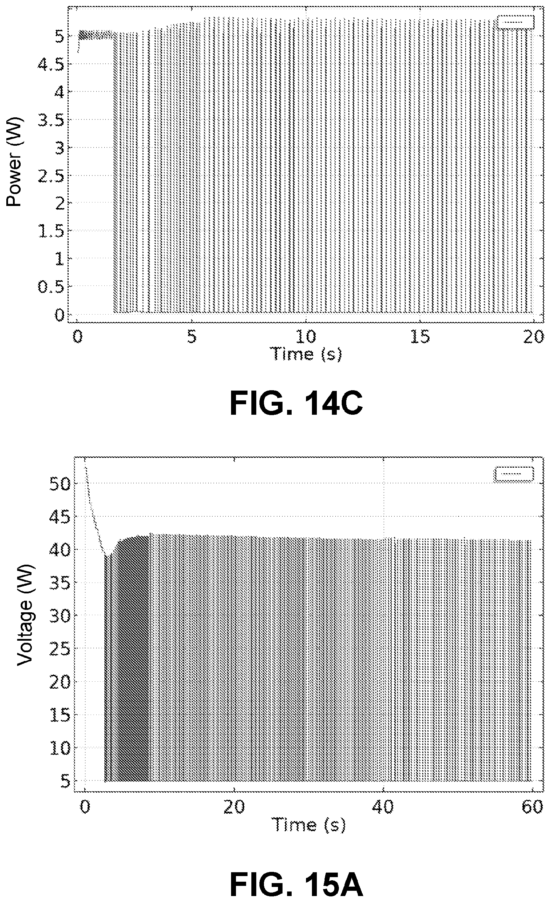

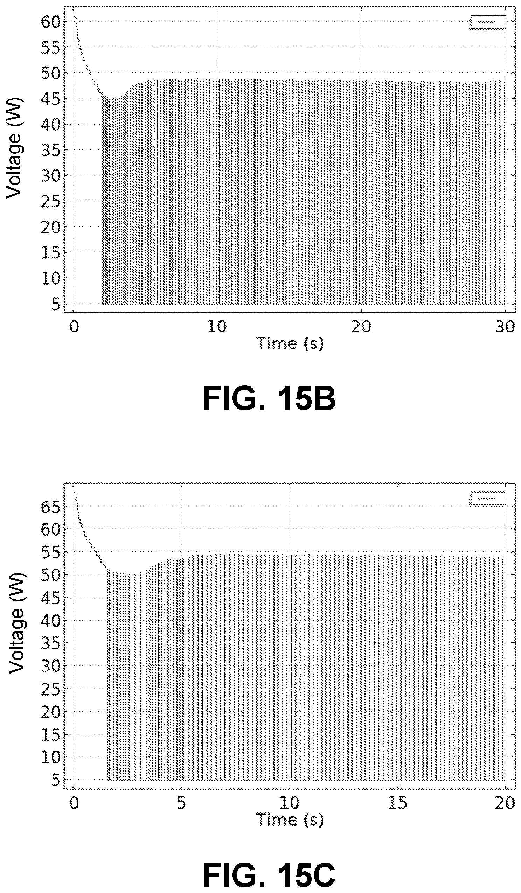

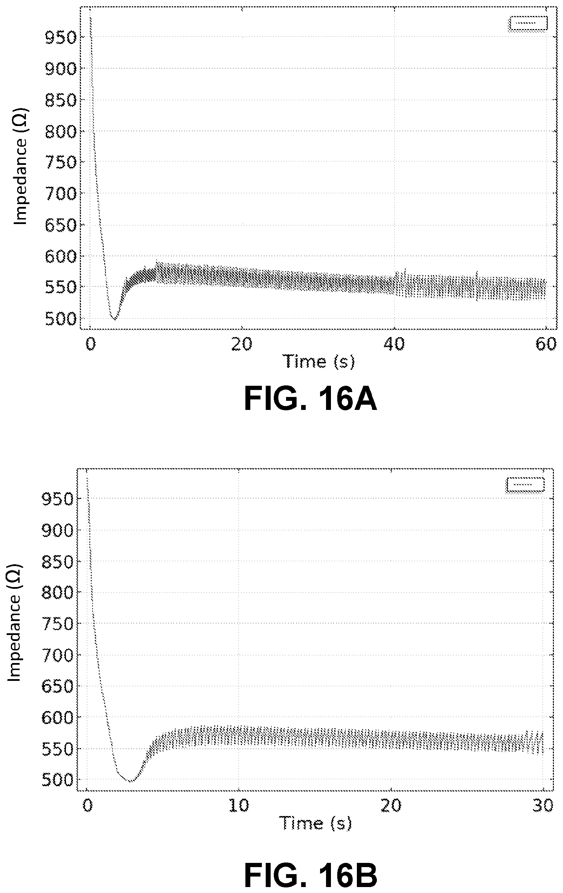

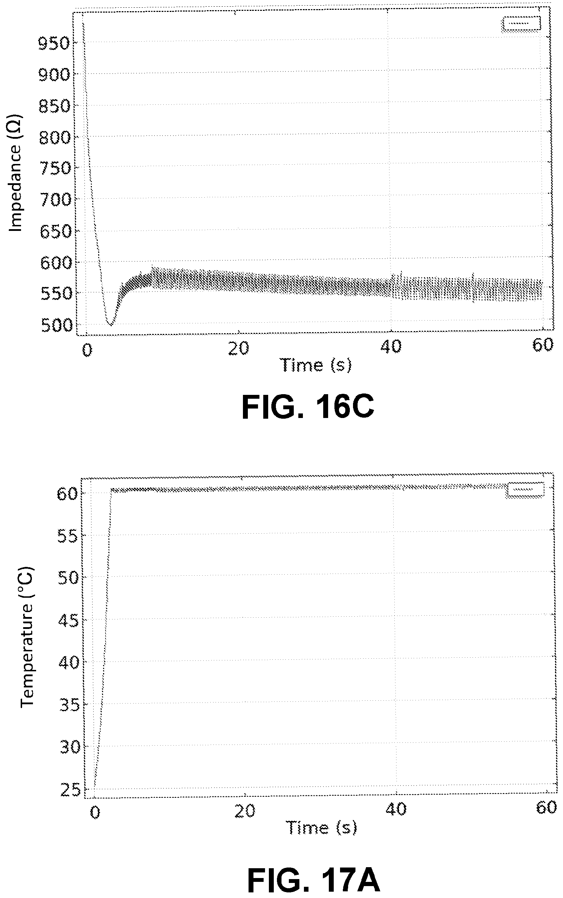

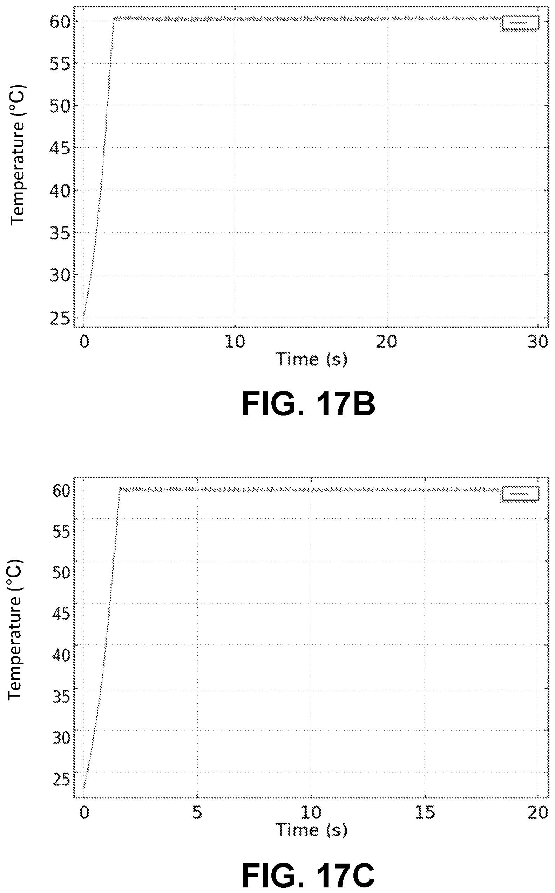

As previously described, the pulsed energy delivery provided by systems and methods of the present invention is controlled via bang-bang control hardware (part of the controller 107). The controller 107 is configured to cause delivery of pulsed energy, via a bang-bang control hardware, to the targeted tissue, wherein the pulsed energy is at a specific level and an associated duty cycle for a specific period of time based on feedback data (i.e. temperature measurement data). The pulsed energy is sufficient to maintain the temperature of the targeted tissue within the appropriate range so as to cause ablation and/or modulation thereof, while further preventing, in some instances, irreversible tissue damage. FIGS. 14 through 17 show graphs illustrating various profiles associated with pulsed energy treatment patterns delivered at different power levels. For example, FIGS. 14A, 14B, and 14C are graphs illustrating power profiles associated with pulsed energy treatment patterns delivered at 3 watts, 4 watts, and 5 watts, respectively. FIGS. 15A, 15B, and 15C are graphs illustrating voltage profiles associated with pulsed energy treatment patterns at 3 watts, 4 watts, and 5 watts, respectively. FIGS. 16A, 16B, and 16C are graphs illustrating impedance profiles associated with pulsed energy treatment patterns at 3 watts, 4 watts, and 5 watts, respectively. FIGS. 17A, 17B, and 17C are graphs illustrating temperature profiles associated with pulsed energy treatment patterns at 3 watts, 4 watts, and 5 watts, respectively.

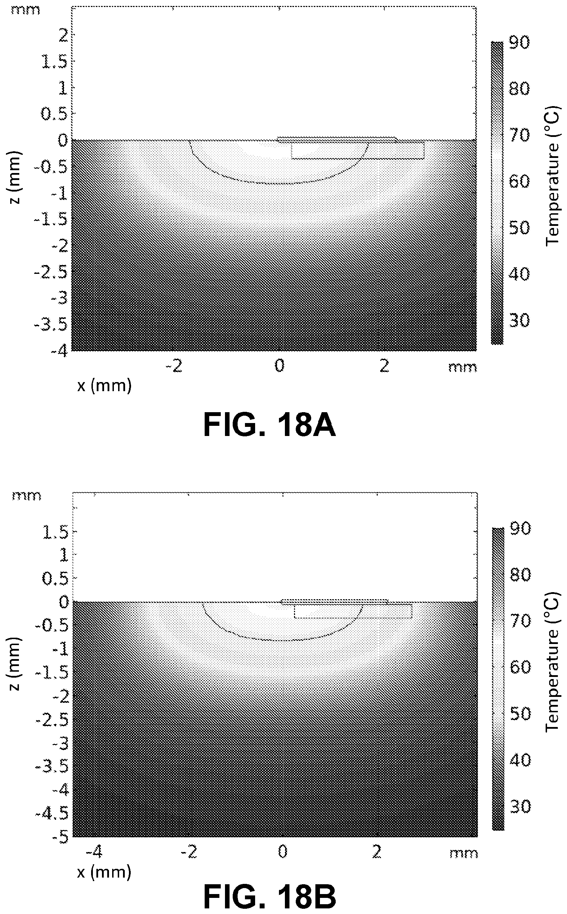

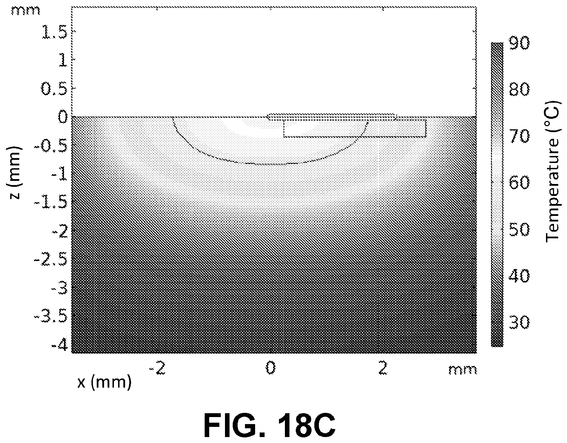

As shown, controlling various aspects of energy delivery (i.e., overall power level, voltage, and impedance) results in maintaining consistent temperature level to thereby fall within a predetermined temperature range. In particular, depending on the tissue of interest (i.e., the tissue to undergo treatment), pulsed energy from one or more electrodes is delivered at a level and an associated duty cycle sufficient to regulate and maintain temperature of the targeted tissue receiving the treatment energy at approximately 60 °C with a tolerance of approximately ±0.2 °C. Maintaining the treatment temperature within a constant range ensures a more comprehensive and accurate treatment of the targeted tissue. FIGS. 18A, 18B, and 18C illustrate resulting ablation zones of tissue depth following delivery of pulsed energy based on a pulsed energy treatment pattern at 3 watts, 4 watts, and 5 watts, respectively.

The following provides a detailed description of the various capabilities of systems and methods of the present invention, including, but not limited to, neuromodulation monitoring, feedback, and mapping capabilities, which, in turn, allowing for detection of anatomical structures and function, neural identification and mapping, and anatomical mapping, for example.

### Neuromodulation Monitoring, Feedback, and Mapping Capabilities

As previously described, the system 100 includes a console 104 to which the device 102 is to be connected. The console 104 is configured to provide various functions for the neuromodulation device 102, which may include, but is not limited to, controlling, monitoring, supplying, and/or otherwise supporting operation of the neuromodulation device 102. The console 104 can further be configured to generate a selected form and/or magnitude of energy for delivery to tissue or nerves at the target site via the end effector (214, 314), and therefore the console 104 may have different configurations depending on the treatment modality of the device 102. For example, when device 102 is configured for electrode-based, heat-element-based, and/or transducer-based treatment, the console 104 includes an energy generator 106 configured to generate RF energy (e.g., monopolar, bipolar, or multi-polar RF energy), pulsed electrical energy, microwave energy, optical energy, ultrasound energy (e.g., intraluminally-delivered ultrasound and/or HIFU), direct heat energy, radiation (e.g., infrared, visible, and/or gamma radiation), and/or another suitable type of energy. When the device 102 is configured for cryotherapeutic treatment, the console 104 can include a refrigerant reservoir (not shown), and can be configured to supply the device 102 with refrigerant. Similarly, when the device 102 is configured for chemical-based treatment (e.g., drug infusion), the console 104 can include a chemical reservoir (not shown) and can be configured to supply the device 102 with one or more chemicals.

In some embodiments, the console 104 may include a controller 107 communicatively coupled to the neuromodulation device 102. However, in the embodiments described herein, the controller 107 may generally be carried by and provided within the handle 118 of the neuromodulation device 102. The controller 107 is configured to initiate, terminate, and/or adjust operation of one or more electrodes provided by the end effector (214, 314) directly and/or via the console 104. For example, the controller 107 can be configured to execute an automated control algorithm and/or to receive control instructions from an operator (e.g., surgeon or other medical professional or clinician). For example, the controller 107 and/or other components of the console 104 (e.g., processors, memory, etc.) can include a computer-readable medium carrying instructions, which when executed by the controller 107, causes the device 102 to perform certain functions (e.g., apply energy in a specific manner, detect impedance, detect temperature, detect nerve locations or anatomical structures, perform nerve mapping, etc.). A memory includes one or more of various hardware devices for volatile and non-volatile storage, and can include both read-only and writable memory. For example, a memory can comprise random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory.

The console 104 may further be configured to provide feedback to an operator before, during, and/or after a treatment procedure via mapping/evaluation/feedback algorithms 110. For example, the mapping/evaluation/feedback algorithms 110 can be configured to provide information associated with the location of nerves at the treatment site, the location of other anatomical structures (e.g., vessels) at the treatment site, the temperature at the treatment site during monitoring and modulation, and/or the effect of the therapeutic neuromodulation on the nerves at the treatment site. In certain embodiments, the mapping/evaluation/feedback algorithm 110 can include features to confirm efficacy of the treatment and/or enhance the desired performance of the system 100. For example, the mapping/evaluation/feedback algorithm 110, in conjunction with the controller 107 and the end effector (214, 314), can be configured to monitor neural activity and/or temperature at the treatment site during therapy and automatically shut off the energy delivery when the neural activity and/or temperature reaches a predetermined threshold (e.g., a threshold reduction in neural activity, a threshold maximum temperature when applying RF energy, or a threshold minimum temperature when applying cryotherapy). In other embodiments, the mapping/evaluation/feedback algorithm 110, in conjunction with the controller 107, can be configured to automatically terminate treatment after a predetermined maximum time, a predetermined maximum impedance or resistance rise of the targeted tissue (i.e., in comparison to a baseline impedance measurement), a predetermined maximum impedance of the targeted tissue), and/or other threshold values for biomarkers associated with autonomic function. This and other information associated with the operation of the system 100 can be communicated to the operator via a display 112 (e.g., a monitor, touchscreen, user interface, etc.) on the console 104 and/or a separate display (not shown) communicatively coupled to the console 104.

In various embodiments, the end effector (214, 314) and/or other portions of the system 100 can be configured to detect various bioelectric-parameters of the tissue at the target site, and this information can be used by the mapping/evaluation/feedback algorithms 110 to determine the anatomy at the target site (e.g., tissue types, tissue locations, vasculature, bone structures, foramen, sinuses, etc.), locate neural tissue, differentiate between different types of neural tissue, map the anatomical and/or neural structure at the target site, and/or identify neuromodulation patterns of the end effector (214, 314) with respect to the patient's anatomy. For example, the end effector (214, 314) can be used to detect resistance, complex electrical impedance, dielectric properties, temperature, and/or other properties that indicate the presence of neural fibers and/or other anatomical structures in the target region. In certain embodiments, the end effector (214, 314), together with the mapping/evaluation/feedback algorithms 110, can be used to determine resistance (rather than impedance) of the tissue (i.e., the load) to more accurately identify the characteristics of the tissue. The mapping/evaluation/feedback algorithms 110 can determine resistance of the tissue by detecting the actual power and current of the load (e.g., via the electrodes (244, 336)).

In some embodiments, the system 100 provides resistance measurements with a high degree of accuracy and a very high degree of precision, such as precision measurements to the hundredths of an Ohm (e.g., 0.01Ω) for the range of 1-50Ω. The high degree of resistance detection accuracy provided by the system 100 allows for the detection sub-microscale structures, including the firing of neural tissue, differences between neural tissue and other anatomical structures (e.g., blood vessels), and event different types of neural tissue. This information can be analyzed by the mapping/evaluation/feedback algorithms and/or the controller 107 and communicated to the operator via a high resolution spatial grid (e.g., on the display 112) and/or other type of display to identify neural tissue and other anatomy at the treatment site and/or indicate predicted neuromodulation regions based on the ablation pattern with respect to the mapped anatomy.

As previously described, in certain embodiments, each electrode (244, 336) can be operated independently of the other electrodes (244, 336). For example, each electrode can be individually activated and the polarity and amplitude of each electrode can be selected by an operator or a control algorithm executed by the controller 107. The selective independent control of the electrodes (244, 336) allows the end effector (214, 314) to detect information and deliver RF energy to highly customized regions. For example, a select portion of the electrodes (244, 336) can be activated to target specific neural fibers in a specific region while the other electrodes (244, 336) remain inactive. In certain embodiments, for example, electrodes (244, 336) may be activated across the portion of a strut that is adjacent to tissue at the target site, and the electrodes (244, 336) that are not proximate to the target tissue can remain inactive to avoid applying energy to non-target tissue. In addition, the electrodes (244, 336) can be individually activated to stimulate or therapeutically modulate certain regions in a specific pattern at different times (e.g., via multiplexing), which facilitates detection of anatomical parameters across a zone of interest and/or regulated therapeutic neuromodulation.

The electrodes (244, 336) can be electrically coupled to the energy generator 106 via wires (not shown) that extend from the electrodes (244, 336), through the shaft 116, and to the energy generator 106. When each of the electrodes (244, 336) is independently controlled, each electrode (244, 336) couples to a corresponding wire that extends through the shaft 116. This allows each electrode (244, 336) to be independently activated for stimulation or neuromodulation to provide precise ablation patterns and/or individually detected via the console 104 to provide information specific to each electrode (244, 336) for neural or anatomical detection and mapping. In other embodiments, multiple electrodes (244, 336) can be controlled together and, therefore, multiple electrodes (244, 336) can be electrically coupled to the same wire extending through the shaft 116. The energy generator 16 and/or components (e.g., a control module) operably coupled thereto can include custom algorithms to control the activation of the electrodes (244, 336). For example, the RF generator can deliver RF power at about 200-100 W to the electrodes (244, 336), and do so while activating the electrodes (244, 336) in a predetermined pattern selected based on the position of the end effector (214, 314) relative to the treatment site and/or the identified locations of the target nerves. In other embodiments, the energy generator 106 delivers power at lower levels (e.g., less than 1 W, 1-5 W, 5-15 W, 15-50 W, 50-150 W, etc.) for stimulation and/or higher power levels. For example, the energy generator 106 can be configured to delivery stimulating energy pulses of 1-3 W via the electrodes (244, 336) to stimulate specific targets in the tissue.

As previously described, the end effector (214, 314) can further include one or more temperature sensors disposed on the struts and/or other portions of the end effector (214, 314) and electrically coupled to the console 104 via wires (not shown) that extend through the shaft 116. In various embodiments, the temperature sensors can be positioned proximate to the electrodes (244, 336) to detect the temperature at the interface between tissue at the target site and the electrodes (244, 336). In other embodiments, the temperature sensors can penetrate the tissue at the target site (e.g., a penetrating thermocouple) to detect the temperature at a depth within the tissue. The temperature measurements can provide the operator or the system with feedback regarding the effect of the therapeutic neuromodulation on the tissue. For example, in certain embodiments the operator may wish to prevent or reduce damage to the tissue at the treatment site (e.g., the nasal mucosa), and therefore the temperature sensors can be used to determine if the tissue temperature reaches a predetermined threshold for irreversible tissue damage. Once the threshold is reached, the application of therapeutic neuromodulation energy can be terminated to allow the tissue to remain intact and avoid significant tissue sloughing during wound healing. In certain embodiments, the energy delivery can automatically terminate based on the mapping/evaluation/feedback algorithm 110 stored on the console 104 operably coupled to the temperature sensors.

In certain embodiments, the system 100 can determine the locations and/or morphology of neural tissue and/or other anatomical structures before therapy such that the therapeutic neuromodulation can be applied to precise regions including target neural tissue, while avoiding negative effects on non-target structures, such as blood vessels. As described in further detail below, the system 100 can detect various bioelectrical parameters in an interest zone (e.g., within in the nasal cavity) to determine the location and morphology of various neural tissue (e.g., different types of neural tissue, neuronal directionality, etc.) and/or other tissue (e.g., glandular structures, vessels, bony regions, etc.). In some embodiments, the system 100 is configured to measure bioelectric potential. To do so, one or more of the electrodes (244, 336) is placed in contact with an epithelial surface at a region of interest (e.g., a treatment site). Electrical stimuli (e.g., constant or pulsed currents at one or more frequencies) are applied to the tissue by one or more electrodes (244, 336) at or near the treatment site, and the voltage and/or current differences at various different frequencies between various pairs of electrodes (244, 336) of the end effector (214, 314) may be measured to produce a spectral profile or map of the detected bioelectric potential, which can be used to identify different types of tissues (e.g., vessels, neural tissue, and/or other types of tissue) in the region of interest. For example, current (i.e., direct or alternating current) can be applied to a pair of electrodes (244, 336) adjacent to each other and the resultant voltages and/or currents between other pairs of adjacent electrodes (244, 336) are measured. It will be appreciated that the current injection electrodes (244, 336) and measurement electrodes (244, 336) need not be adjacent, and that modifying the spacing between the two current injection electrodes (244, 336) can affect the depth of the recorded signals. For example, closely-spaced current injection electrodes (244, 336) provided recorded signals associated with tissue deeper from the surface of the tissue than further spaced apart current injection electrodes (244, 336) that provide recorded signals associated with tissue at shallower depths. Recordings from electrode pairs with different spacings may be merged to provide additional information on depth and localization of anatomical structures.

Further, complex impedance and/or resistance measurements of the tissue at the region of interest can be detected directly from current-voltage data provided by the bioelectric potential measurements while differing levels of frequency currents are applied to the tissue (e.g., via the end effector (214, 314)), and this information can be used to map the neural and anatomical structures by the use of frequency differentiation reconstruction. Applying the stimuli at different frequencies will target different stratified layers or cellular bodies or clusters. At high signal frequencies (e.g., electrical injection or stimulation), for example, cell membranes of the neural tissue do not impede current flow, and the current passes directly through the cell membranes. In this case, the resultant measurement (e.g., impedance, resistance, capacitance, and/or induction) is a function of the intracellular and extracellular tissue and liquids. At low signal frequencies, the membranes impede current flow to provide different defining characteristics of the tissues, such as the shapes of the cells or cell spacing. The stimulation frequencies can be in the megahertz range, in the kilohertz range (e.g., 400-500 kHz, 450-480 kHz, etc.), and/or other frequencies attuned to the tissue being stimulated and the characteristics of the device being used. The detected complex impedance or resistances levels from the zone of interest can be displayed to the user (e.g., via the display 112) to visualize certain structures based on the stimulus frequency.

Further, the inherent morphology and composition of the anatomical structures in the nasal region react differently to different frequencies and, therefore, specific frequencies can be selected to identify very specific structures. For example, the morphology or composition of targeted structures for anatomical mapping may depend on whether the cells of tissue or other structure are membranonic, stratified, and/or annular. In various embodiments, the applied stimulation signals can have predetermined frequencies attuned to specific neural tissue, such as the level of myelination and/or morphology of the myelination. For example, second axonal parasympathetic structures are poorly myelinated than sympathetic nerves or other structures and, therefore, will have a distinguishable response (e.g., complex impedance, resistance, etc.) with respect to a selected frequency than sympathetic nerves. Accordingly, applying signals with different frequencies to the target site can distinguish the targeted parasympathetic nerves from the non-targeted sensory nerves, and therefore provide highly specific target sites for neural mapping before or after therapy and/or neural evaluation post-therapy. In some embodiments, the neural and/or anatomical mapping includes measuring data at a region of interest with at least two different frequencies to identify certain anatomical structures such that the measurements are taken first based on a response to an injection signal having a first frequency and then again based on an injection signal having a second frequency different from the first. For example, there are two frequencies at which hypertrophied (i.e., disease-state characteristics) sub-mucosal targets have a different electrical conductivity or permittivity compared to "normal" (i.e., healthy) tissue. Complex conductivity may be determined based on one or more measured physiological parameters (e.g., complex impedance, resistance, dielectric measurements, dipole measurements, etc.) and/or observance of one or more confidently known attributes or signatures. Furthermore, the system 100 can also apply neuromodulation energy via the electrodes (244, 336) at one or more predetermined frequencies attuned to a target neural structure to provide highly targeted ablation of the selected neural structure associated with the frequency(ies). This highly targeted neuromodulation also reduces the collateral effects of neuromodulation therapy to non-target sites/structures (e.g., blood vessels) because the targeted signal (having a frequency tuned to a target neural structure) will not have the same modulating effects on the non-target structures.

Accordingly, bioelectric properties, such as complex impedance and resistance, can be used by the system 100 before, during, and/or after neuromodulation therapy to guide one or more treatment parameters. For example, before, during, and/or after treatment, impedance or resistance measurements may be used to confirm and/or detect contact between one or more electrodes (244, 336) and the adjacent tissue. The impedance or resistance measurements can also be used to detect whether the electrodes (244, 336) are placed appropriately with respect to the targeted tissue type by determining whether the recorded spectra have a shape consistent with the expected tissue types and/or whether serially collected spectra were reproducible. In some embodiments, impedance or resistance measurements may be used to identify a boundary for the treatment zone (e.g., specific neural tissue that are to be disrupted), anatomical landmarks, anatomical structures to avoid (e.g., vascular structures or neural tissue that should not be disrupted), and other aspects of delivering energy to tissue.

The bioelectric information can be used to produce a spectral profile or map of the different anatomical features tissues at the target site, and the anatomical mapping can be visualized in a 3D or 2D image via the display 112 and/or other user interface to guide the selection of a suitable treatment site. This neural and anatomical mapping allows the system 100 to accurately detect and therapeutically modulate the postganglionic parasympathetic neural fibers that innervate the mucosa at the numerous neural entrance points into the nasal cavity. Further, because there are not any clear anatomical markers denoting the location of the SPF, accessory foramen, and microforamina, the neural mapping allows the operator to identify and therapeutically modulate nerves that would otherwise be unidentifiable without intricate dissection of the mucosa. In addition, anatomical mapping also allows the clinician to identify certain structures that the clinician may wish to avoid during therapeutic neural modulation (e.g., certain arteries). The neural and anatomical bioelectric properties detected by the system 100 can also be used during and after treatment to determine the real-time effect of the therapeutic neuromodulation on the treatment site. For example, the mapping/evaluation/feedback algorithms 110 can also compare the detected neural locations and/or activity before and after therapeutic neuromodulation, and compare the change in neural activity to a predetermined threshold to assess whether the application of therapeutic neuromodulation was effective across the treatment site.

In various embodiments, the system 100 can also be configured to map the expected therapeutic modulation patterns of the electrodes (244, 336) at specific temperatures and, in certain embodiments, take into account tissue properties based on the anatomical mapping of the target site. For example, the system 100 can be configured to map the ablation pattern of a specific electrode ablation pattern at the 45° C. isotherm, the 55° C. isotherm, the 65° C. isotherm, and/or other temperature/ranges (e.g., temperatures ranging from 45° C. to 70° C. or higher) depending on the target site and/or structure.

The system 100 may provide, via the display 112, three-dimensional views of such projected ablation patterns of the electrodes (244, 336) of the end effector (214, 314). The ablation pattern mapping may define a region of influence that each electrode (244, 336) has on the surrounding tissue. The region of influence may correspond to the region of tissue that would be exposed to therapeutically modulating energy based on a defined electrode activation pattern (i.e., one, two, three, four, or more electrodes on any given strut). In other words, the ablation pattern mapping can be used to illustrate the ablation pattern of any number of electrodes (244, 336), any geometry of the electrode layout, and/or any ablation activation protocol (e.g., pulsed activation, multi-polar/sequential activation, etc.).

In some embodiments, the ablation pattern may be configured such that each electrode (244, 336) has a region of influence surrounding only the individual electrode (244, 336) (i.e., a "dot" pattern). In other embodiments, the ablation pattern may be such that two or more electrodes (244, 336) may link together to form a sub-grouped regions of influence that define peanut-like or linear shapes between two or more electrodes (244, 336). In further embodiments, the ablation pattern can result in a more expansive or contiguous pattern in which the region of influence extends along multiple electrodes (244, 336) (e.g., along each strut). In still further embodiments, the ablation pattern may result in different regions of influence depending upon the electrode activation pattern, phase angle, target temperature, pulse duration, device structure, and/or other treatment parameters. The three-dimensional views of the ablation patterns can be output to the display 112 and/or other user interfaces to allow the clinician to visualize the changing regions of influence based on different durations of energy application, different electrode activation sequences (e.g., multiplexing), different pulse sequences, different temperature isotherms, and/or other treatment parameters. This information can be used to determine the appropriate ablation algorithm for a patient's specific anatomy. In other embodiments, the three-dimensional visualization of the regions of influence can be used to illustrate the regions from which the electrodes (244, 336) detect data when measuring bioelectrical properties for anatomical mapping. In this embodiment, the three dimensional visualization can be used to determine which electrode activation pattern should be used to determine the desired properties (e.g., impedance, resistance, etc.) in the desired area. In certain embodiments, it may be better to use dot assessments, whereas in other embodiments it may be more appropriate to detect information from linear or larger contiguous regions.

In some embodiments, the mapped ablation pattern is superimposed on the anatomical mapping to identify what structures (e.g., neural tissue, vessels, etc.) will be therapeutically modulated or otherwise affected by the therapy. An image may be provided to the surgeon which includes a digital illustration of a predicted or planned neuromodulation zone in relation to previously identified anatomical structures in a zone of interest. For example, the illustration may show numerous neural tissue and, based on the predicted neuromodulation zone, identifies which neural tissue are expected to be therapeutically modulated. The expected therapeutically modulated neural tissue may be shaded to differentiate them from the non-affected neural tissue. In other embodiments, the expected therapeutically modulated neural tissue can be differentiated from the non-affected neural tissue using different colors and/or other indicators. In further embodiments, the predicted neuromodulation zone and surrounding anatomy (based on anatomical mapping) can be shown in a three dimensional view (and/or include different visualization features (e.g., color-coding to identify certain anatomical structures, bioelectric properties of the target tissue, etc.). The combined predicted ablation pattern and anatomical mapping can be output to the display 112 and/or other user interfaces to allow the clinician to select the appropriate ablation algorithm for a patient's specific anatomy.

The imaging provided by the system 100 allows the clinician to visualize the ablation pattern before therapy and adjust the ablation pattern to target specific anatomical structures while avoiding others to prevent collateral effects. For example, the clinician can select a treatment pattern to avoid blood vessels, thereby reducing exposure of the vessel to the therapeutic neuromodulation energy. This reduces the risk of damaging or rupturing vessels and, therefore, prevents immediate or latent bleeding. Further, the selective energy application provided by the neural mapping reduces collateral effects of the therapeutic neuromodulation, such as tissue sloughing off during wound healing (e.g., 1-3 weeks post ablation), thereby reducing the aspiration risk associated with the neuromodulation procedure.

The system 100 can be further configured to apply neuromodulation energy (via the electrodes (244, 336)) at specific frequencies attuned to the target neural structure and, therefore, specifically target desired neural tissue over non-target structures. For example, the specific neuromodulation frequencies can correspond to the frequencies identified as corresponding to the target structure during neural mapping. As described above, the inherent morphology and composition of the anatomical structures react differently to different frequencies. Thus, frequency-tuned neuromodulation energy tailored to a target structure does not have the same modulating effects on non-target structures. More specifically, applying the neuromodulation energy at the target-specific frequency causes ionic agitation in the target neural structure, leading to differentials in osmotic potentials of the targeted neural tissue and dynamic changes in neuronal membronic potentials (resulting from the difference in intra-cellular and extra-cellular fluidic pressure). This causes degeneration, possibly resulting in vacuolar degeneration and, eventually, necrosis at the target neural structure, but is not expected to functionally affect at least some non-target structures (e.g., blood vessels). Accordingly, the system 100 can use the neural-structure specific frequencies to both (1) identify the locations of target neural tissue to plan electrode ablation configurations (e.g., electrode geometry and/or activation pattern) that specifically focus the neuromodulation on the target neural structure; and (2) apply the neuromodulation energy at the characteristic neural frequencies to selectively ablate the neural tissue responsive to the characteristic neural frequencies. For example, the end effector (214, 314) of the system 100 may selectively stimulate and/or modulate parasympathetic fibers, sympathetic fibers, sensory fibers, alpha/beta/delta fibers, C-fibers, anoxic terminals of one or more of the foregoing, insulated over non-insulated fibers (regions with fibers), and/or other neural tissue. In some embodiments, the system 100 may also selectively target specific cells or cellular regions during anatomical mapping and/or therapeutic modulation, such as smooth muscle cells, sub-mucosal glands, goblet cells, stratified cellular regions within the nasal mucosa. Therefore, the system 100 provides highly selective neuromodulation therapy specific to targeted neural tissue, and reduces the collateral effects of neuromodulation therapy to non-target structures (e.g., blood vessels).

The present disclosure provides a method of anatomical mapping and therapeutic neuromodulation. The method includes expanding an end effector (i.e., end effector (214, 314)) at a zone of interest ("interest zone"), such as in a portion of the nasal cavity. For example, the end effector (214, 314) can be expanded such that at least some of the electrodes (244, 336) are placed in contact with mucosal tissue at the interest zone. The expanded device can then take bioelectric measurements via the electrodes (244, 336) and/or other sensors to ensure that the desired electrodes are in proper contact with the tissue at the interest zone. In some embodiments, for example, the system 100 detects the impedance and/or resistance across pairs of the electrodes (244, 336) to confirm that the desired electrodes have appropriate surface contact with the tissue and that all of the electrodes are (244, 336) functioning properly.

The method continues by optionally applying an electrical stimulus to the tissue, and detecting bioelectric properties of the tissue to establish baseline norms of the tissue. For example, the method can include measuring resistance, complex impedance, current, voltage, nerve firing rate, neuromagnetic field, muscular activation, and/or other parameters that are indicative of the location and/or function of neural tissue and/or other anatomical structures (e.g., glandular structures, blood vessels, etc.). In some embodiments, the electrodes (244, 336) send one or more stimulation signals (e.g., pulsed signals or constant signals) to the interest zone to stimulate neural activity and initiate action potentials. The stimulation signal can have a frequency attuned to a specific target structure (e.g., a specific neural structure, a glandular structure, a vessel) that allows for identification of the location of the specific target structure. The specific frequency of the stimulation signal is a function of the host permeability and, therefore, applying the unique frequency alters the tissue attenuation and the depth into the tissue the RF energy will penetrate. For example, lower frequencies typically penetrate deeper into the tissue than higher frequencies.

Pairs of the non-stimulating electrodes (244, 336) of the end effector (214, 314) can then detect one or more bioelectric properties of the tissue that occur in response to the stimulus, such as impedance or resistance. For example, an array of electrodes (e.g., the electrodes (244, 336)) can be selectively paired together in a desired pattern (e.g., multiplexing the electrodes (244, 336)) to detect the bioelectric properties at desired depths and/or across desired regions to provide a high level of spatial awareness at the interest zone. In certain embodiments, the electrodes (244, 336) can be paired together in a time-sequenced manner according to an algorithm (e.g., provided by the mapping/evaluation/feedback algorithms 110). In various embodiments, stimuli can be injected into the tissue at two or more different frequencies, and the resultant bioelectric responses (e.g., action potentials) in response to each of the injected frequencies can be detected via various pairs of the electrodes (244, 336). For example, an anatomical or neural mapping algorithm can cause the end effector (214, 314) to deliver pulsed RF energy at specific frequencies between different pairs of the electrodes (244, 336) and the resultant bioelectric response can be recorded in a time sequenced rotation until the desired interest zone is adequately mapped (i.e., "multiplexing"). For example, the end effector (214, 314) can deliver stimulation energy at a first frequency via adjacent pairs of the electrodes (244, 336) for a predetermined time period (e.g., 1-50 milliseconds), and the resultant bioelectric activity (e.g., resistance) can be detected via one or more other pairs of electrodes (244, 336) (e.g., spaced apart from each other to reach varying depths within the tissue). The end effector (214, 314) can then apply stimulation energy at a second frequency different from the first frequency, and the resultant bioelectric activity can be detected via the other electrodes. This can continue when the interest zone has been adequately mapped at the desired frequencies. As described in further detail below, in some embodiments the baseline tissue bioelectric properties (e.g., nerve firing rate) are detected using static detection methods (without the injection of a stimulation signal).

After detecting the baseline bioelectric properties, the information can be used to map anatomical structures and/or functions at the interest zone. For example, the bioelectric properties detected by the electrodes (244, 336) can be analyzed via the mapping/evaluation/feedback algorithms 110, and an anatomical map can be output to a user via the display 112. In some embodiments, complex impedance, dielectric, or resistance measurements can be used to map parasympathetic nerves and, optionally, identify neural tissue in a diseased state of hyperactivity. The bioelectric properties can also be used to map other non-target structures and the general anatomy, such as blood vessels, bone, and/or glandular structures. The anatomical locations can be provided to a user (e.g., on the display 112) as a two-dimensional map (e.g., illustrating relative intensities, illustrating specific sites of potential target structures) and/or as a three-dimensional image. This information can be used to differentiate structures on a submicron, cellular level and identify very specific target structures (e.g., hyperactive parasympathetic nerves). The method can also predict the ablation patterns of the end effector (214, 314) based on different electrode neuromodulation protocol and, optionally, superimpose the predicted neuromodulation patterns onto the mapped anatomy to indicate to the user which anatomical structures will be affected by a specific neuromodulation protocol. For example, when the predicted neuromodulation pattern is displayed in relation to the mapped anatomy, a clinician can determine whether target structures will be appropriately ablated and whether non-target structures (e.g., blood vessels) will be undesirably exposed to the therapeutic neuromodulation energy. Thus, the method can be used for planning neuromodulation therapy to locate very specific target structures, avoid non-target structures, and select electrode neuromodulation protocols.

Once the target structure is located and a desired electrode neuromodulation protocol has been selected, the method continues by applying therapeutic neuromodulation to the target structure. The neuromodulation energy can be applied to the tissue in a highly targeted manner that forms micro-lesions to selectively modulate the target structure, while avoiding non-targeted blood vessels and allowing the surrounding tissue structure to remain healthy for effective wound healing. In some embodiments, the neuromodulation energy can be applied in a pulsed manner, allowing the tissue to cool between modulation pulses to ensure appropriate modulation without undesirably affecting non-target tissue. In some embodiments, the neuromodulation algorithm can deliver pulsed RF energy between different pairs of the electrodes (244, 336) in a time sequenced rotation until neuromodulation is predicted to be complete (i.e., "multiplexing"). For example, the end effector (214, 314) can deliver neuromodulation energy (e.g., having a power of 5-10 W (e.g., 7 W, 8 W, 9 W) and a current of about 50-100 mA) via adjacent pairs of the electrodes (244, 336) until at least one of the following conditions is met: (a) load resistance reaches a predefined maximum resistance (e.g., 350Ω); (b) a thermocouple temperature associated with the electrode pair reaches a predefined maximum temperature (e.g., 80° C.); or (c) a predetermined time period has elapsed (e.g., 10 seconds). After the predetermined conditions are met, the end effector (214, 314) can move to the next pair of electrodes in the sequence, and the neuromodulation algorithm can terminate when all of the load resistances of the individual pairs of electrodes is at or above a predetermined threshold (e.g., 100Ω). In various embodiments, the RF energy can be applied at a predetermined frequency (e.g., 450-500 kHz) and is expected to initiate ionic agitation of the specific target structure, while avoiding functional disruption of non-target structures.

During and/or after neuromodulation therapy, the method continues by detecting and, optionally, mapping the post-therapy bioelectric properties of the target site. This can be performed in a similar manner as described above. The post-therapy evaluation can indicate if the target structures (e.g., hyperactive parasympathetic nerves) were adequately modulated or ablated. If the target structures are not adequately modulated (i.e., if neural activity is still detected in the target structure and/or the neural activity has not decreased), the method can continue by again applying therapeutic neuromodulation to the target. If the target structures were adequately ablated, the neuromodulation procedure can be completed.

### Detection of Anatomical Structures and Function

Various embodiments of the present technology can include features that measure bioelectric, dielectric, and/or other properties of tissue at target sites to determine the presence, location, and/or activity of neural tissue and other anatomical structures and, optionally, map the locations of the detected neural tissue and/or other anatomical structures. For example, the present technology can be used to detect glandular structures and, optionally, their mucoserous functions and/or other functions. The present technology can also be configured to detect vascular structures (e.g., arteries) and, optionally, their arterial functions, volumetric pressures, and/or other functions. The mapping features discussed below can be incorporated into any the system 100 and/or any other devices disclosed herein to provide an accurate depiction of nerves at the target site.

Neural and/or anatomical detection can occur (a) before the application of a therapeutic neuromodulation energy to determine the presence or location of neural tissue and other anatomical structures (e.g., blood vessels, glands, etc.) at the target site and/or record baseline levels of neural activity; (b) during therapeutic neuromodulation to determine the real-time effect of the energy application on the neural fibers at the treatment site; and/or (c) after therapeutic neuromodulation to confirm the efficacy of the treatment on the targeted structures (e.g., nerves glands, etc.). This allows for the identification of very specific anatomical structures (even to the micro-scale or cellular level) and, therefore, provides for highly targeted neuromodulation. This enhances the efficacy and efficiency of the neuromodulation therapy. In addition, the anatomical mapping reduces the collateral effects of neuromodulation therapy to non-target sites. Accordingly, the targeted neuromodulation inhibits damage or rupture of blood vessels (i.e., inhibits undesired bleeding) and collateral damage to tissue that may be of concern during wound healing (e.g., when damage tissue sloughs off of the wall of the nasal wall).

In certain embodiments, the systems disclosed herein can use bioelectric measurements, such as impedance, resistance, voltage, current density, and/or other parameters (e.g., temperature) to determine the anatomy, in particular the neural, glandular, and vascular anatomy, at the target site. The bioelectric properties can be detected after the transmission of a stimulus (e.g., an electrical stimulus, such as RF energy delivered via the electrodes (244, 336); i.e., "dynamic" detection) and/or without the transmission of a stimulus (i.e., "static" detection).

Dynamic measurements include various embodiments to excite and/or detect primary or secondary effects of neural activation and/or propagation. Such dynamic embodiments involve the heightened states of neural activation and propagation and use this dynamic measurement for nerve location and functional identification relative to the neighboring tissue types. For example, a method of dynamic detection can include: (1) delivering stimulation energy to a treatment site via a treatment device (e.g., the end effector) to excite parasympathetic nerves at the treatment site; (2) measuring one or more physiological parameters (e.g., resistance, impedance, etc.) at the treatment site via a measuring/sensing array of the treatment device (e.g., the electrodes (244, 336)); (4) based on the measurements, identifying the relative presence and position of parasympathetic nerves at the treatment site; and (5) delivering ablation energy to the identified parasympathetic nerves to block the detected para-sympathetic nerves.

Static measurements include various embodiments associated with specific native properties of the stratified or cellular composition at or near the treatment site. The static embodiments are directed to inherent biologic and electrical properties of tissue types at or near the treatment site, the stratified or cellular compositions at or near the treatment site, and contrasting both foregoing measurements with tissue types adjacent the treatment site (and that are not targeted for neuromodulation). This information can be used to localize specific targets (e.g., parasympathetic fibers) and non-targets (e.g., vessels, sensory nerves, etc.). For example, a method of static detection can include: (1) before ablation, utilizing a measuring/sensing array of a treatment device (e.g., the electrodes (244, 336)) to determine one or more baseline physiological parameters; (2) geometrically identifying inherent tissue properties within a region of interest based on the measured physiological parameters (e.g., resistance, impedance, etc.); (3) delivering ablation energy to one or more nerves within the region of via treatment device interest; (4) during the delivery of the ablation energy, determining one or more mid-procedure physiological parameters via the measuring/sensing array; and (5) after the delivery of ablation energy, determining one or more post-procedure physiological parameters via the measurement/sensing array to determine the effectiveness of the delivery of the ablation energy on blocking the nerves that received the ablation energy.

After the initial static and/or dynamic detection of bioelectric properties, the location of anatomical features can be used to determine where the treatment site(s) should be with respect to various anatomical structures for therapeutically effective neuromodulation of the targeted parasympathetic nasal nerves. The bioelectric and other physiological properties described herein can be detected via electrodes (e.g., the electrodes (244, 336) of the end effector (214, 314)), and the electrode pairings on a device (e.g., end effector (214, 314)) can be selected to obtain the bioelectric data at specific zones or regions and at specific depths of the targeted regions. The specific properties detected at or surrounding target neuromodulation sites and associated methods for obtaining these properties are described below. These specific detection and mapping methods discussed below are described with reference to the system 100 , although the methods can be implemented on other suitable systems and devices that provide for anatomical identification, anatomical mapping and/or neuromodulation therapy.

### Neural Identification and Mapping

In many neuromodulation procedures, it is beneficial to identify the portions of the nerves that fall within a zone and/or region of influence (referred to as the "interest zone") of the energy delivered by a neuromodulation device 102, as well as the relative three-dimensional position of the neural tissue relative to the neuromodulation device 102. Characterizing the portions of the neural tissue within the interest zone and/or determining the relative positions of the neural tissue within the interest zone enables the clinician to (1) selectively activate target neural tissue over non-target structures (e.g., blood vessels), and (2) sub-select specific targeted neural tissue (e.g., parasympathetic nerves) over non-target neural tissue (e.g., sensory nerves, subgroups of neural tissue, neural tissue having certain compositions or morphologies). The target structures (e.g., parasympathetic nerves) and non-target structures (e.g., blood vessels, sensory nerves, etc.) can be identified based on the inherent signatures of specific structures, which are defined by the unique morphological compositions of the structures and the bioelectrical properties associated with these morphological compositions. For example, unique, discrete frequencies can be associated with morphological compositions and, therefore, be used to identify certain structures. The target and non-target structures can also be identified based on relative bioelectrical activation of the structures to sub-select specific neural structures. Further, target and non-target structures can be identified by the differing detected responses of the structures to a tailored injected stimuli. For example, the systems described herein can detect the magnitude of response of structures and the difference in the responses of anatomical structures with respect to differing stimuli (e.g., stimuli injected at different frequencies).

At least for purposes of this disclosure, a nerve can include the following portions that are defined based on their respective orientations relative to the interest zone: terminating neural tissue (e.g., terminating axonal structures), branching neural tissue (e.g., branching axonal structures), and travelling neural tissue (e.g., travelling axonal structures). For example, terminating neural tissue enter the zone but do not exit. As such, terminating neural tissue are terminal points for neuronal signaling and activation. Branching neural tissue are nerves that enter the interest zone and increase number of nerves exiting the interest zone. Branching neural tissue are typically associated with a reduction in relative geometry of nerve bundle. Travelling neural tissue are nerves that enter the interest zone and exit the zone with no substantially no change in geometry or numerical value.

The system 100 can be used to detect voltage, current, complex impedance, resistance, permittivity, and/or conductivity, which are tied to the compound action potentials of nerves, to determine and/or map the relative positions and proportionalities of nerves in the interest zone. Neuronal cross-sectional area ("CSA") is expected to be due to the increase in axonic structures. Each axon is a standard size. Larger nerves (in cross-sectional dimension) have a larger number of axons than nerves having smaller cross-sectional dimensions. The compound action responses from the larger nerves, in both static and dynamic assessments, are greater than smaller nerves. This is at least in part because the compound action potential is the cumulative action response from each of the axons. When using static analysis, for example, the system 100 can directly measure and map impedance or resistance of nerves and, based on the determined impedance or resistance, determine the location of nerves and/or relative size of the nerves. In dynamic analysis, the system 100 can be used to apply a stimulus to the interest zone and detect the dynamic response of the neural tissue to the stimulus. Using this information, the system 100 can determine and/or map impedance or resistance in the interest zone to provide information related to the neural positions or relative nerve sizes. Neural impedance mapping can be illustrated by showing the varying complex impedance levels at a specific location at differing cross-sectional depths. In other embodiments, neural impedance or resistance can be mapped in a three-dimensional display.

Identifying the portions and/or relative positions of the nerves within the interest zone can inform and/or guide selection of one or more treatment parameters (e.g., electrode ablation patterns, electrode activation plans, etc.) of the system 100 for improving treatment efficiency and efficacy. For example, during neural monitoring and mapping, the system 100 can identify the directionality of the nerves based at least in part on the length of the neural structure extending along the interest zone, relative sizing of the neural tissue, and/or the direction of the action potentials. This information can then be used by the system 100 or the clinician to automatically or manually adjust treatment parameters (e.g., selective electrode activation, bipolar and/or multipolar activation, and/or electrode positioning) to target specific nerves or regions of nerves. For example, the system 100 can selectively activate specific electrodes (244, 336), electrode combinations (e.g., asymmetric or symmetric), and/or adjust the bi-polar or multi-polar electrode configuration. In some embodiments, the system 100 can adjust or select the waveform, phase angle, and/or other energy delivery parameters based on the nerve portion/position mapping and/or the nerve proportionality mapping. In some embodiments, structure and/or properties of the electrodes (244, 336) themselves (e.g., material, surface roughening, coatings, cross-sectional area, perimeter, penetrating, penetration depth, surface-mounted, etc.) may be selected based on the nerve portion and proportionality mapping.

In various embodiments, treatment parameters and/or energy delivery parameters can be adjusted to target on-axis or near axis travelling neural tissue and/or avoid the activation of traveling neural tissue that are at least generally perpendicular to the end effector (214, 314). Greater portions of the on-axis or near axis travelling neural tissue are exposed and susceptible to the neuromodulation energy provided by the end effector (214, 314) than a perpendicular travelling neural structure, which may only be exposed to therapeutic energy at a discrete cross-section. Therefore, the end effector (214, 314) is more likely to have a greater effect on the on-axis or near axis travelling neural tissue. The identification of the neural structure positions (e.g., via complex impedance or resistance mapping) can also allow targeted energy delivery to travelling neural tissue rather than branching neural tissue (typically downstream of the travelling neural tissue) because the travelling neural tissue are closer to the nerve origin and, therefore, more of the nerve is affected by therapeutic neuromodulation, thereby resulting in a more efficient treatment and/or a higher efficacy of treatment. Similarly, the identification of neural structure positions can be used to target travelling and branching neural tissue over terminal neural tissue. In some embodiments, the treatment parameters can be adjusted based on the detected neural positions to provide a selective regional effect. For example, a clinician can target downstream portions of the neural tissue if only wanting to influence partial effects on very specific anatomical structures or positions.

In various embodiments, neural locations and/or relative positions of nerves can be determined by detecting the nerve-firing voltage and/or current over time. An array of the electrodes (244, 336) can be positioned in contact with tissue at the interest zone, and the electrodes (244, 336) can measure the voltage and/or current associated with nerve-firing. This information can optionally be mapped (e.g., on a display 112) to identify the location of nerves in a hyper state (i.e., excessive parasympathetic tone). Rhinitis is at least in part the result of over-firing nerves because this hyper state drives the hyper-mucosal production and hyper-mucosal secretion. Therefore, detection of nerve firing rate via voltage and current measurements can be used to locate the portions of the interest region that include hyper-parasympathetic neural function (i.e., nerves in the diseased state). This allows the clinician to locate specific nerves (i.e., nerves with excessive parasympathetic tone) before neuromodulation therapy, rather than simply targeting all parasympathetic nerves (including non-diseased state parasympathetic nerves) to ensure that the correct tissue is treated during neuromodulation therapy. Further, nerve firing rate can be detected during or after neuromodulation therapy so that the clinician can monitor changes in nerve firing rate to validate treatment efficacy. For example, recording decreases or elimination of nerve firing rate after neuromodulation therapy can indicate that the therapy was effective in therapeutically treating the hyper/diseased nerves.

In various embodiments, the system 100 can detect neural activity using dynamic activation by injecting a stimulus signal (i.e., a signal that temporarily activates nerves) via one or more of the electrodes (244, 336) to induce an action potential, and other pairs of electrodes (244, 336) can detect bioelectric properties of the neural response. Detecting neural tissue using dynamic activation involves detecting the locations of action potentials within the interest zone by measuring the discharge rate in neurons and the associated processes. The ability to numerically measure, profile, map, and/or image fast neuronal depolarization for generating an accurate index of activity is a factor in measuring the rate of discharge in neurons and their processes. The action potential causes a rapid increase in the voltage across nerve fiber and the electrical impulse then spreads along the fiber. As an action potential occurs, the conductance of a neural cell membrane changes, becoming about 40 times larger than it is when the cell is at rest. During the action potential or neuronal depolarization, the membrane resistance diminishes by about 80 times, thereby allowing an applied current to enter the intracellular space as well. Over a population of neurons, this leads to a net decrease in the resistance during coherent neuronal activity, such as chronic para-sympathetic responses, as the intracellular space will provide additional conductive ions. The magnitude of such fast changes has been estimated to have local resistivity changes with recording near DC is 2.8-3.7% for peripheral nerve bundles (e.g., including the nerves in the nasal cavity).

Detecting neural tissue using dynamic activation includes detecting the locations of action potentials within the interest zone by measuring the discharge rate in neurons and the associated processes. The basis of each this discharge is the action potential, during which there is a depolarization of the neuronal membrane of up to 110 mV or more, lasting approximately 2 milliseconds, and due to the transfer of micromolar quantities of ions (e.g., sodium and potassium) across the cellular membrane. The complex impedance or resistance change due to the neuronal membrane falls from 1000 to 25 Ωcm. The introduction of a stimulus and subsequent measurement of the neural response can attenuate noise and improve signal to noise ratios to precisely focus on the response region to improve neural detection, measurement, and mapping.

In some embodiments, the difference in measurements of physiological parameters (e.g., complex impedance, resistance, voltage) over time, which can reduce errors, can be used to create a neural profiles, spectrums, or maps. For example, the sensitivity of the system 100 can be improved because this process provides repeated averaging to a stimulus. As a result, the mapping function outputs can be a unit-less ratio between the reference and test collated data at a single frequency and/or multiple frequencies and/or multiple amplitudes. Additional considerations may include multiple frequency evaluation methods that consequently expand the parameter assessments, such as resistivity, admittivity, center frequency, or ratio of extra- to intracellular resistivity.

In some embodiments, the system 100 may also be configured to indirectly measure the electrical activity of neural tissue to quantify the metabolic recovery processes that accompany action potential activity and act to restore ionic gradients to normal. These are related to an accumulation of ions in the extracellular space. The indirect measurement of electrical activity can be approximately a thousand times larger (in the order of millimolar), and thus are easier to measure and can enhance the accuracy of the measured electrical properties used to generate the neural maps.

The system 100 can perform dynamic neural detection by detecting nerve-firing voltage and/or current and, optionally, nerve firing rate over time, in response to an external stimulation of the nerves. For example, an array of the electrodes (244, 336) can be positioned in contact with tissue at the interest zone, one or more of the electrodes (244, 336) can be activated to inject a signal into the tissue that stimulates the nerves, and other electrodes (244, 336) of the electrode array can measure the neural voltage and/or current due to nerve firing in response to the stimulus. This information can optionally be mapped (e.g., on a display 112) to identify the location of nerves and, in certain embodiments, identify parasympathetic nerves in a hyper state (e.g., indicative of Rhinitis or other diseased state). The dynamic detection of neural activity (voltage, current, firing rate, etc.) can be performed before neuromodulation therapy to detect target nerve locations to select the target site and treatment parameters to ensure that the correct tissue is treated during neuromodulation therapy. Further, dynamic detection of neural activity can be performed during or after neuromodulation therapy to allow the clinician to monitor changes in neural activity to validate treatment efficacy. For example, recording decreases or elimination of neural activity after neuromodulation therapy can indicate that the therapy was effective in therapeutically treating the hyper/diseased nerves.

In some embodiments, a stimulating signal can be delivered to the vicinity of the targeted nerve via one or more penetrating electrodes (e.g., microneedles that penetrate tissue) associated with the end effector (214, 314) and/or a separate device. The stimulating signal generates an action potential, which causes smooth muscle cells or other cells to contract. The location and strength of this contraction can be detected via the penetrating electrode(s) and, thereby, indicate to the clinician the distance to the nerve and/or the location of the nerve relative to the stimulating needle electrode. In some embodiments, the stimulating electrical signal may have a voltage of typically 1-2 mA or greater and a pulse width of typically 100-200 microseconds or greater. Shorter pulses of stimulation result in better discrimination of the detected contraction, but may require more current. The greater the distance between the electrode and the targeted nerve, the more energy is required to stimulate. The stimulation and detection of contraction strength and/or location enables identification of how close or far the electrodes are from the nerve, and therefore can be used to localize the nerve spatially. In some embodiments, varying pulse widths may be used to measure the distance to the nerve. As the needle becomes closer to the nerve, the pulse duration required to elicit a response becomes less and less.

To localize nerves via muscle contraction detection, the system 100 can vary pulse-width or amplitude to vary the energy (Energy=pulse-width*amplitude) of the stimulus delivered to the tissue via the penetrating electrode(s). By varying the stimulus energy and monitoring muscle contraction via the penetrating electrodes and/or other type of sensor, the system 100 can estimate the distance to the nerve. If a large amount of energy is required to stimulate the nerve/contract the muscle, the stimulating/penetrating electrode is far from the nerve. As the stimulating/penetrating electrode, moves closer to the nerve, the amount of energy required to induce muscle contraction will drop. For example, an array of penetrating electrodes can be positioned in the tissue at the interest zone and one or more of the electrodes can be activated to apply stimulus at different energy levels until they induce muscle contraction. Using an iterative process, localize the nerve (e.g., via the mapping/evaluation/feedback algorithm 110).

In some embodiments, the system 100 can measure the muscular activation from the nerve stimulus (e.g., via the electrodes (244, 336)) to determine neural positioning for neural mapping, without the use of penetrating electrodes. In this embodiment, the treatment device targets the smooth muscle cells' varicosities surrounding the submucosal glands and the vascular supply, and then the compound muscle action potential. This can be used to summate voltage response from the individual muscle fiber action potentials. The shortest latency is the time from stimulus artifact to onset of the response. The corresponding amplitude is measured from baseline to negative peak and measured in millivolts (mV). Nerve latencies (mean±SD) in adults typically range about 2-6 milliseconds, and more typically from about 3.4±0.8 to about 4.0±0.5 milliseconds. A comparative assessment may then be made which compares the outputs at each time interval (especially pre- and post-energy delivery) in addition to a group evaluation using the alternative nasal cavity. This is expected to provide an accurate assessment of the absolute value of the performance of the neural functioning because muscular action/activation may be used to infer neural action/activation and muscle action/activation is a secondary effect or byproduct whilst the neural function is the absolute performance measure.

In some embodiments, the system 100 can record a neuromagnetic field outside of the nerves to determine the internal current of the nerves without physical disruption of the nerve membrane. Without being bound by theory, the contribution to the magnetic field from the current inside the membrane is two orders of magnitude larger than that from the external current, and that the contribution from current within the membrane is substantially negligible. Electrical stimulation of the nerve in tandem with measurements of the magnetic compound action fields ("CAFs") can yield sequential positions of the current dipoles such that the location of the conduction change can be estimated (e.g., via the least-squares method). Visual representation (e.g., via the display 112) using magnetic contour maps can show normal or non-normal neural characteristics (e.g., normal can be equated with a characteristic quadrupolar pattern propagating along the nerve), and therefore indicate which nerves are in a diseases, hyperactive state and suitable targets for neuromodulation.

During magnetic field detection, an array of the electrodes (244, 336) can be positioned in contact with tissue at the interest zone and, optionally, one or more of the electrodes (244, 336) can be activated to inject an electrical stimulus into the tissue. As the nerves in the interest zone fire (either in response to a stimulus or in the absence of it), the nerve generates a magnetic field (e.g., similar to a current carrying wire), and therefore changing magnetic fields are indicative of the nerve nerve-firing rate. The changing magnetic field caused by neural firing can induce a current detected by nearby sensor wire (e.g., the sensor 314) and/or wires associated with the nearby electrodes (244, 336). By measuring this current, the magnetic field strength can be determined. The magnetic fields can optionally be mapped (e.g., on a display 112) to identify the location of nerves and select target nerves (nerves with excessive parasympathetic tone) before neuromodulation therapy to ensure that the desired nerves are treated during neuromodulation therapy. Further, the magnetic field information can be used during or after neuromodulation therapy so that the clinician can monitor changes in nerve firing rate to validate treatment efficacy.

In other embodiments, the neuromagnetic field is measured with a Hall Probe or other suitable device, which can be integrated into the end effector (214, 314) and/or part of a separate device delivered to the interest zone. Alternatively, rather than measuring the voltage in the second wire, the changing magnetic field can be measured in the original wire (i.e. the nerve) using a Hall probe. A current going through the Hall probe will be deflected in the semiconductor. This will cause a voltage difference between the top and bottom portions, which can be measured. In some aspects of this embodiments, three orthogonal planes are utilized.

In some embodiments, the system 100 can be used to induce electromotive force ("EMF") in a wire (i.e., a frequency-selective circuit, such as a tunable/LC circuit) that is tunable to resonant frequency of a nerve. In this embodiment, the nerve can be considered to be a current carrying wire, and the firing action potential is a changing voltage. This causes a changing current which, in turn, causes a changing magnetic flux (i.e., the magnetic field that is perpendicular to the wire). Under Faraday's Law of Induction/Faraday's Principle, the changing magnetic flux induces EMF (including a changing voltage) in a nearby sensor wire (e.g., integrated into the end effector (214, 314), the sensor 314, and/or other structure), and the changing voltage can be measured via the system 100.

In further embodiments, the sensor wire (e.g., the sensor 314) is an inductor and, therefore, provides an increase of the magnetic linkage between the nerve (i.e., first wire) and the sensor wire (i.e., second wire), with more turns for increasing effect. (e.g., V2,rms=V1,rms (N2/N1)). Due to the changing magnetic field, a voltage is induced in the sensor wire, and this voltage can be measured and used to estimate current changes in the nerve. Certain materials can be selected to enhance the efficiency of the EMF detection. For example, the sensor wire can include a soft iron core or other high permeability material for the inductor.

During induced EMF detection, the end effector (214, 314) and/or other device including a sensor wire is positioned in contact with tissue at the interest zone and, optionally, one or more of the electrodes (244, 336) can be activated to inject an electrical stimulus into the tissue. As the nerves in the interest zone fire (either in response to a stimulus or in the absence of it), the nerve generates a magnetic field (e.g., similar to a current carrying wire) that induces a current in the sensor wire (e.g., the sensor 314). This information can be used to determine neural location and/or map the nerves (e.g., on a display 112) to identify the location of nerves and select target nerves (nerves with excessive parasympathetic tone) before neuromodulation therapy to ensure that the desired nerves are treated during neuromodulation therapy. EMF information can also be used during or after neuromodulation therapy so that the clinician can monitor changes in nerve firing rate to validate treatment efficacy.

In some embodiments, the system 100 can detect magnetic fields and/or EMF generated at a selected frequency that corresponds to a particular type of nerve. The frequency and, by extension, the associated nerve type of the detected signal can be selected based on an external resonant circuit. Resonance occurs on the external circuit when it is matched to the frequency of the magnetic field of the particular nerve type and that nerve is firing. In manner, the system 100 can be used to locate a particular sub-group/type of nerves.

In some embodiments, the system 100 can include a variable capacitor frequency-selective circuit to identify the location and/or map specific nerves (e.g., parasympathetic nerve, sensory nerve, nerve fiber type, nerve subgroup, etc.). The variable capacitor frequency-selective circuit can be defined by the sensor 314 and/or other feature of the end effector (214, 314). Nerves have different resonant frequencies based on their function and structure. Accordingly, the system 100 can include a tunable LC circuit with a variable capacitor (C) and/or variable inductor (L) that can be selectively tuned to the resonant frequency of desired nerve types. This allows for the detection of neural activity only associated with the selected nerve type and its associated resonant frequency. Tuning can be achieved by moving the core in and out of the inductor. For example, tunable LC circuits can tune the inductor by: (i) changing the number of coils around the core; (ii) changing the cross-sectional area of the coils around the core; (iii) changing the length of the coil; and/or (iv) changing the permeability of the core material (e.g., changing from air to a core material). Systems including such a tunable LC circuit provide a high degree of dissemination and differentiation not only as to the activation of a nerve signal, but also with respect to the nerve type that is activated and the frequency at which the nerve is firing.

### Anatomical Mapping

In various embodiments, the system 100 is further configured to provide minimally-invasive anatomical mapping that uses focused energy current/voltage stimuli from a spatially localized source (e.g., the electrodes (244, 336)) to cause a change in the conductivity of the of the tissue at the interest zone and detect resultant biopotential and/or bioelectrical measurements (e.g., via the electrodes (244, 336)). The current density in the tissue changes in response to changes of voltage applied by the electrodes (244, 336), which creates a change in the electric current that can be measured with the end effector (214, 314) and/or other portions of the system 100. The results of the bioelectrical and/or biopotential measurements can be used to predict or estimate relative absorption profilometry to predict or estimate the tissue structures in the interest zone. More specifically, each cellular construct has unique conductivity and absorption profiles that can be indicative of a type of tissue or structure, such as bone, soft tissue, vessels, nerves, types of nerves, and/or certain neural tissue. For example, different frequencies decay differently through different types of tissue. Accordingly, by detecting the absorption current in a region, the system 100 can determine the underlying structure and, in some instances, to a sub-microscale, cellular level that allows for highly specialized target localization and mapping. This highly specific target identification and mapping enhances the efficacy and efficiency of neuromodulation therapy, while also enhancing the safety profile of the system 100 to reduce collateral effects on non-target structures.

To detect electrical and dielectric tissue properties (e.g., resistance, complex impedance, conductivity, and/or, permittivity as a function of frequency), the electrodes (244, 336) and/or another electrode array is placed on tissue at an interest region, and an internal or external source (e.g., the generator 106) applies stimuli (current/voltage) to the tissue. The electrical properties of the tissue between the source and the receiver electrodes (244, 336) are measured, as well as the current and/or voltage at the individual receiver electrodes (244, 336). These individual measurements can then be converted into an electrical map/image/profile of the tissue and visualized for the user on the display 112 to identify anatomical features of interest and, in certain embodiments, the location of firing nerves. For example, the anatomical mapping can be provided as a color-coded or gray-scale three-dimensional or two-dimensional map showing differing intensities of certain bioelectric properties (e.g., resistance, impedance, etc.), or the information can be processed to map the actual anatomical structures for the clinician. This information can also be used during neuromodulation therapy to monitor treatment progression with respect to the anatomy, and after neuromodulation therapy to validate successful treatment. In addition, the anatomical mapping provided by the bioelectrical and/or biopotential measurements can be used to track the changes to non-target tissue (e.g., vessels) due to neuromodulation therapy to avoid negative collateral effects. For example, a clinician can identify when the therapy begins to ligate a vessel and/or damage tissue, and modify the therapy to avoid bleeding, detrimental tissue ablation, and/or other negative collateral effects.

Furthermore, the threshold frequency of electric current used to identify specific targets can subsequently be used when applying therapeutic neuromodulation energy. For example, the neuromodulation energy can be applied at the specific threshold frequencies of electric current that are target neuronal-specific and differentiated from other non-targets (e.g., blood vessels, non-target nerves, etc.). Applying ablation energy at the target-specific frequency results in an electric field that creates ionic agitation in the target neural structure, which leads to differentials in osmotic potentials of the targeted neural tissue. These osmotic potential differentials cause dynamic changes in neuronal membronic potentials (resulting from the difference in intra-cellular and extra-cellular fluidic pressure) that lead to vacuolar degeneration of the targeted neural tissue and, eventually, necrosis. Using the highly targeted threshold neuromodulation energy to initiate the degeneration allows the system 100 to deliver therapeutic neuromodulation to the specific target, while surrounding blood vessels and other non-target structures are functionally maintained.

In some embodiments, the system 100 can further be configured to detect bioelectrical properties of tissue by non-invasively recording resistance changes during neuronal depolarization to map neural activity with electrical impedance, resistance, bio-impedance, conductivity, permittivity, and/or other bioelectrical measurements. Without being bound by theory, when a nerve depolarizes, the cell membrane resistance decreases (e.g., by approximately 80×) so that current will pass through open ion channels and into the intracellular space. Otherwise the current remains in the extracellular space. For non-invasive resistance measurements, tissue can be stimulated by applying a current of less than 100 Hz, such as applying a constant current square wave at 1 Hz with an amplitude less than 25% (e.g., 10%) of the threshold for stimulating neuronal activity, and thereby preventing or reducing the likelihood that the current does not cross into the intracellular space or stimulating at 2 Hz. In either case, the resistance and/or complex impedance is recorded by recording the voltage changes. A complex impedance or resistance map or profile of the area can then be generated.

For impedance/conductivity/permittivity detection, the electrodes (244, 336) and/or another electrode array are placed on tissue at an interest region, and an internal or external source (e.g., the generator 106) applies stimuli to the tissue, and the current and/or voltage at the individual receiver electrodes (244, 336) is measured. The stimuli can be applied at different frequencies to isolate different types of nerves. These individual measurements can then be converted into an electrical map/image/profile of the tissue and visualized for the user on the display 112 to identify anatomical features of interest. The neural mapping can also be used during neuromodulation therapy to select specific nerves for therapy, monitor treatment progression with respect to the nerves and other anatomy, and validate successful treatment.

In some embodiments of the neural and/or anatomical detection methods described above, the procedure can include comparing the mid-procedure physiological parameter(s) to the baseline physiological parameter(s) and/or other, previously-acquired mid-procedure physiological parameter(s) (within the same energy delivery phase). Such a comparison can be used to analyze state changes in the treated tissue. The mid-procedure physiological parameter(s) may also be compared to one or more predetermined thresholds, for example, to indicate when to stop delivering treatment energy. In some embodiments of the present technology, the measured baseline, mid-, and post-procedure parameters include a complex impedance. In some embodiments of the present technology, the post-procedure physiological parameters are measured after a pre-determined time period to allow the dissipation of the electric field effects (ionic agitation and/or thermal thresholds), thus facilitating accurate assessment of the treatment.

In some embodiments, the anatomical mapping methods described above can be used to differentiate the depth of soft tissues within the nasal mucosa. The depth of mucosa on the turbinates is relatively deep while the depth off the turbinate is relatively shallow and, therefore, identifying the tissue depth in the present technology also identifies positions within the nasal mucosa and where precisely to target. Further, by providing the micro-scale spatial impedance mapping of epithelial tissues as described above, the inherent unique signatures of stratified layers or cellular bodies can be used as identifying the region of interest. For example, different regions have larger or small populations of specific structures, such as submucosal glands, so target regions can be identified via the identification of these structures.

In some embodiments, the system 100 includes additional features that can be used to detect anatomical structures and map anatomical features. For example, the system 100 can include an ultrasound probe for identification of neural tissue and/or other anatomical structures. Higher frequency ultrasound provides higher resolution, but less depth of penetration. Accordingly, the frequency can be varied to achieve the appropriate depth and resolution for neural/anatomical localization. Functional identification may rely on the spatial pulse length ("SPL") (wavelength multiplied by number of cycles in a pulse). Axial resolution (SPL/2) may also be determined to locate nerves.

In some embodiments, the system 100 can further be configured to emit stimuli with selective parameters that suppress rather than fully stimulate neural activity. for example, in embodiments where the strength-duration relationship for extracellular neural stimulation is selected and controlled, a state exists where the extracellular current can hyperpolarize cells, resulting in suppression rather than stimulation spiking behavior (i.e., a full action potential is not achieved). Both models of ion channels, HH and RGC, suggest that it is possible to hyperpolarize cells with appropriately designed burst extracellular stimuli, rather than extending the stimuli. This phenomenon could be used to suppress rather than stimulate neural activity during any of the embodiments of neural detection and/or modulation described herein.

In various embodiments, the system 100 could apply the anatomical mapping techniques disclosed herein to locate or detect the targeted vasculature and surrounding anatomy before, during, and/or after treatment.

## Claims

1. A system (100) for treating a condition within a sino-nasal cavity of a patient, the system comprising:
a treatment device including an end effector (114a, 114b, 214, 314) comprising one or more electrodes (244, 336), wherein:
a subset of the one or more electrodes being configured to deliver non-therapeutic stimulating energy at a frequency/waveform to respective positions at one or more target sites to thereby sense bioelectric properties of one or more tissues at the one or more target sites; and
a subset of the one or more electrodes being configured to deliver therapeutic treatment energy to at least targeted tissue at the one or more target sites;
a database (400) containing a plurality of profiles (402) of known tissue types, each profile including electric signature data for an associated tissue type and a modulation treatment of the tissue type including a pulsed energy treatment pattern for controlling the delivery of pulsed treatment energy, wherein the electric signature data includes bioelectric properties of the tissue type; and
a controller (107) operably associated with the treatment device and configured to control delivery of at least therapeutic treatment energy from the one or more electrodes to the targeted tissue at the one or more target sites within a sino-nasal cavity of the patient based, at least in part, on the pulsed energy treatment pattern sufficient to maintain a consistent temperature of targeted tissue receiving the treatment energy to thereby cause modulation of targeted tissue for the treatment of a nasal condition while further preventing irreversible tissue damage wherein the controller (107) is configured to:
receive identifying data from the treatment device, the identifying data including bioelectric properties of the one or more tissues;
process the identifying data to identify one or more tissue types at the one or more target sites by comparing the identifying data with the electric signature data;
upon a positive correlation of the identifying data and the electric signature data, look up the respective pulsed energy treatment pattern from the database based on the identified tissue types, wherein each of the one or more respective pulsed energy treatment patterns comprises a predetermined treatment time, a level of energy to be delivered from the electrodes, and a predetermined temperature range; and
upon initiating the pulsed energy treatment pattern, automatically control and adjust, via a bang-bang control hardware, delivery of the pulsed treatment energy to a respective identified targeted tissue by processing real-time feedback data associated with the identified targeted tissue receiving treatment energy, including automatically controlling overall power level, voltage, and impedance,
wherein the feedback data comprises temperature measurement data associated with the targeted tissue, a level of energy delivered, and an elapsed delivery time.

2. The system (100) of claim 1, wherein the electric signature data is from a supervised and/or an unsupervised trained neural network.

3. The system (100) of claim 1, further comprising one or more temperature sensors (252) operably associated with the end effector (114a, 114b, 214, 314).

4. The system (100) of claim 3, wherein the temperature measurement data is collected via the one or more temperature sensors.

5. The system (100) of claim 4, wherein the one or more electrodes (244, 336) of the end effector (114a, 114b, 214, 314) are operably associated with the one or more temperature sensors.

6. The system (100) of claim 1, wherein the controller (107) is configured to process the feedback data using an algorithm to determine efficacy of ablation/modulation of the targeted tissue based, at least in part, on a comparison of the feedback data with pulsed energy treatment pattern data.

7. The system (100) of claim 1, wherein delivery of treatment energy to the targeted tissue comprises delivering pulsed energy from one or more electrodes (244, 336) at a level and an associated duty cycle sufficient to regulate and maintain temperature of the targeted tissue receiving the treatment energy at approximately 60 °C with a tolerance of approximately ±0.2 °C.

## Patentansprüche

1. System (100) zum Behandeln einer Erkrankung innerhalb einer sinunasalen Höhle eines Patienten, wobei das System Folgendes umfasst:
eine Behandlungsvorrichtung, die einen Endeffektor (114a, 114b, 214, 314) enthält, der eine oder mehrere Elektroden (244, 336) umfasst, wobei:
eine Teilmenge der einen oder mehreren Elektroden dazu konfiguriert ist, nicht therapeutische Stimulationsenergie mit einer Frequenz/Wellenform an jeweilige Positionen an einer oder mehreren Zielstellen abzugeben, um dadurch bioelektrische Eigenschaften eines oder mehrerer Gewebe an der einen oder den mehreren Zielstellen zu erfassen; und
eine Teilmenge der einen oder mehreren Elektroden dazu konfiguriert ist, therapeutische Behandlungsenergie mindestens an Zielgewebe an der einen oder den mehreren Zielstellen abzugeben;
eine Datenbank (400), die eine Vielzahl von Profilen (402) bekannter Gewebearten beinhaltet, wobei jedes Profil elektrische Signaturdaten für eine zugeordnete Gewebeart und eine Modulationsbehandlung der Gewebeart einschließlich eines gepulsten Energiebehandlungsmusters zum Steuern der Abgabe gepulster Behandlungsenergie enthält, wobei die elektrischen Signaturdaten bioelektrische Eigenschaften der Gewebeart enthalten; und
eine Steuerung (107), die betriebsfähig der Behandlungsvorrichtung zugeordnet und dazu konfiguriert ist, die Abgabe von mindestens therapeutischer Behandlungsenergie von der einen oder den mehreren Elektroden an das Zielgewebe an der einen oder den mehreren Zielstellen innerhalb einer sinunasalen Höhe des Patienten zu steuern, mindestens teilweise auf der Grundlage des gepulsten Energiebehandlungsmusters, das ausreicht, um eine konstante Temperatur des Zielgewebes, das die Behandlungsenergie empfängt, aufrechtzuerhalten, um dadurch eine Modulation des Zielgewebes für die Behandlung einer nasalen Erkrankung zu bewirken, während ferner irreversible Gewebeschäden verhindert werden, wobei die Steuerung (107) zu Folgendem konfiguriert ist:
Empfangen von Identifizierungsdaten von der Behandlungsvorrichtung, wobei die Identifizierungsdaten bioelektrische Eigenschaften des einen oder der mehreren Gewebe enthalten;
Verarbeiten der Identifizierungsdaten zum Identifizieren einer oder mehrerer Gewebearten an den Zielstellen durch Vergleichen der Identifizierungsdaten mit den elektrischen Signaturdaten;
bei einer positiven Korrelation der Identifizierungsdaten und der elektrischen Signaturdaten Nachschlagen des jeweiligen gepulsten Energiebehandlungsmusters in der Datenbank basierend auf den identifizierten Gewebearten, wobei jedes des einen oder der mehreren jeweiligen gepulsten Energiebehandlungsmuster eine vorbestimmte Behandlungszeit, ein von den Elektroden abzugebendes Energieniveau und einen vorbestimmten Temperaturbereich umfasst; und,
nach einem Auslösen des gepulsten Energiebehandlungsmusters, automatisches Steuern und Einstellen, über eine Zweipunkt-Steuerungshardware, der Abgabe der gepulsten Behandlungsenergie an ein jeweiliges identifiziertes Zielgewebe durch Verarbeiten von Echtzeit-Rückkopplungsdaten, die dem identifizierten Zielgewebe zugeordnet sind, das Behandlungsenergie empfängt, einschließlich automatischen Steuerns des Gesamtleistungspegels, der Spannung und der Impedanz,
wobei die Rückkopplungsdaten dem Zielgewebe zugeordnete Temperaturmessdaten, ein Niveau der abgegebenen Energie und eine verstrichene Abgabezeit umfassen.

2. System (100) nach Anspruch 1, wobei die elektrischen Signaturdaten aus einem überwachten und/oder einem nicht überwachten trainierten neuronalen Netz stammen.

3. System (100) nach Anspruch 1, ferner umfassend einen oder mehrere Temperatursensoren (252), die betriebsfähig dem Endeffektor (114a, 114b, 214, 314) zugeordnet sind.

4. System (100) nach Anspruch 3, wobei die Temperaturmessdaten über den einen oder die mehreren Temperatursensoren gesammelt werden.

5. System (100) nach Anspruch 4, wobei die eine oder mehreren Elektroden (244, 336) des Endeffektors (114a, 114b, 214, 314) betriebsfähig dem einen oder den mehreren Temperatursensoren zugeordnet sind.

6. System (100) nach Anspruch 1, wobei die Steuerung (107) dazu konfiguriert ist, die Rückkopplungsdaten unter Verwendung eines Algorithmus zu verarbeiten, um die Wirksamkeit der Ablation/Modulation des Zielgewebes mindestens zum Teil auf Grundlage eines Vergleichs der Rückkopplungsdaten mit gepulsten Energiebehandlungsmusterdaten zu bestimmen.

7. System (100) nach Anspruch 1, wobei die Abgabe von Behandlungsenergie an das Zielgewebe Abgeben gepulster Energie von einer oder mehreren Elektroden (244, 336) auf einem Niveau und mit einem zugeordneten Arbeitszyklus umfasst, die ausreichen, um die Temperatur des Zielgewebes, das die Behandlungsenergie empfängt, bei etwa 60 °C mit einer Toleranz von annähernd ±0,2 °C zu regulieren und aufrechtzuerhalten.

## Revendications

1. Système (100) destiné au traitement d'une affection à l'intérieur d'une cavité sino-nasale d'un patient, le système comprenant :
un dispositif de traitement comprenant un effecteur terminal (114a, 114b, 214, 314) comprenant une ou plusieurs électrodes (244, 336) :
un sous-ensemble desdites une ou plusieurs électrodes étant conçu pour délivrer une énergie de stimulation non thérapeutique à une fréquence/forme d'onde à des positions respectives au niveau d'un ou de plusieurs sites cibles de manière à ainsi détecter les propriétés bioélectriques d'un ou de plusieurs tissus au niveau desdits un ou plusieurs sites cibles ; et
un sous-ensemble desdites une ou plusieurs électrodes étant conçu pour délivrer une énergie de traitement thérapeutique à au moins un tissu ciblé au niveau desdits un ou plusieurs sites cibles ;
une base de données (400) contenant une pluralité de profils (402) de types de tissus connus, chaque profil comprenant des données de signature électrique pour un type de tissu associé et un traitement de modulation du type de tissu comprenant un modèle de traitement à énergie pulsée pour commander la délivrance d'énergie de traitement pulsée, lesdites données de signature électrique comprenant les propriétés bioélectriques du type de tissu ; et
un dispositif de commande (107) associé de manière fonctionnelle au dispositif de traitement et configuré pour commander la délivrance d'au moins l'énergie de traitement thérapeutique à partir desdites une ou plusieurs électrodes au tissu ciblé au niveau desdits un ou plusieurs sites cibles à l'intérieur d'une cavité sino-nasale du patient sur la base, au moins en partie, du modèle de traitement à énergie pulsée suffisant pour maintenir une température constante du tissu ciblé recevant l'énergie de traitement de manière à ainsi provoquer une modulation du tissu ciblé pour le traitement d'une affection nasale tout en empêchant en outre des lésions irréversibles au tissu, ledit dispositif de commande (107) étant configuré pour :
recevoir des données d'identification en provenance du dispositif de traitement, les données d'identification comprenant les propriétés bioélectriques desdits un ou plusieurs tissus ;
traiter les données d'identification pour identifier un ou plusieurs types de tissus au niveau desdits un ou plusieurs sites cibles en comparant les données d'identification à des données de signature électrique ;
au moment de la corrélation positive des données d'identification et des données de signature électrique, rechercher le modèle de traitement à énergie pulsée respectif dans la base de données en fonction des types de tissus identifiés, chacun desdits un ou plusieurs modèles de traitement à énergie pulsée respectifs comprenant un temps de traitement prédéfini, un niveau d'énergie à délivrer à partir des électrodes et une plage de température prédéfinie ; et
au moment de l'initiation du modèle de traitement à énergie pulsée, commander et ajuster automatiquement, par l'intermédiaire d'un matériel de commande tout ou rien, la délivrance de l'énergie de traitement pulsée à un tissu ciblé identifié respectif en traitant les données de rétroaction en temps réel associées au tissu ciblé identifié recevant l'énergie de traitement, notamment en commandant automatiquement le niveau de puissance global, la tension et l'impédance,
lesdites données de rétroaction comprenant des données de mesure de température associées au tissu ciblé, un niveau d'énergie délivré et un temps de délivrance écoulé.

2. Système (100) selon la revendication 1, lesdites données de signature électrique provenant d'un réseau neuronal entraîné supervisé et/ou d'un réseau neuronal entraîné non supervisé.

3. Système (100) selon la revendication 1, comprenant en outre un ou plusieurs capteurs de température (252) associés de manière fonctionnelle à l'effecteur terminal (114a, 114b, 214, 314).

4. Système (100) selon la revendication 3, lesdites données de mesure de température étant collectées par l'intermédiaire desdits un ou plusieurs capteurs de température.

5. Système (100) selon la revendication 4, lesdites une ou plusieurs électrodes (244, 336) de l'effecteur terminal (114a, 114b, 214, 314) étant associées de manière fonctionnelle auxdits un ou plusieurs capteurs de température.

6. Système (100) selon la revendication 1, ledit dispositif de commande (107) étant configuré pour traiter les données de rétroaction à l'aide d'un algorithme de manière à déterminer l'efficacité de l'ablation/modulation du tissu ciblé sur la base, au moins en partie, de la comparaison des données de rétroaction à des données de modèle de traitement à énergie pulsée.

7. Système (100) selon la revendication 1, ladite délivrance d'énergie de traitement au tissu ciblé comprenant la délivrance d'énergie pulsée à partir d'une ou plusieurs électrodes (244, 336) à un niveau et à un cycle de service associé suffisants pour réguler et maintenir la température du tissu ciblé en recevant de l'énergie de traitement à environ 60°C avec une tolérance d'environ ±0,2°C.
